# EUROPÄISCHE PATENTANMELDUNG

(11) **EP 1 260 519 A1**
(43) Veröffentlichungstag der Anmeldung: **27.11.2002**
(21) Anmeldenummer: 02090148.4
(22) Anmeldetag: 19.04.2002
(51) Int. Cl.: C07K 14/72

(54) **Membranrezeptoren für Steroide und Sterole**

(30) Priorität: 26.04.2001 DE 10120337; 13.11.2001 DE 10156597
(71) Anmelder: SCHERING AKTIENGESELLSCHAFT, 13342 Berlin (DE)
(72) Erfinder: Weiss, Bertram, 14163 Berlin (DE); Lessl, Monika, 13467 Berlin (DE); Fritzemeier, Karl-Heinrich, 13505 Berlin (DE); Toschi, Luisella, 10589 Berlin (DE)

(57) **Zusammenfassung**

Es werden acht Splicevarianten eines Membranrezeptors für Steroide und Sterole und ihre Verwendung beschrieben.

SEQUENZPROTOKOLL

<110> Schering AG

<120> Membranrezeptoren für Steroide und Sterole

<130> 52138

<140>
<141>

<160> 16

<170> PatentIn Ver. 2.1

<210> 1
<211> 7093
<212> DNA
<213> Homo sapiens

<400> 1
cgaggggacg cctcgcgacg gttcctggga gagctggcgg cggccttgct ctgcgcgctc 60
ttcgcgccgc cctccccgcc cgcccgcctc aggattgagg aagtgcgtct gggcccggcc 120
ccggcgcggg gggcagacgg cggtgggacg gccaggcccc ggccccgcca gtgtgtccgc 180
ccggccccgc gtcccggagg agtcagctgt gtgtccagaa cgtgccatgg agacgcttaa 240
cggtgccggg gacacgggcg gcaagccgtc cacgcggggc ggtgaccctg cagcgcggtc 300
ccgcaggacg gaaggcatcc gcgccgcgta caggcgggga gaccgcggcg gcgcccggga 360
cctgctggag gaggcctgcg accagtgcgc gtcccagctg gaaaagggcc agcttctgag 420
catcccggca gcctatgggg atctggagat ggtccgctac ctactcagca agagactggt 480
ggagctgccc accgagccca cggatgacaa cccagccgtg gtggcagcgt attttggaca 540
cacggcagtt gtgcaaaata cgctgcccac cgagcccacg gatgacaacc cagccgtggt 600
ggcagcgtat tttggacaca cggcagttgt gcaggaattg cttgagtcct taccaggtcc 660
ctgcagtccc cagcggcttc tgaactggat gctggccttg gcttgccagc gagggcacct 720
gggggttgtg aagctcctgg tcctgacgca cggggctgac ccggagagct acgctgtcag 780
gaagaatgag ttccctgtca tcgtgcgctt gcccctgtat gcggccatca agtcagggaa 840
tgaagacatt gcaatattcc tgcttcggca tggggcctat ttctgttcct acatcttgct 900
ggatagtcct gaccccagca aacatctgct gagaaagtac ttcattgaag ccagtccctt 960
gcccagcagt tatccgggaa aaacagctct ccgtgtgaaa tggtcccatc tcagactgcc 1020
ctgggtagac ctagactggc tcatagacat ctcctgccag atcacggagc tcgacctttc 1080
tgccaactgc ctggcgaccc tcccctcggt tatcccctgg ggcctcatca atctccggaa 1140
gctgaacctc tccgacaacc acctggggga gctgcctggc gtgcagtcat cggacgaaat 1200
catctgttcc aggctacttg aaattgacat ttccagcaac aagttgtccc acctccctcc 1260
tggattcttg cacctctcaa aacttcaaaa actgacagct tcaaaaaatt gtttagaaaa 1320
attgttcgaa gaagaaaatg ccactaactg gataggttta cggaagctac aggaacttga 1380
tatatctgac aataaattga cagaactccc tgccctgttc cttcactctt tcaagtccct 1440
caattctctg aatgtctcca gaaacaacct gaaggtgttt ccagatccct gggcctgccc 1500
tttgaaatgt tgtaaagctt ccagaaatgc cctggaatgt ctgccagaca aaatggctgt 1560
cttttggaaa aatcacctga aggatgtgga tttctcagaa aacgcactca aagaagttcc 1620
cctgggactt ttccagcttg atgccctcat gttcttgagg ttacagggga accagctggc 1680
ggcacttcca cctcaagaga agtggacctg caggcagctc aaaaccctgg atctctccag 1740
aaaccaactt ggcaaaaatg aagatggact gaaaacgaag cgtattgcct ttttcaccac 1800
cagaggtcgc cagcgctccg ggactgaggc agagacaact atggagttca gtgcatctct 1860
ggtaaccatt gtgttcctgt ctaacaactg taacctctgt gcatacacat gtgcagcaag 1920
tgtgctggaa tttccggcct tcctaagtga gtctttggaa gtcctttgcc tgaacgacaa 1980
ccacctcgac acagtccctc cctcggtttg cctactgaag agcttatcag agctctactt 2040
gggaaacaac cctggcctcc gggagctccc tcctgagctg gggcagctgg gcaacctctg 2100
gcagctggac actgaagacc tgaccatcag caatgtgcct gcagaaatcc aaaaagaagg 2160
ccccaaagca atgctgtctt acctgcgtgc tcagctgcgg aaagcggaaa agtgcaagct 2220
gatgaagatg atcatcgtgg gtcccccgcg ccagggcaag tccaccctcc tggagatctt 2280
acagacgggg agggcccccc aggtggtgca tggagaggcc accatcagga ccaccaagtg 2340
ggagctccag aggccggctg gctcgagagc caaggtcaag gatggtctgc gtgcagagtc 2400
cctgtgggtt gagtccgtgg agttcaacgt ctgggacatc gggggaccgg ccagcatggc 2460
cactgtcaac cagtgcttct tcacggacaa ggccctgtac gtggtggtct ggaacctggc 2520

gctgggggag gaggccgtgg ccaacctcca gttctggctg ctcaacatcg aggccaaggc 2580
cccaaacgcc gtggtgctgg tggtcgggac gcacctggat ttaattgaag ccaagttccg 2640
tgtggaaagg attgcaacgc tgcgtgccta tgtgctggca ctctgccgct ccccctccgg 2700
ctccagggcc acaggcttcc cagacatcac cttcaaacac ttacatgaga tttcctgcaa 2760
gagcctggaa ggtcaggaag ggctgcgaca gctgattttc cacgtcacgt gcagcatgaa 2820
ggacgtcggc agcaccatcg gctgccagcg actggcaggg cggctgatcc ccaggagcta 2880
cctgagcctg caggaggccg tgctggcaga gcagcagcgc cgcagccggg acgacgacgt 2940
gcagtacctg acggacaggc agctggagca gctggtggag cagacgcccg acaacgacat 3000
caaggactac gaggacctgc agtcagccat cagcttcctc atagaaaccg gcaccctgct 3060
ccatttcccg gacaccagcc acggcctgag gaacctctac ttcctcgacc ctatttggct 3120
ctccgaatgt ctgcagagga tctttaatat taagggctct cggtcagtgg ccaagaatgg 3180
ggtgatcaga gcagaagacc tcaggatgct gctggtgggg actggcttca cgcagcagac 3240
ggaagagcag tacttccagt tcctggccaa gtttgagatc gccctgcccg tcgccaatga 3300
cagctacctc ctgccccatc tccttccatc taaacctggc ctggacaccc acggtatgcg 3360
gcaccccaca gccaacacca ttcagagggt atttaagatg agcttcgttc ccgttggctt 3420
ctggcaaagg tttatagcac ggatgctgat cagcctggcg gagatggacc tgcagctttt 3480
tgaaaacaag aagaatacta aaagcaggaa caggaaagtc accatttaca gttttacagg 3540
aaaccagaga aatcgctgta gcacattcag agtgaaaaga aatcagacca tctattggca 3600
ggaagggctc ctggtcactt ttgatggggg ctacctcagt gtggaatctt ccgacgtgaa 3660
ctggaaaaag aagaaaagcg gaggaatgaa aattgtttgc caatcagaag tgagggactt 3720
ctcagccatg gctttcatca cggaccacgt caattccttg attgatcagt ggtttcccgc 3780
cctgacagcc acagagagcg acgggacgcc actcatggag cagtacgtgc cctgcccggt 3840
ctgcgagaca gcctgggccc agcacacgga ccccagtgag aaatcagagg atgtgcagta 3900
cttcgacatg gaagactgtg tcctgacggc catcgagcgg gacttcatct cctgccccag 3960
acacccggac ctccccgtgc cgctgcagga gctggtccct gaactgttca tgaccgactt 4020
cccggccagg ctcttcctgg agaacagcaa gctggagcac agcgaggacg agggcagcgt 4080
cctgggccag ggcggcagtg gcaccgtcat ctaccgggcc cggtaccagg gccagcctgt 4140
ggccgtcaag cgcttccaca tcaaaaaatt caagaacttt gctaacgtac cggcagacac 4200
catgctgagg cacctgcggg ccaccgatgc catgaagaac ttctccgagt tccggcagga 4260
ggccagcatg ctgcacgcgc tgcagcaccc ctgcatcgtg gcgctcatcg gcatcagcat 4320
ccacccgctc tgcttcgccc tggagctcgc gccgctcagc agcctcaaca ccgtgctgtc 4380
cgagaacgcc agagattctt cctttatacc cctgggacac atgctcaccc aaaaaatagc 4440
ctaccagatc gcctcgggcc tggcctacct gcacaagaaa aacatcatct tctgtgacct 4500
gaagtcggac aacattctgg tgtggtccct tgacgtcaag gagcacatca acatcaagct 4560
atctgactac gggatttcga ggcagtcatt ccatgagggc gccctaggcg tggagggcac 4620
tcctggctac caggccccag agatcaggcc tcgcattgta tatgatgaga aggtagatat 4680
gttctcctat ggaatggtgc tctacgagtt gctgtcagga cagcgccctg cactgggcca 4740
ccaccagctc cagattgcca agaagctgtc caagggcatc cgcccggttc tggggcagcc 4800
ggaggaagtg cagttccggc gactgcaggc gctcatgatg gagtgctggg acactaagcc 4860
agagaagcga ccgctggccc tgtcggtggt gagccagatg aaggacccga cttttgccac 4920
cttcatgtat gaactgtgct gtgggaagca gacagccttc ttctcatccc agggccagga 4980
gtacaccgtg gtgttttggg atggaaaaga ggagtccagg aactacacgg tggtgaacac 5040
agagaagggc ctcatggagg tgcagaggat gtgctgccct gggatgaagg tgagctgcca 5100
gctccaggtc cagagatccc tgtggacagc caccgagaat tcctacctgg tcttagcggg 5160
cctcgccgat gggcttgtgg ctgtgtttcc cgtggtgcgg ggcaccccaa aggacagctg 5220
ctcctacctg tgctcacaca cagccaacag gtccaagttc agcatcgcgg atgaagacgc 5280
acggcagaac ccctacccag tgaaggccat ggaggtggtc aacagcggct ctgaggtctg 5340
gtacagcaat gggccgggcc tccttgtcat cgactgtgcc tccctggaga tctgcaggcg 5400
gctggagccc tacatggccc cctccatggt tacgtcagtc gtgtgcagct ctgagggcag 5460
aggggaggag gtcgtctggt gcctggatga caaggccaac tccttggtga tgtaccactc 5520
caccacctac cagctgtgtg cccggtactt ctgcggggtc cccagccccc tcagggacat 5580
gtttcccgtg cggcccttgg acacggaacc cccggcagcc agccacacgg ccaacccaaa 5640
ggtgcctgag ggggactcca tcgcggacgt gagcatcatg tacagtgagg agctgggcac 5700
gcagatcctg atccaccagg aatcactcac tgactactgc tccatgtcct cctactcctc 5760
atccccaccc cgccaggctg ccaggtcccc ctcaagcctc cccagctccc cagcaagttc 5820
ttccagtgtg cctttctcca ccgactgcga ggactcagac atgctacata cgcccggtgc 5880
tgcctccgac aggtctgagc atgacctgac ccccatggac ggggagacct tcagccagca 5940
cctgcaggcc gtgaagatcc tcgccgtcag agacctcatt tgggtcccca ggcgcggtgg 6000
agatgttatc gtcattggcc tggagaagga ttctggcgcc cagcggggcc gagtcattgc 6060
cgtcttaaaa gcccgagagc tgactccgca tggggtgctg gtggatgctg ccgtggtggc 6120
aaaggacact gttgtgtgca cctttgaaaa tgaaaacaca gagtggtgcc tggccgtctg 6180
gaggggctgg ggcgccaggg agttcgacat tttctaccag tcctacgagg agctgggccg 6240
gctggaggct tgcactcgca agagaaggta attcctgtgg aatgactgtc acacatcaga 6300

gctggctggc ccggggctgc agcctgactc ctctgccatc ggcctctagt tctccaagga 6360
cctagaagac agatggagtt ctcccctgaa ctccttgctg ctaagaagtg ctgagaagtt 6420
actcgcctgg cggtggctcc agggttctct ggttctctgg agcagagttc tctgaatacc 6480
ccatccccca actgctgatt ttacagcccc agggaagaca gtggtatcag gctgggagcg 6540
gcctcctctg gcctccccca tcagtttgca ggagcagggg tgcaggatcc tgttctgagc 6600
tgggtcaaac aaagcagggc cgggccttcc tgccatcccc aggtctcaga tggaattaca 6660
ctagaggccc tccgctggga agcacttgag gtagggcagg aggggggctg tgacccctgc 6720
cctttccccg ccagagacct caggctctca gcacattcca caggctcctg agtccccgag 6780
gcctgggcca gcttgggcaa gccaagatca gatgtctctg tgttcgggaa ggtctccgtg 6840
tgggaaagcc cttgggggat cccgggtgag gagtgttgcc ccatccagag aatgaatgag 6900
ttcctttaag tgccaccgcc agcaagccca gaggcacaca ttctgagtgc acccgcttag 6960
cctttacatt cctctccacc gacaaaagga aggggaaact caatcagcag gacttcagaa 7020
agggccttgt gtttatagct ttgtcaagta aatttggacg cagctggaaa cacaggcctg 7080
tttgttgcac ata 7093

<210> 2
<211> 2014
<212> PRT
<213> Homo sapiens

<400> 2
Met Glu Thr Leu Asn Gly Ala Gly Asp Thr Gly Gly Lys Pro Ser Thr
1               5                   10                  15

Arg Gly Gly Asp Pro Ala Ala Arg Ser Arg Arg Thr Glu Gly Ile Arg
            20                  25                  30

Ala Ala Tyr Arg Arg Gly Asp Arg Gly Gly Ala Arg Asp Leu Leu Glu
        35                  40                  45

Glu Ala Cys Asp Gln Cys Ala Ser Gln Leu Glu Lys Gly Gln Leu Leu
    50                  55                  60

Ser Ile Pro Ala Ala Tyr Gly Asp Leu Glu Met Val Arg Tyr Leu Leu
65                  70                  75                  80

Ser Lys Arg Leu Val Glu Leu Pro Thr Glu Pro Thr Asp Asp Asn Pro
                85                  90                  95

Ala Val Val Ala Ala Tyr Phe Gly His Thr Ala Val Val Gln Asn Thr
            100                 105                 110

Leu Pro Thr Glu Pro Thr Asp Asp Asn Pro Ala Val Val Ala Ala Tyr
        115                 120                 125

Phe Gly His Thr Ala Val Val Gln Glu Leu Leu Glu Ser Leu Pro Gly
    130                 135                 140

Pro Cys Ser Pro Gln Arg Leu Leu Asn Trp Met Leu Ala Leu Ala Cys
145                 150                 155                 160

Gln Arg Gly His Leu Gly Val Val Lys Leu Leu Val Leu Thr His Gly
                165                 170                 175

Ala Asp Pro Glu Ser Tyr Ala Val Arg Lys Asn Glu Phe Pro Val Ile
            180                 185                 190

Val Arg Leu Pro Leu Tyr Ala Ala Ile Lys Ser Gly Asn Glu Asp Ile
        195                 200                 205

Ala Ile Phe Leu Leu Arg His Gly Ala Tyr Phe Cys Ser Tyr Ile Leu
    210                 215                 220

Leu Asp Ser Pro Asp Pro Ser Lys His Leu Leu Arg Lys Tyr Phe Ile
225                 230                 235                 240

Glu Ala Ser Pro Leu Pro Ser Ser Tyr Pro Gly Lys Thr Ala Leu Arg
                245                 250                 255

Val Lys Trp Ser His Leu Arg Leu Pro Trp Val Asp Leu Asp Trp Leu
            260                 265                 270

Ile Asp Ile Ser Cys Gln Ile Thr Glu Leu Asp Leu Ser Ala Asn Cys
        275                 280                 285

Leu Ala Thr Leu Pro Ser Val Ile Pro Trp Gly Leu Ile Asn Leu Arg
    290                 295                 300

Lys Leu Asn Leu Ser Asp Asn His Leu Gly Glu Leu Pro Gly Val Gln
305                 310                 315                 320

Ser Ser Asp Glu Ile Ile Cys Ser Arg Leu Leu Glu Ile Asp Ile Ser
                325                 330                 335

Ser Asn Lys Leu Ser His Leu Pro Pro Gly Phe Leu His Leu Ser Lys
            340                 345                 350

Leu Gln Lys Leu Thr Ala Ser Lys Asn Cys Leu Glu Lys Leu Phe Glu
        355                 360                 365

Glu Glu Asn Ala Thr Asn Trp Ile Gly Leu Arg Lys Leu Gln Glu Leu
    370                 375                 380

Asp Ile Ser Asp Asn Lys Leu Thr Glu Leu Pro Ala Leu Phe Leu His
385                 390                 395                 400

Ser Phe Lys Ser Leu Asn Ser Leu Asn Val Ser Arg Asn Asn Leu Lys
                405                 410                 415

Val Phe Pro Asp Pro Trp Ala Cys Pro Leu Lys Cys Cys Lys Ala Ser
            420                 425                 430

Arg Asn Ala Leu Glu Cys Leu Pro Asp Lys Met Ala Val Phe Trp Lys
        435                 440                 445

Asn His Leu Lys Asp Val Asp Phe Ser Glu Asn Ala Leu Lys Glu Val
    450                 455                 460

Pro Leu Gly Leu Phe Gln Leu Asp Ala Leu Met Phe Leu Arg Leu Gln
465                 470                 475                 480

Gly Asn Gln Leu Ala Ala Leu Pro Pro Gln Glu Lys Trp Thr Cys Arg
                485                 490                 495

Gln Leu Lys Thr Leu Asp Leu Ser Arg Asn Gln Leu Gly Lys Asn Glu
            500                 505                 510

Asp Gly Leu Lys Thr Lys Arg Ile Ala Phe Phe Thr Thr Arg Gly Arg
        515                 520                 525

Gln Arg Ser Gly Thr Glu Ala Glu Thr Thr Met Glu Phe Ser Ala Ser
    530                 535                 540

Leu Val Thr Ile Val Phe Leu Ser Asn Asn Cys Asn Leu Cys Ala Tyr
545                 550                 555                 560

Thr Cys Ala Ala Ser Val Leu Glu Phe Pro Ala Phe Leu Ser Glu Ser
                565                 570                 575

Leu Glu Val Leu Cys Leu Asn Asp Asn His Leu Asp Thr Val Pro Pro
            580                 585                 590

Ser Val Cys Leu Leu Lys Ser Leu Ser Glu Leu Tyr Leu Gly Asn Asn
        595                 600                 605

Pro Gly Leu Arg Glu Leu Pro Pro Glu Leu Gly Gln Leu Gly Asn Leu
    610                 615                 620

Trp Gln Leu Asp Thr Glu Asp Leu Thr Ile Ser Asn Val Pro Ala Glu
625                 630                 635                 640

Ile Gln Lys Glu Gly Pro Lys Ala Met Leu Ser Tyr Leu Arg Ala Gln
                645                 650                 655

Leu Arg Lys Ala Glu Lys Cys Lys Leu Met Lys Met Ile Ile Val Gly
            660                 665                 670

Pro Pro Arg Gln Gly Lys Ser Thr Leu Leu Glu Ile Leu Gln Thr Gly
        675                 680                 685

Arg Ala Pro Gln Val Val His Gly Glu Ala Thr Ile Arg Thr Thr Lys
    690                 695                 700

Trp Glu Leu Gln Arg Pro Ala Gly Ser Arg Ala Lys Val Lys Asp Gly
705                 710                 715                 720

Leu Arg Ala Glu Ser Leu Trp Val Glu Ser Val Glu Phe Asn Val Trp
                725                 730                 735

Asp Ile Gly Gly Pro Ala Ser Met Ala Thr Val Asn Gln Cys Phe Phe
            740                 745                 750

Thr Asp Lys Ala Leu Tyr Val Val Val Trp Asn Leu Ala Leu Gly Glu
        755                 760                 765

Glu Ala Val Ala Asn Leu Gln Phe Trp Leu Leu Asn Ile Glu Ala Lys
    770                 775                 780

Ala Pro Asn Ala Val Val Leu Val Val Gly Thr His Leu Asp Leu Ile
785                 790                 795                 800

Glu Ala Lys Phe Arg Val Glu Arg Ile Ala Thr Leu Arg Ala Tyr Val
                805                 810                 815

Leu Ala Leu Cys Arg Ser Pro Ser Gly Ser Arg Ala Thr Gly Phe Pro
            820                 825                 830

Asp Ile Thr Phe Lys His Leu His Glu Ile Ser Cys Lys Ser Leu Glu
        835                 840                 845

Gly Gln Glu Gly Leu Arg Gln Leu Ile Phe His Val Thr Cys Ser Met
    850                 855                 860

Lys Asp Val Gly Ser Thr Ile Gly Cys Gln Arg Leu Ala Gly Arg Leu
865                 870                 875                 880

Ile Pro Arg Ser Tyr Leu Ser Leu Gln Glu Ala Val Leu Ala Glu Gln
                885                 890                 895

Gln Arg Arg Ser Arg Asp Asp Asp Val Gln Tyr Leu Thr Asp Arg Gln
            900                 905                 910

Leu Glu Gln Leu Val Glu Gln Thr Pro Asp Asn Asp Ile Lys Asp Tyr
        915                 920                 925

Glu Asp Leu Gln Ser Ala Ile Ser Phe Leu Ile Glu Thr Gly Thr Leu
    930                 935                 940

Leu His Phe Pro Asp Thr Ser His Gly Leu Arg Asn Leu Tyr Phe Leu
945                 950                 955                 960

Asp Pro Ile Trp Leu Ser Glu Cys Leu Gln Arg Ile Phe Asn Ile Lys
                965                 970                 975

Gly Ser Arg Ser Val Ala Lys Asn Gly Val Ile Arg Ala Glu Asp Leu
            980                 985                 990

Arg Met Leu Leu Val Gly Thr Gly Phe Thr Gln Gln Thr Glu Glu Gln
        995                 1000                1005

Tyr Phe Gln Phe Leu Ala Lys Phe Glu Ile Ala Leu Pro Val Ala Asn
    1010                1015                1020

Asp Ser Tyr Leu Leu Pro His Leu Leu Pro Ser Lys Pro Gly Leu Asp
1025                1030                1035                1040

Thr His Gly Met Arg His Pro Thr Ala Asn Thr Ile Gln Arg Val Phe
                1045                1050                1055

Lys Met Ser Phe Val Pro Val Gly Phe Trp Gln Arg Phe Ile Ala Arg
            1060                1065                1070

Met Leu Ile Ser Leu Ala Glu Met Asp Leu Gln Leu Phe Glu Asn Lys
        1075                1080                1085

Lys Asn Thr Lys Ser Arg Asn Arg Lys Val Thr Ile Tyr Ser Phe Thr
    1090                1095                1100

Gly Asn Gln Arg Asn Arg Cys Ser Thr Phe Arg Val Lys Arg Asn Gln
1105                1110                1115                1120

Thr Ile Tyr Trp Gln Glu Gly Leu Leu Val Thr Phe Asp Gly Gly Tyr
                1125                1130                1135

Leu Ser Val Glu Ser Ser Asp Val Asn Trp Lys Lys Lys Lys Ser Gly
            1140                1145                1150

Gly Met Lys Ile Val Cys Gln Ser Glu Val Arg Asp Phe Ser Ala Met
        1155                1160                1165

Ala Phe Ile Thr Asp His Val Asn Ser Leu Ile Asp Gln Trp Phe Pro
    1170                1175                1180

Ala Leu Thr Ala Thr Glu Ser Asp Gly Thr Pro Leu Met Glu Gln Tyr
1185                1190                1195                1200

Val Pro Cys Pro Val Cys Glu Thr Ala Trp Ala Gln His Thr Asp Pro
                1205                1210                1215

Ser Glu Lys Ser Glu Asp Val Gln Tyr Phe Asp Met Glu Asp Cys Val
            1220                1225                1230

Leu Thr Ala Ile Glu Arg Asp Phe Ile Ser Cys Pro Arg His Pro Asp
        1235                1240                1245

Leu Pro Val Pro Leu Gln Glu Leu Val Pro Glu Leu Phe Met Thr Asp
    1250                1255                1260

Phe Pro Ala Arg Leu Phe Leu Glu Asn Ser Lys Leu Glu His Ser Glu
1265                1270                1275                1280

Asp Glu Gly Ser Val Leu Gly Gln Gly Gly Ser Gly Thr Val Ile Tyr
                1285                1290                1295

Arg Ala Arg Tyr Gln Gly Gln Pro Val Ala Val Lys Arg Phe His Ile
            1300                1305                1310

Lys Lys Phe Lys Asn Phe Ala Asn Val Pro Ala Asp Thr Met Leu Arg
        1315                1320                1325

His Leu Arg Ala Thr Asp Ala Met Lys Asn Phe Ser Glu Phe Arg Gln
    1330                1335                1340

Glu Ala Ser Met Leu His Ala Leu Gln His Pro Cys Ile Val Ala Leu
1345                1350                1355                1360

Ile Gly Ile Ser Ile His Pro Leu Cys Phe Ala Leu Glu Leu Ala Pro
                1365                1370                1375

Leu Ser Ser Leu Asn Thr Val Leu Ser Glu Asn Ala Arg Asp Ser Ser
            1380                1385                1390

Phe Ile Pro Leu Gly His Met Leu Thr Gln Lys Ile Ala Tyr Gln Ile
        1395                1400                1405

Ala Ser Gly Leu Ala Tyr Leu His Lys Lys Asn Ile Ile Phe Cys Asp
    1410                1415                1420

Leu Lys Ser Asp Asn Ile Leu Val Trp Ser Leu Asp Val Lys Glu His
1425                1430                1435                1440

Ile Asn Ile Lys Leu Ser Asp Tyr Gly Ile Ser Arg Gln Ser Phe His
                1445                1450                1455

Glu Gly Ala Leu Gly Val Glu Gly Thr Pro Gly Tyr Gln Ala Pro Glu
            1460                1465                1470

Ile Arg Pro Arg Ile Val Tyr Asp Glu Lys Val Asp Met Phe Ser Tyr
        1475                1480                1485

Gly Met Val Leu Tyr Glu Leu Leu Ser Gly Gln Arg Pro Ala Leu Gly
    1490                1495                1500

His His Gln Leu Gln Ile Ala Lys Lys Leu Ser Lys Gly Ile Arg Pro
1505                1510                1515                1520

Val Leu Gly Gln Pro Glu Glu Val Gln Phe Arg Arg Leu Gln Ala Leu
                1525                1530                1535

Met Met Glu Cys Trp Asp Thr Lys Pro Glu Lys Arg Pro Leu Ala Leu
            1540                1545                1550

Ser Val Val Ser Gln Met Lys Asp Pro Thr Phe Ala Thr Phe Met Tyr
        1555                1560                1565

Glu Leu Cys Cys Gly Lys Gln Thr Ala Phe Phe Ser Ser Gln Gly Gln
    1570                1575                1580

Glu Tyr Thr Val Val Phe Trp Asp Gly Lys Glu Glu Ser Arg Asn Tyr
1585                1590                1595                1600

Thr Val Val Asn Thr Glu Lys Gly Leu Met Glu Val Gln Arg Met Cys
                1605                1610                1615

Cys Pro Gly Met Lys Val Ser Cys Gln Leu Gln Val Gln Arg Ser Leu
            1620                1625                1630

Trp Thr Ala Thr Glu Asn Ser Tyr Leu Val Leu Ala Gly Leu Ala Asp
        1635                1640                1645

Gly Leu Val Ala Val Phe Pro Val Val Arg Gly Thr Pro Lys Asp Ser
    1650                1655                1660

Cys Ser Tyr Leu Cys Ser His Thr Ala Asn Arg Ser Lys Phe Ser Ile
1665                1670                1675                1680

Ala Asp Glu Asp Ala Arg Gln Asn Pro Tyr Pro Val Lys Ala Met Glu
                1685                1690                1695

Val Val Asn Ser Gly Ser Glu Val Trp Tyr Ser Asn Gly Pro Gly Leu
            1700                1705                1710

Leu Val Ile Asp Cys Ala Ser Leu Glu Ile Cys Arg Arg Leu Glu Pro
        1715                1720                1725

Tyr Met Ala Pro Ser Met Val Thr Ser Val Val Cys Ser Ser Glu Gly
    1730                1735                1740

Arg Gly Glu Glu Val Val Trp Cys Leu Asp Asp Lys Ala Asn Ser Leu
1745                1750                1755                1760

Val Met Tyr His Ser Thr Thr Tyr Gln Leu Cys Ala Arg Tyr Phe Cys
                1765                1770                1775

Gly Val Pro Ser Pro Leu Arg Asp Met Phe Pro Val Arg Pro Leu Asp
            1780                1785                1790

Thr Glu Pro Pro Ala Ala Ser His Thr Ala Asn Pro Lys Val Pro Glu
        1795                1800                1805

Gly Asp Ser Ile Ala Asp Val Ser Ile Met Tyr Ser Glu Glu Leu Gly
    1810                1815                1820

Thr Gln Ile Leu Ile His Gln Glu Ser Leu Thr Asp Tyr Cys Ser Met
1825                1830                1835                1840

Ser Ser Tyr Ser Ser Ser Pro Pro Arg Gln Ala Ala Arg Ser Pro Ser
                1845                1850                1855

Ser Leu Pro Ser Ser Pro Ala Ser Ser Ser Ser Val Pro Phe Ser Thr
            1860                1865                1870

Asp Cys Glu Asp Ser Asp Met Leu His Thr Pro Gly Ala Ala Ser Asp
        1875                1880                1885

Arg Ser Glu His Asp Leu Thr Pro Met Asp Gly Glu Thr Phe Ser Gln
    1890                1895                1900

His Leu Gln Ala Val Lys Ile Leu Ala Val Arg Asp Leu Ile Trp Val
1905                1910                1915                1920

Pro Arg Arg Gly Gly Asp Val Ile Val Ile Gly Leu Glu Lys Asp Ser
                1925                1930                1935

Gly Ala Gln Arg Gly Arg Val Ile Ala Val Leu Lys Ala Arg Glu Leu
            1940                1945                1950

Thr Pro His Gly Val Leu Val Asp Ala Ala Val Val Ala Lys Asp Thr
        1955                1960                1965

Val Val Cys Thr Phe Glu Asn Glu Asn Thr Glu Trp Cys Leu Ala Val
    1970                1975                1980

Trp Arg Gly Trp Gly Ala Arg Glu Phe Asp Ile Phe Tyr Gln Ser Tyr
1985                1990                1995                2000

Glu Glu Leu Gly Arg Leu Glu Ala Cys Thr Arg Lys Arg Arg
                2005                2010


<210> 3
<211> 7307
<212> DNA
<213> Homo sapiens

<400> 3
gcggccgccg aggggacgcc tcgtgacggt tcctgggaga gctggcggcg gccttgctct 60
gcgcgctctt cgcgccgccc tccccgcccg cccgcctcag gattgaggaa gtgcgtctgg 120
gcccggcccc ggcgcggggg gcagacggcg gtgggacggc caggccccgg ccccgccagt 180
gtgtccgccc ggccccgcgt cccggagcgc ccgcacccgg ccccgccgcc gcctcagagc 240
aagaaagctt tctgctcagc catggctacg agtccacgcc ttaatgcacc ccacagccag 300
cggcagtggc agtgacaaca gcgggacctg cctttgaaga tcggctgctg caagggttga 360
tggctggcat gtcgcaaaga ccccccagca tgtactggtg tgtggggccg gaggagtcag 420
ctgtgtgtcc agaacgtgcc atggagacgc ttaacggtgc cggggacacg ggcggcaagc 480
cgtccacgcg gggcggtgac cctgcagcgc ggtcccgcag gacggaaggc atccgcgccg 540
cgtacaggcg gggagaccgc ggcggcgccc gggacctgct ggaggaggcc tgcgaccagt 600
gcgcgtccca gctggaaaag ggccagcttc tgagcatccc ggcagcctat ggggatctgg 660
agatggtccg ctacctactc agcaagagac tggtggagct gcccaccgag cccacggatg 720
acaacccagc cgtggtggca gcgtattttg gacacacggc agttgtgcaa aatacgctgc 780
ccaccgagcc cacggatgac aacccagccg tggtggcagc gtattttgga cacacggcag 840
ttgtgcagga attgcttgag tccttaccag gtccctgcag tccccagcgg cttctgaact 900
ggatgctggc cttggcttgc cagcgagggc acctgggggt tgtgaagctc ctggtcctga 960
cgcacggggc tgacccggag agctacgctg tcaggaagaa tgagttccct gtcatcgtgc 1020
gcttgcccct gtatgcggcc atcaagtcag ggaatgaaga cattgcaata ttcctgcttc 1080
ggcatggggc ctatttctgt tcctacatct tgctggatag tcctgacccc agcaaacatc 1140
tgctgagaaa gtacttcatt gaagccagtc ccttgcccag cagttatccg ggaaaaacag 1200
ctctccgtgt gaaatggtcc catctcagac tgccctgggt agacctagac tggctcatag 1260
acatctcctg ccagatcacg gagctcgacc tttctgccaa ctgcctggcg accctcccct 1320
cggttatccc ctggggcctc atcaatctcc ggaagctgaa cctctccgac aaccacctgg 1380
gggagctgcc tggcgtgcag tcatcggacg aaatcatctg ttccaggcta cttgaaattg 1440
acatttccag caacaagttg tcccacctcc ctcctggatt cttgcacctc tcaaaacttc 1500
aaaaactgac agcttcaaaa aattgtttag aaaaattgtt cgaagaagaa aatgccacta 1560
actggatagg tttacggaag ctacaggaac ttgatatatc tgacaataaa ttgacagaac 1620
tccctgccct gttccttcac tctttcaagt ccctcaattc tctgaatgtc tccagaaaca 1680
acctgaaggt gtttccagat ccctgggcct gccctttgaa atgttgtaaa gcttccagaa 1740
atgccctgga atgtctgcca gacaaaatgg ctgtcttttg gaaaaatcac ctgaaggatg 1800
tggatttctc agaaaacgca ctcaaagaag ttcccctggg acttttccag cttgatgccc 1860
tcatgttctt gaggttacag gggaaccagc tggcggcact tccacctcaa gagaagtgga 1920
cctgcaggca gctcaaaacc ctggatctct ccagaaacca acttggcaaa aatgaagatg 1980
gactgaaaac gaagcgtatt gcctttttca ccaccagagg tcgccagcgc tccgggactg 2040

aggcagagac aactatggag ttcagtgcat ctctggtaac cattgtgttc ctgtctaaca 2100
actgtaacct ctgtgcatac acatgtgcag caagtgtgct ggaatttccg gccttcctaa 2160
gtgagtcttt ggaagtcctt tgcctgaacg acaaccacct cgacacagtc cctccctcgg 2220
tttgcctact gaagagctta tcagagctct acttgggaaa caaccctggc ctccgggagc 2280
tccctcctga gctggggcag ctgggcaacc tctggcagct ggacactgaa gacctgacca 2340
tcagcaatgt gcctgcagaa atccaaaaag aaggccccaa agcaatgctg tcttacctgc 2400
gtgctcagct gcggaaagcg gaaaagtgca agctgatgaa gatgatcatc gtgggtcccc 2460
cgcgccaggg caagtccacc ctcctggaga tcttacagac ggggagggcc ccccaggtgg 2520
tgcatggaga ggccaccatc aggaccacca agtgggagct ccagaggccg gctggctcga 2580
gagccaaggt caaggatggt ctgcgtgcag agtccctgtg ggttgagtcc gtggagttca 2640
acgtctggga catcggggga ccggccagca tggccactgt caaccagtgc ttcttcacgg 2700
acaaggccct gtacgtggtg gtctggaacc tggcgctggg ggaggaggcc gtggccaacc 2760
tccagttctg gctgctcaac atcgaggcca aggccccaaa cgccgtggtg ctggtggtcg 2820
ggacgcacct ggatttaatt gaagccaagt tccgtgtgga aaggattgca acgctgcgtg 2880
cctatgtgct ggcactctgc cgctccccct ccggctccag ggccacaggc ttcccagaca 2940
tcaccttcaa acacttacat gagatttcct gcaagagcct ggaaggtcag gaaggcctgc 3000
gacagctgat tttccacgtc acgtgcagca tgaaggacgt cggcagcacc atcggctgcc 3060
agcgactggc agggcggctg atccccagga gctacctgag cctgcaggag gccgtgctgg 3120
cagagcagca gcgccgcagc cgggacgacg acgtgcagta cctgacggac aggcagctgg 3180
agcagctggt ggagcagacg cccgacaacg acatcaagga ctacgaggac ctgcagtcag 3240
ccatcagctt cctcatagaa accggcaccc tgctccattt cccggacacc agccacggcc 3300
tgaggaacct ctacttcctc gaccctattt ggctctccga atgtctgcag aggatcttta 3360
atattaaggg ctctcggtca gtggccaaga atggggtgat cagagcagaa gacctcagga 3420
tgctgctggt ggggactggc ttcacgcagc agacggaaga gcagtacttc cagttcctgg 3480
ccaagtttga gatcgccctg cccgtcgcca atgacagcta cctcctgccc catctccttc 3540
catctaaacc tggcctggac acccacggta tgcggcaccc cacagccaac accattcaga 3600
gggtatttaa gatgagcttc gttcccgttg gcttctggca aaggtttata gcacggatgc 3660
tgatcagcct ggcggagatg gacctgcagc tttttgaaaa caagaagaat actaaaagca 3720
ggaacaggaa agtcaccatt tacagtttta caggaaacca gagaaatcgc tgtagcacat 3780
tcagagtgaa aagaaatcag accatctatt ggcaggaagg gctcctggtc acttttgatg 3840
ggggctacct cagtgtggaa tcttccgacg tgaactggaa aaagaagaaa agcggaggaa 3900
tgaaaattgt ttgccaatca gaagtgaggg acttctcagc catggctttc atcacggacc 3960
acgtcaattc cttgattgat cagtggtttc ccgccctgac agccacagag agcgacggga 4020
cgccactcat ggagcagtac gtgccctgcc cggtctgcga gacagcctgg gcccagcaca 4080
cggaccccag tgagaaatca gaggatgtgc agtacttcga catggaagac tgtgtcctga 4140
cggccatcga gcgggacttc atctcctgcc ccagacaccc ggacctcccc gtgccgctgc 4200
aggagctggt ccctgaactg ttcatgaccg acttcccggc caggctcttc ctggagaaca 4260
gcaagctgga gcacagcgag gacgagggca gcgtcctggg ccagggcggc agtggcaccg 4320
tcatctaccg ggcccggtac cagggccagc ctgtggccgt caagcgcttc cacatcaaaa 4380
aattcaagaa ctttgctaac gtaccggcag acaccatgct gaggcacctg cgggccaccg 4440
atgccatgaa gaacttctcc gagttccggc aggaggccag catgctgcac gcgctgcagc 4500
acccctgcat cgtggcgctc atcggcatca gcatccaccc gctctgcttc gccctggagc 4560
tcgcgccgct cagcagcctc aacaccgtgc tgtccgagaa cgccagagat tcttccttta 4620
tacccctggg acacatgctc acccaaaaaa tagcctacca gatcgcctcg ggcctggcct 4680
acctgcacaa gaaaaacatc atcttctgtg acctgaagtc ggacaacatt ctggtgtggt 4740
cccttgacgt caaggagcac atcaacatca agctatctga ctacggattt tcgaggcagt 4800
cattccatga gggcgcccta ggcgtggagg gcactcctgg ctaccaggcc ccagagatca 4860
ggcctcgcat tgtatatgat gagaaggtag atatgttctc ctatggaatg gtgctctacg 4920
agttgctgtc aggacagcgc cctgcactgg gccaccacca gctccagatt gccaagaagc 4980
tgtccaaggg catccgcccg gttctggggc agccggagga agtgcagttc cggcgactgc 5040
aggcgctcat gatggagtgc tgggacacta agccagagaa gcgaccgctg gccctgtcgg 5100
tggtgagcca gatgaaggac ccgacttttg ccaccttcat gtatgaactg tgctgtggga 5160
agcagacagc cttcttctca tcccagggcc aggagtacac cgtggtgttt tgggatggaa 5220
aagaggagtc caggaactac acggtggtga acacagagaa gggcctcatg gaggtgcaga 5280
ggatgtgctg ccctgggatg aaggtgagct gccagctcca ggtccagaga tccctgtgga 5340
cagccaccga gaattcctac ctggtcttag cgggcctcgc cgatgggctt gtggctgtgt 5400
ttcccgtggt gcgggcaccc ccaaaggaca gctgctccta cctgtgctca cacacagcca 5460
acaggtccaa gttcagcatc gcggatgaag acgcacggca gaacccctac ccagtgaagg 5520
ccatggaggt ggtcaacagc ggctctgagg tctggtacag caatgggccg ggcctccttg 5580
tcatcgactg tgcctccctg gagatctgca ggcggctgga gccctacatg gccccctcca 5640
tggttacgtc agtcgtgtgc agctctgagg gcagagggga ggaggtcgtc tggtgcctgg 5700
atgacaaggc caactccttg gtgatgtacc actccaccac ctaccagctg tgtgcccggt 5760
acttctgcgg ggtccccagc cccctcaggg acatgtttcc cgtgcggccc ttggacacgg 5820

aacccccggc agccagccac acggccaacc caaaggtgcc tgagggggac tccatcgcgg 5880
acgtgagcat catgtacagt gaggagctgg gcacgcagat cctgatccac caggaatcac 5940
tcactgacta ctgctccatg tcctcctact cctcatcccc accccgccag gctgccaggt 6000
ccccctcaag cctccccagc tccccagcaa gttcttccag tgtgcctttc tccaccgact 6060
gcgaggactc agacatgcta catacgcccg gtgctgcctc cgacaggtct gagcatgacc 6120
tgacccccat ggacggggag accttcagcc agcacctgca ggccgtgaag atcctcgccg 6180
tcagagacct catttgggtc cccaggcgcg gtggagatgt tatcgtcatt ggcctggaga 6240
aggattctgg cgcccagcgg ggccgagtca ttgccgtctt aaaagcccga gagctgactc 6300
cgcatggggt gctggtggat gctgccgtgg tggcaaagga cactgttgtg tgcacctttg 6360
aaaatgaaaa cacagagtgg tgcctggccg tctggagggg ctggggcgcc agggagttcg 6420
acattttcta ccagtcctac gaggagctgg gccggctgga ggcttgcact cgcaagagaa 6480
ggtaattcct gtggaatgac tgtcacacat cagagctggc tggcccgggg ctgcagcctg 6540
actcctctgc catcggcctc tagttctcca aggacctaga agacagatgg agttctcccc 6600
tgaactcctt gctgctaaga agtgctgaga agttactcgc ctggcggtgg ctccagggtt 6660
ctctggttct ctggagcaga gttctctgaa taccccatcc cccaactgct gattttacag 6720
ccccagggaa gacagtggta tcaggctggg agcggcctcc tctggcctcc cccatcagtt 6780
tgcaggagca ggggtgcagg atcctgttct gagctgggtc aaacaaagca gggccgggcc 6840
ttcctgccat ccccaggtct cagatggaat tacactagag gccctccgct gggaagcact 6900
tgaggtaggg caggaggggg gctgtgaccc ctgccctttc cccgccagag acctcaggct 6960
ctcagcacat tccacaggct cctgagtccc cgaggcctgg gccagcttgg gcaagccaag 7020
atcagatgtc tctgtgttcg ggaaggtctc cgtgtgggaa agcccttggg ggatcccggg 7080
tgaggagtgt tgccccatcc agagaatgaa tgagttcctt taagtgccac cgccagcaag 7140
cccagaggca cacattctga gtgcacccgc ttagccttta cattcctctc caccgacaaa 7200
aggaagggga aactcaatca gcaggacttc agaaaggccc ttgtgtttat agctttgtca 7260
agtaaatttg gacgcagctg gaaacacagg cctgtttgtt gcacata 7307

<210> 4
<211> 2041
<212> PRT
<213> Homo sapiens

<400> 4
Met Ala Gly Met Ser Gln Arg Pro Pro Ser Met Tyr Trp Cys Val Gly
1               5                   10                  15

Pro Glu Glu Ser Ala Val Cys Pro Glu Arg Ala Met Glu Thr Leu Asn
            20                  25                  30

Gly Ala Gly Asp Thr Gly Gly Lys Pro Ser Thr Arg Gly Gly Asp Pro
        35                  40                  45

Ala Ala Arg Ser Arg Arg Thr Glu Gly Ile Arg Ala Ala Tyr Arg Arg
    50                  55                  60

Gly Asp Arg Gly Gly Ala Arg Asp Leu Leu Glu Glu Ala Cys Asp Gln
65                  70                  75                  80

Cys Ala Ser Gln Leu Glu Lys Gly Gln Leu Leu Ser Ile Pro Ala Ala
                85                  90                  95

Tyr Gly Asp Leu Glu Met Val Arg Tyr Leu Leu Ser Lys Arg Leu Val
            100                 105                 110

Glu Leu Pro Thr Glu Pro Thr Asp Asp Asn Pro Ala Val Val Ala Ala
        115                 120                 125

Tyr Phe Gly His Thr Ala Val Val Gln Asn Thr Leu Pro Thr Glu Pro
    130                 135                 140

Thr Asp Asp Asn Pro Ala Val Val Ala Ala Tyr Phe Gly His Thr Ala
145                 150                 155                 160

Val Val Gln Glu Leu Leu Glu Ser Leu Pro Gly Pro Cys Ser Pro Gln
                165                 170                 175

Arg Leu Leu Asn Trp Met Leu Ala Leu Ala Cys Gln Arg Gly His Leu
            180                 185                 190

Gly Val Val Lys Leu Leu Val Leu Thr His Gly Ala Asp Pro Glu Ser
        195                 200                 205

Tyr Ala Val Arg Lys Asn Glu Phe Pro Val Ile Val Arg Leu Pro Leu
    210                 215                 220

Tyr Ala Ala Ile Lys Ser Gly Asn Glu Asp Ile Ala Ile Phe Leu Leu
225                 230                 235                 240

Arg His Gly Ala Tyr Phe Cys Ser Tyr Ile Leu Leu Asp Ser Pro Asp
                245                 250                 255

Pro Ser Lys His Leu Leu Arg Lys Tyr Phe Ile Glu Ala Ser Pro Leu
            260                 265                 270

Pro Ser Ser Tyr Pro Gly Lys Thr Ala Leu Arg Val Lys Trp Ser His
        275                 280                 285

Leu Arg Leu Pro Trp Val Asp Leu Asp Trp Leu Ile Asp Ile Ser Cys
    290                 295                 300

Gln Ile Thr Glu Leu Asp Leu Ser Ala Asn Cys Leu Ala Thr Leu Pro
305                 310                 315                 320

Ser Val Ile Pro Trp Gly Leu Ile Asn Leu Arg Lys Leu Asn Leu Ser
                325                 330                 335

Asp Asn His Leu Gly Glu Leu Pro Gly Val Gln Ser Ser Asp Glu Ile
            340                 345                 350

Ile Cys Ser Arg Leu Leu Glu Ile Asp Ile Ser Ser Asn Lys Leu Ser
        355                 360                 365

His Leu Pro Pro Gly Phe Leu His Leu Ser Lys Leu Gln Lys Leu Thr
    370                 375                 380

Ala Ser Lys Asn Cys Leu Glu Lys Leu Phe Glu Glu Glu Asn Ala Thr
385                 390                 395                 400

Asn Trp Ile Gly Leu Arg Lys Leu Gln Glu Leu Asp Ile Ser Asp Asn
                405                 410                 415

Lys Leu Thr Glu Leu Pro Ala Leu Phe Leu His Ser Phe Lys Ser Leu
            420                 425                 430

Asn Ser Leu Asn Val Ser Arg Asn Asn Leu Lys Val Phe Pro Asp Pro
        435                 440                 445

Trp Ala Cys Pro Leu Lys Cys Cys Lys Ala Ser Arg Asn Ala Leu Glu
    450                 455                 460

Cys Leu Pro Asp Lys Met Ala Val Phe Trp Lys Asn His Leu Lys Asp
465                 470                 475                 480

Val Asp Phe Ser Glu Asn Ala Leu Lys Glu Val Pro Leu Gly Leu Phe
                485                 490                 495

Gln Leu Asp Ala Leu Met Phe Leu Arg Leu Gln Gly Asn Gln Leu Ala
            500                 505                 510

Ala Leu Pro Pro Gln Glu Lys Trp Thr Cys Arg Gln Leu Lys Thr Leu
        515                 520                 525

Asp Leu Ser Arg Asn Gln Leu Gly Lys Asn Glu Asp Gly Leu Lys Thr
    530                 535                 540

Lys Arg Ile Ala Phe Phe Thr Thr Arg Gly Arg Gln Arg Ser Gly Thr
545                 550                 555                 560

Glu Ala Glu Thr Thr Met Glu Phe Ser Ala Ser Leu Val Thr Ile Val
                565                 570                 575

Phe Leu Ser Asn Asn Cys Asn Leu Cys Ala Tyr Thr Cys Ala Ala Ser
            580                 585                 590

Val Leu Glu Phe Pro Ala Phe Leu Ser Glu Ser Leu Glu Val Leu Cys
        595                 600                 605

Leu Asn Asp Asn His Leu Asp Thr Val Pro Pro Ser Val Cys Leu Leu
    610                 615                 620

Lys Ser Leu Ser Glu Leu Tyr Leu Gly Asn Asn Pro Gly Leu Arg Glu
625                 630                 635                 640

Leu Pro Pro Glu Leu Gly Gln Leu Gly Asn Leu Trp Gln Leu Asp Thr
                645                 650                 655

Glu Asp Leu Thr Ile Ser Asn Val Pro Ala Glu Ile Gln Lys Glu Gly
            660                 665                 670

Pro Lys Ala Met Leu Ser Tyr Leu Arg Ala Gln Leu Arg Lys Ala Glu
        675                 680                 685

Lys Cys Lys Leu Met Lys Met Ile Ile Val Gly Pro Pro Arg Gln Gly
    690                 695                 700

Lys Ser Thr Leu Leu Glu Ile Leu Gln Thr Gly Arg Ala Pro Gln Val
705                 710                 715                 720

Val His Gly Glu Ala Thr Ile Arg Thr Thr Lys Trp Glu Leu Gln Arg
                725                 730                 735

Pro Ala Gly Ser Arg Ala Lys Val Lys Asp Gly Leu Arg Ala Glu Ser
            740                 745                 750

Leu Trp Val Glu Ser Val Glu Phe Asn Val Trp Asp Ile Gly Gly Pro
        755                 760                 765

Ala Ser Met Ala Thr Val Asn Gln Cys Phe Phe Thr Asp Lys Ala Leu
    770                 775                 780

Tyr Val Val Val Trp Asn Leu Ala Leu Gly Glu Glu Ala Val Ala Asn
785                 790                 795                 800

Leu Gln Phe Trp Leu Leu Asn Ile Glu Ala Lys Ala Pro Asn Ala Val
                805                 810                 815

Val Leu Val Val Gly Thr His Leu Asp Leu Ile Glu Ala Lys Phe Arg
            820                 825                 830

Val Glu Arg Ile Ala Thr Leu Arg Ala Tyr Val Leu Ala Leu Cys Arg
        835                 840                 845

Ser Pro Ser Gly Ser Arg Ala Thr Gly Phe Pro Asp Ile Thr Phe Lys
    850                 855                 860

His Leu His Glu Ile Ser Cys Lys Ser Leu Glu Gly Gln Glu Gly Leu
865                 870                 875                 880

Arg Gln Leu Ile Phe His Val Thr Cys Ser Met Lys Asp Val Gly Ser
                885                 890                 895

Thr Ile Gly Cys Gln Arg Leu Ala Gly Arg Leu Ile Pro Arg Ser Tyr
            900                 905                 910

Leu Ser Leu Gln Glu Ala Val Leu Ala Glu Gln Gln Arg Arg Ser Arg
        915                 920                 925

Asp Asp Asp Val Gln Tyr Leu Thr Asp Arg Gln Leu Glu Gln Leu Val
    930                 935                 940

Glu Gln Thr Pro Asp Asn Asp Ile Lys Asp Tyr Glu Asp Leu Gln Ser
945                 950                 955                 960

Ala Ile Ser Phe Leu Ile Glu Thr Gly Thr Leu Leu His Phe Pro Asp
                965                 970                 975

Thr Ser His Gly Leu Arg Asn Leu Tyr Phe Leu Asp Pro Ile Trp Leu
            980                 985                 990

Ser Glu Cys Leu Gln Arg Ile Phe Asn Ile Lys Gly Ser Arg Ser Val
        995                 1000                1005

Ala Lys Asn Gly Val Ile Arg Ala Glu Asp Leu Arg Met Leu Leu Val
    1010                1015                1020

Gly Thr Gly Phe Thr Gln Gln Thr Glu Glu Gln Tyr Phe Gln Phe Leu
1025                1030                1035                1040

Ala Lys Phe Glu Ile Ala Leu Pro Val Ala Asn Asp Ser Tyr Leu Leu
                1045                1050                1055

Pro His Leu Leu Pro Ser Lys Pro Gly Leu Asp Thr His Gly Met Arg
            1060                1065                1070

His Pro Thr Ala Asn Thr Ile Gln Arg Val Phe Lys Met Ser Phe Val
        1075                1080                1085

Pro Val Gly Phe Trp Gln Arg Phe Ile Ala Arg Met Leu Ile Ser Leu
    1090                1095                1100

Ala Glu Met Asp Leu Gln Leu Phe Glu Asn Lys Lys Asn Thr Lys Ser
1105                1110                1115                1120

Arg Asn Arg Lys Val Thr Ile Tyr Ser Phe Thr Gly Asn Gln Arg Asn
                1125                1130                1135

Arg Cys Ser Thr Phe Arg Val Lys Arg Asn Gln Thr Ile Tyr Trp Gln
            1140                1145                1150

Glu Gly Leu Leu Val Thr Phe Asp Gly Gly Tyr Leu Ser Val Glu Ser
        1155                1160                1165

Ser Asp Val Asn Trp Lys Lys Lys Lys Ser Gly Gly Met Lys Ile Val
1170                1175                1180

Cys Gln Ser Glu Val Arg Asp Phe Ser Ala Met Ala Phe Ile Thr Asp
1185                1190                1195                1200

His Val Asn Ser Leu Ile Asp Gln Trp Phe Pro Ala Leu Thr Ala Thr
                1205                1210                1215

Glu Ser Asp Gly Thr Pro Leu Met Glu Gln Tyr Val Pro Cys Pro Val
            1220                1225                1230

Cys Glu Thr Ala Trp Ala Gln His Thr Asp Pro Ser Glu Lys Ser Glu
        1235                1240                1245

Asp Val Gln Tyr Phe Asp Met Glu Asp Cys Val Leu Thr Ala Ile Glu
    1250                1255                1260

Arg Asp Phe Ile Ser Cys Pro Arg His Pro Asp Leu Pro Val Pro Leu
1265                1270                1275                1280

Gln Glu Leu Val Pro Glu Leu Phe Met Thr Asp Phe Pro Ala Arg Leu
                1285                1290                1295

Phe Leu Glu Asn Ser Lys Leu Glu His Ser Glu Asp Glu Gly Ser Val
            1300                1305                1310

Leu Gly Gln Gly Gly Ser Gly Thr Val Ile Tyr Arg Ala Arg Tyr Gln
        1315                1320                1325

Gly Gln Pro Val Ala Val Lys Arg Phe His Ile Lys Lys Phe Lys Asn
    1330                1335                1340

Phe Ala Asn Val Pro Ala Asp Thr Met Leu Arg His Leu Arg Ala Thr
1345                1350                1355                1360

Asp Ala Met Lys Asn Phe Ser Glu Phe Arg Gln Glu Ala Ser Met Leu
                1365                1370                1375

His Ala Leu Gln His Pro Cys Ile Val Ala Leu Ile Gly Ile Ser Ile
            1380                1385                1390

His Pro Leu Cys Phe Ala Leu Glu Leu Ala Pro Leu Ser Ser Leu Asn
        1395                1400                1405

Thr Val Leu Ser Glu Asn Ala Arg Asp Ser Ser Phe Ile Pro Leu Gly
    1410                1415                1420

His Met Leu Thr Gln Lys Ile Ala Tyr Gln Ile Ala Ser Gly Leu Ala
1425                1430                1435                1440

Tyr Leu His Lys Lys Asn Ile Ile Phe Cys Asp Leu Lys Ser Asp Asn
                1445                1450                1455

Ile Leu Val Trp Ser Leu Asp Val Lys Glu His Ile Asn Ile Lys Leu
            1460                1465                1470

Ser Asp Tyr Gly Ile Ser Arg Gln Ser Phe His Glu Gly Ala Leu Gly
        1475                1480                1485

Val Glu Gly Thr Pro Gly Tyr Gln Ala Pro Glu Ile Arg Pro Arg Ile
    1490                1495                1500

Val Tyr Asp Glu Lys Val Asp Met Phe Ser Tyr Gly Met Val Leu Tyr
1505                1510                1515                1520

Glu Leu Leu Ser Gly Gln Arg Pro Ala Leu Gly His His Gln Leu Gln
                1525                1530                1535

Ile Ala Lys Lys Leu Ser Lys Gly Ile Arg Pro Val Leu Gly Gln Pro
            1540                1545                1550

Glu Glu Val Gln Phe Arg Arg Leu Gln Ala Leu Met Met Glu Cys Trp
        1555                1560                1565

Asp Thr Lys Pro Glu Lys Arg Pro Leu Ala Leu Ser Val Val Ser Gln
    1570                1575                1580

Met Lys Asp Pro Thr Phe Ala Thr Phe Met Tyr Glu Leu Cys Cys Gly
1585                1590                1595                1600

Lys Gln Thr Ala Phe Phe Ser Ser Gln Gly Gln Glu Tyr Thr Val Val
                1605                1610                1615

Phe Trp Asp Gly Lys Glu Glu Ser Arg Asn Tyr Thr Val Val Asn Thr
            1620                1625                1630

Glu Lys Gly Leu Met Glu Val Gln Arg Met Cys Cys Pro Gly Met Lys
        1635                1640                1645

Val Ser Cys Gln Leu Gln Val Gln Arg Ser Leu Trp Thr Ala Thr Glu
    1650                1655                1660

Asn Ser Tyr Leu Val Leu Ala Gly Leu Ala Asp Gly Leu Val Ala Val
1665                1670                1675                1680

Phe Pro Val Val Arg Gly Thr Pro Lys Asp Ser Cys Ser Tyr Leu Cys
                1685                1690                1695

Ser His Thr Ala Asn Arg Ser Lys Phe Ser Ile Ala Asp Glu Asp Ala
            1700                1705                1710

Arg Gln Asn Pro Tyr Pro Val Lys Ala Met Glu Val Val Asn Ser Gly
        1715                1720                1725

Ser Glu Val Trp Tyr Ser Asn Gly Pro Gly Leu Leu Val Ile Asp Cys
    1730                1735                1740

Ala Ser Leu Glu Ile Cys Arg Arg Leu Glu Pro Tyr Met Ala Pro Ser
1745                1750                1755                1760

Met Val Thr Ser Val Val Cys Ser Ser Glu Gly Arg Gly Glu Glu Val
                1765                1770                1775

Val Trp Cys Leu Asp Asp Lys Ala Asn Ser Leu Val Met Tyr His Ser
            1780                1785                1790

Thr Thr Tyr Gln Leu Cys Ala Arg Tyr Phe Cys Gly Val Pro Ser Pro
        1795                1800                1805

Leu Arg Asp Met Phe Pro Val Arg Pro Leu Asp Thr Glu Pro Pro Ala
    1810                1815                1820

Ala Ser His Thr Ala Asn Pro Lys Val Pro Glu Gly Asp Ser Ile Ala
1825                1830                1835                1840

Asp Val Ser Ile Met Tyr Ser Glu Glu Leu Gly Thr Gln Ile Leu Ile
            1845                1850                1855

His Gln Glu Ser Leu Thr Asp Tyr Cys Ser Met Ser Ser Tyr Ser Ser
        1860                1865                1870

Ser Pro Pro Arg Gln Ala Ala Arg Ser Pro Ser Ser Leu Pro Ser Ser
    1875                1880                1885

Pro Ala Ser Ser Ser Ser Val Pro Phe Ser Thr Asp Cys Glu Asp Ser
1890                1895                1900

Asp Met Leu His Thr Pro Gly Ala Ala Ser Asp Arg Ser Glu His Asp
1905                1910                1915                1920

Leu Thr Pro Met Asp Gly Glu Thr Phe Ser Gln His Leu Gln Ala Val
            1925                1930                1935

Lys Ile Leu Ala Val Arg Asp Leu Ile Trp Val Pro Arg Arg Gly Gly
        1940                1945                1950

Asp Val Ile Val Ile Gly Leu Glu Lys Asp Ser Gly Ala Gln Arg Gly
    1955                1960                1965

Arg Val Ile Ala Val Leu Lys Ala Arg Glu Leu Thr Pro His Gly Val
1970                1975                1980

Leu Val Asp Ala Ala Val Val Ala Lys Asp Thr Val Val Cys Thr Phe
1985                1990                1995                2000

Glu Asn Glu Asn Thr Glu Trp Cys Leu Ala Val Trp Arg Gly Trp Gly
            2005                2010                2015

Ala Arg Glu Phe Asp Ile Phe Tyr Gln Ser Tyr Glu Glu Leu Gly Arg
        2020                2025                2030

Leu Glu Ala Cys Thr Arg Lys Arg Arg
    2035                2040


<210> 5
<211> 7015
<212> DNA
<213> Homo sapiens

<400> 5
   cgaggggacg cctcgcgacg gttcctggga gagctggcgg cggccttgct ctgcgcgctc 60
   ttcgcgccgc cctccccgcc cgcccgcctc aggattgagg aagtgcgtct gggcccggcc 120
   ccggcgcggg gggcagacgg cggtgggacg gccaggcccc ggccccgcca gtgtgtccgc 180
   ccggccccgc gtcccggagg agtcagctgt gtgtccagaa cgtgccatgg agacgcttaa 240
   cggtgccggg gacacgggcg gcaagccgtc cacgcggggc ggtgaccctg cagcgcggtc 300
   ccgcaggacg gaaggcatcc gcgccgcgta caggcgggga gaccgcggcg gcgcccggga 360
   cctgctggag gaggcctgcg accagtgcgc gtcccagctg gaaaagggcc agcttctgag 420
   catcccggca gcctatgggg atctggagat ggtccgctac ctactcagca agagactggt 480
   ggagctgccc accgagccca cggatgacaa cccagccgtg gtggcagcgt attttggaca 540
   cacggcagtt gtgcaggaat tgcttgagtc cttaccaggt ccctgcagtc cccagcggct 600
   tctgaactgg atgctggcct tggcttgcca gcgagggcac ctgggggttg tgaagctcct 660
   ggtcctgacg cacggggctg acccggagag ctacgctgtc aggaagaatg agttccctgt 720
   catcgtgcgc ttgcccctgt atgcggccat caagtcaggg aatgaagaca ttgcaatatt 780
   cctgcttcgg catggggcct atttctgttc ctacatcttg ctggatagtc ctgaccccag 840
   caaacatctg ctgagaaagt acttcattga agccagtccc ttgcccagca gttatccggg 900
   aaaaacagct ctccgtgtga aatggtccca tctcagactg ccctgggtag acctagactg 960
   gctcatagac atctcctgcc agatcacgga gctcgacctt tctgccaact gcctggcgac 1020
   cctcccctcg gttatcccct ggggcctcat caatctccgg aagctgaacc tctccgacaa 1080
   ccacctgggg gagctgcctg gcgtgcagtc atcggacgaa atcatctgtt ccaggctact 1140
   tgaaattgac atttccagca acaagttgtc ccacctccct cctggattct tgcacctctc 1200
   aaaacttcaa aaactgacag cttcaaaaaa ttgtttagaa aaattgttcg aagaagaaaa 1260
   tgccactaac tggataggtt tacggaagct acaggaactt gatatatctg acaataaatt 1320
   gacagaactc cctgccctgt tccttcactc tttcaagtcc ctcaattctc tgaatgtctc 1380
   cagaaacaac ctgaaggtgt ttccagatcc ctgggcctgc cctttgaaat gttgtaaagc 1440
   ttccagaaat gccctggaat gtctgccaga caaaatggct gtcttttgga aaaatcacct 1500
   gaaggatgtg gatttctcag aaaacgcact caaagaagtt cccctgggac ttttccagct 1560
   tgatgccctc atgttcttga ggttacaggg gaaccagctg gcggcacttc cacctcaaga 1620
   gaagtggacc tgcaggcagc tcaaaaccct ggatctctcc agaaaccaac ttggcaaaaa 1680
   tgaagatgga ctgaaaacga agcgtattgc ctttttcacc accagaggtc gccagcgctc 1740
   cgggactgag gcagagacaa ctatggagtt cagtgcatct ctggtaacca ttgtgttcct 1800
   gtctaacaac tgtaacctct gtgcatacac atgtgcagca agtgtgctgg aatttccggc 1860
   cttcctaagt gagtctttgg aagtcctttg cctgaacgac aaccacctcg acacagtccc 1920
   tccctcggtt tgcctactga agagcttatc agagctctac ttgggaaaca accctggcct 1980
   ccgggagctc cctcctgagc tggggcagct gggcaacctc tggcagctgg acactgaaga 2040
   cctgaccatc agcaatgtgc ctgcagaaat ccaaaaagaa ggccccaaag caatgctgtc 2100
   ttacctgcgt gctcagctgc ggaaagcgga aaagtgcaag ctgatgaaga tgatcatcgt 2160
   gggtcccccg cgccagggca agtccaccct cctggagatc ttacagacgg ggagggcccc 2220
   ccaggtggtg catggagagg ccaccatcag gaccaccaag tgggagctcc agaggccggc 2280
   tggctcgaga gccaaggtca aggatggtct gcgtgcagag tccctgtggg ttgagtccgt 2340
   ggagttcaac gtctgggaca tcgggggacc ggccagcatg gccactgtca accagtgctt 2400
   cttcacggac aaggccctgt acgtggtggt ctggaacctg gcgctggggg aggaggccgt 2460
   ggccaacctc cagttctggc tgctcaacat cgaggccaag gccccaaacg ccgtggtgct 2520
   ggtggtcggg acgcacctgg atttaattga agccaagttc cgtgtggaaa ggattgcaac 2580
   gctgcgtgcc tatgtgctgg cactctgccg ctccccctcc ggctccaggg ccacaggctt 2640
   cccagacatc accttcaaac acttacatga gatttcctgc aagagcctgg aaggtcagga 2700
   agggctgcga cagctgattt tccacgtcac gtgcagcatg aaggacgtcg gcagcaccat 2760
   cggctgccag cgactggcag ggcggctgat ccccaggagc tacctgagcc tgcaggaggc 2820
   cgtgctggca gagcagcagc gccgcagccg ggacgacgac gtgcagtacc tgacggacag 2880
   gcagctggag cagctggtgg agcagacgcc cgacaacgac atcaaggact acgaggacct 2940
   gcagtcagcc atcagcttcc tcatagaaac cggcaccctg ctccatttcc cggacaccag 3000
   ccacggcctg aggaacctct acttcctcga ccctatttgg ctctccgaat gtctgcagag 3060
   gatctttaat attaagggct ctcggtcagt ggccaagaat ggggtgatca gagcagaaga 3120
   cctcaggatg ctgctggtgg ggactggctt cacgcagcag acggaagagc agtacttcca 3180
   gttcctggcc aagtttgaga tcgccctgcc cgtcgccaat gacagctacc tcctgcccca 3240
   tctccttcca tctaaacctg gcctggacac ccacggtatg cggcacccca cagccaacac 3300
   cattcagagg gtatttaaga tgagcttcgt tcccgttggc ttctggcaaa ggtttatagc 3360
   acggatgctg atcagcctgg cggagatgga cctgcagctt tttgaaaaca agaagaatac 3420
   taaaagcagg aacaggaaag tcaccattta cagttttaca ggaaaccaga gaaatcgctg 3480
   tagcacattc agagtgaaaa gaaatcagac catctattgg caggaagggc tcctggtcac 3540
   ttttgatggg ggctacctca gtgtggaatc ttccgacgtg aactggaaaa agaagaaaag 3600
   cggaggaatg aaaattgttt gccaatcaga agtgagggac ttctcagcca tggctttcat 3660
   cacggaccac gtcaattcct tgattgatca gtggtttccc gccctgacag ccacagagag 3720
   cgacgggacg ccactcatgg agcagtacgt gccctgcccg gtctgcgaga cagcctgggc 3780
   ccagcacacg gaccccagtg agaaatcaga ggatgtgcag tacttcgaca tggaagactg 3840
   tgtcctgacg gccatcgagc gggacttcat ctcctgcccc agacacccgg acctccccgt 3900
   gccgctgcag gagctggtcc ctgaactgtt catgaccgac ttcccggcca ggctcttcct 3960
   ggagaacagc aagctggagc acagcgagga cgagggcagc gtcctgggcc agggcggcag 4020
   tggcaccgtc atctaccggg cccggtacca gggccagcct gtggccgtca agcgcttcca 4080
   catcaaaaaa ttcaagaact ttgctaacgt accggcagac accatgctga ggcacctgcg 4140
   ggccaccgat gccatgaaga acttctccga gttccggcag gaggccagca tgctgcacgc 4200
   gctgcagcac ccctgcatcg tggcgctcat cggcatcagc atccacccgc tctgcttcgc 4260
   cctggagctc gcgccgctca gcagcctcaa caccgtgctg tccgagaacg ccagagattc 4320
   ttcctttata cccctgggac acatgctcac ccaaaaaata gcctaccaga tcgcctcggg 4380
   cctggcctac ctgcacaaga aaaacatcat cttctgtgac ctgaagtcgg acaacattct 4440
   ggtgtggtcc cttgacgtca aggagcacat caacatcaag ctatctgact acgggatttc 4500
   gaggcagtca ttccatgagg gcgccctagg cgtggagggc actcctggct accaggcccc 4560
   agagatcagg cctcgcattg tatatgatga gaaggtagat atgttctcct atggaatggt 4620
   gctctacgag ttgctgtcag gacagcgccc tgcactgggc caccaccagc tccagattgc 4680
   caagaagctg tccaagggca tccgcccggt tctggggcag ccggaggaag tgcagttccg 4740
   gcgactgcag gcgctcatga tggagtgctg ggacactaag ccagagaagc gaccgctggc 4800
   cctgtcggtg gtgagccaga tgaaggaccc gacttttgcc accttcatgt atgaactgtg 4860
   ctgtgggaag cagacagcct tcttctcatc ccagggccag gagtacaccg tggtgttttg 4920
   ggatggaaaa gaggagtcca ggaactacac ggtggtgaac acagagaagg gcctcatgga 4980
   ggtgcagagg atgtgctgcc ctgggatgaa ggtgagctgc cagctccagg tccagagatc 5040
   cctgtggaca gccaccgaga attcctacct ggtcttagcg ggcctcgccg atgggcttgt 5100
   ggctgtgttt cccgtggtgc ggggcacccc aaaggacagc tgctcctacc tgtgctcaca 5160
   cacagccaac aggtccaagt tcagcatcgc ggatgaagac gcacggcaga acccctaccc 5220
   agtgaaggcc atggaggtgg tcaacagcgg ctctgaggtc tggtacagca atgggccggg 5280
   cctccttgtc atcgactgtg cctccctgga gatctgcagg cggctggagc cctacatggc 5340
   cccctccatg gttacgtcag tcgtgtgcag ctctgagggc agaggggagg aggtcgtctg 5400
   gtgcctggat gacaaggcca actccttggt gatgtaccac tccaccacct accagctgtg 5460
   tgcccggtac ttctgcgggg tccccagccc cctcagggac atgtttcccg tgcggccctt 5520
   ggacacggaa cccccggcag ccagccacac ggccaaccca aaggtgcctg agggggactc 5580
   catcgcggac gtgagcatca tgtacagtga ggagctgggc acgcagatcc tgatccacca 5640
   ggaatcactc actgactact gctccatgtc ctcctactcc tcatccccac cccgccaggc 5700
   tgccaggtcc ccctcaagcc tccccagctc cccagcaagt tcttccagtg tgcctttctc 5760
   caccgactgc gaggactcag acatgctaca tacgcccggt gctgcctccg acaggtctga 5820
   gcatgacctg acccccatgg acggggagac cttcagccag cacctgcagg ccgtgaagat 5880
   cctcgccgtc agagacctca tttgggtccc caggcgcggt ggagatgtta tcgtcattgg 5940
   cctggagaag gattctggcg cccagcgggg ccgagtcatt gccgtcttaa aagcccgaga 6000
   gctgactccg catggggtgc tggtggatgc tgccgtggtg gcaaaggaca ctgttgtgtg 6060
   cacctttgaa aatgaaaaca cagagtggtg cctggccgtc tggaggggct ggggcgccag 6120
   ggagttcgac attttctacc agtcctacga ggagctgggc cggctggagg cttgcactcg 6180
   caagagaagg taattcctgt ggaatgactg tcacacatca gagctggctg gcccggggct 6240
   gcagcctgac tcctctgcca tcggcctcta gttctccaag gacctagaag acagatggag 6300
   ttctcccctg aactccttgc tgctaagaag tgctgagaag ttactcgcct ggcggtggct 6360
   ccagggttct ctggttctct ggagcagagt tctctgaata ccccatcccc caactgctga 6420
   ttttacagcc ccagggaaga cagtggtatc aggctgggag cggcctcctc tggcctcccc 6480
   catcagtttg caggagcagg ggtgcaggat cctgttctga gctgggtcaa acaaagcagg 6540
   gccgggcctt cctgccatcc ccaggtctca gatggaatta cactagaggc cctccgctgg 6600
   gaagcacttg aggtagggca ggaggggggc tgtgacccct gccctttccc cgccagagac 6660
   ctcaggctct cagcacattc cacaggctcc tgagtccccg aggcctgggc cagcttgggc 6720
   aagccaagat cagatgtctc tgtgttcggg aaggtctccg tgtgggaaag cccttggggg 6780
   atcccgggtg aggagtgttg ccccatccag agaatgaatg agttccttta agtgccaccg 6840
   ccagcaagcc cagaggcaca cattctgagt gcacccgctt agcctttaca ttcctctcca 6900
   ccgacaaaag gaaggggaaa ctcaatcagc aggacttcag aaaggccctt gtgtttatag 6960
   ctttgtcaag taaatttgga cgcagctgga aacacaggcc tgtttgttgc acata 7015

<210> 6
<211> 1988
<212> PRT
<213> Homo sapiens

<400> 6
Met Glu Thr Leu Asn Gly Ala Gly Asp Thr Gly Gly Lys Pro Ser Thr
1               5                   10                  15

Arg Gly Gly Asp Pro Ala Ala Arg Ser Arg Arg Thr Glu Gly Ile Arg
            20                  25                  30

Ala Ala Tyr Arg Arg Gly Asp Arg Gly Gly Ala Arg Asp Leu Leu Glu
        35                  40                  45

Glu Ala Cys Asp Gln Cys Ala Ser Gln Leu Glu Lys Gly Gln Leu Leu
    50                  55                  60

Ser Ile Pro Ala Ala Tyr Gly Asp Leu Glu Met Val Arg Tyr Leu Leu
65                  70                  75                  80

Ser Lys Arg Leu Val Glu Leu Pro Thr Glu Pro Thr Asp Asp Asn Pro
                85                  90                  95

Ala Val Val Ala Ala Tyr Phe Gly His Thr Ala Val Val Gln Glu Leu
            100                 105                 110

Leu Glu Ser Leu Pro Gly Pro Cys Ser Pro Gln Arg Leu Leu Asn Trp
        115                 120                 125

Met Leu Ala Leu Ala Cys Gln Arg Gly His Leu Gly Val Val Lys Leu
    130                 135                 140

Leu Val Leu Thr His Gly Ala Asp Pro Glu Ser Tyr Ala Val Arg Lys
145                 150                 155                 160

Asn Glu Phe Pro Val Ile Val Arg Leu Pro Leu Tyr Ala Ala Ile Lys
                165                 170                 175

Ser Gly Asn Glu Asp Ile Ala Ile Phe Leu Leu Arg His Gly Ala Tyr
            180                 185                 190

Phe Cys Ser Tyr Ile Leu Leu Asp Ser Pro Asp Pro Ser Lys His Leu
        195                 200                 205

Leu Arg Lys Tyr Phe Ile Glu Ala Ser Pro Leu Pro Ser Ser Tyr Pro
    210                 215                 220

Gly Lys Thr Ala Leu Arg Val Lys Trp Ser His Leu Arg Leu Pro Trp
225                 230                 235                 240

Val Asp Leu Asp Trp Leu Ile Asp Ile Ser Cys Gln Ile Thr Glu Leu
                245                 250                 255

Asp Leu Ser Ala Asn Cys Leu Ala Thr Leu Pro Ser Val Ile Pro Trp
            260                 265                 270

Gly Leu Ile Asn Leu Arg Lys Leu Asn Leu Ser Asp Asn His Leu Gly
        275                 280                 285

Glu Leu Pro Gly Val Gln Ser Ser Asp Glu Ile Ile Cys Ser Arg Leu
    290                 295                 300

Leu Glu Ile Asp Ile Ser Ser Asn Lys Leu Ser His Leu Pro Pro Gly
305                 310                 315                 320

Phe Leu His Leu Ser Lys Leu Gln Lys Leu Thr Ala Ser Lys Asn Cys
                325                 330                 335

Leu Glu Lys Leu Phe Glu Glu Glu Asn Ala Thr Asn Trp Ile Gly Leu
            340                 345                 350

Arg Lys Leu Gln Glu Leu Asp Ile Ser Asp Asn Lys Leu Thr Glu Leu
        355                 360                 365

Pro Ala Leu Phe Leu His Ser Phe Lys Ser Leu Asn Ser Leu Asn Val
    370                 375                 380

Ser Arg Asn Asn Leu Lys Val Phe Pro Asp Pro Trp Ala Cys Pro Leu
385                 390                 395                 400

Lys Cys Cys Lys Ala Ser Arg Asn Ala Leu Glu Cys Leu Pro Asp Lys
                405                 410                 415

Met Ala Val Phe Trp Lys Asn His Leu Lys Asp Val Asp Phe Ser Glu
            420                 425                 430

Asn Ala Leu Lys Glu Val Pro Leu Gly Leu Phe Gln Leu Asp Ala Leu
        435                 440                 445

Met Phe Leu Arg Leu Gln Gly Asn Gln Leu Ala Ala Leu Pro Pro Gln
    450                 455                 460

Glu Lys Trp Thr Cys Arg Gln Leu Lys Thr Leu Asp Leu Ser Arg Asn
465                 470                 475                 480

Gln Leu Gly Lys Asn Glu Asp Gly Leu Lys Thr Lys Arg Ile Ala Phe
                485                 490                 495

Phe Thr Thr Arg Gly Arg Gln Arg Ser Gly Thr Glu Ala Glu Thr Thr
            500                 505                 510

Met Glu Phe Ser Ala Ser Leu Val Thr Ile Val Phe Leu Ser Asn Asn
        515                 520                 525

Cys Asn Leu Cys Ala Tyr Thr Cys Ala Ala Ser Val Leu Glu Phe Pro
    530                 535                 540

Ala Phe Leu Ser Glu Ser Leu Glu Val Leu Cys Leu Asn Asp Asn His
545                 550                 555                 560

Leu Asp Thr Val Pro Pro Ser Val Cys Leu Leu Lys Ser Leu Ser Glu
                565                 570                 575

Leu Tyr Leu Gly Asn Asn Pro Gly Leu Arg Glu Leu Pro Pro Glu Leu
            580                 585                 590

Gly Gln Leu Gly Asn Leu Trp Gln Leu Asp Thr Glu Asp Leu Thr Ile
        595                 600                 605

Ser Asn Val Pro Ala Glu Ile Gln Lys Glu Gly Pro Lys Ala Met Leu
    610                 615                 620

Ser Tyr Leu Arg Ala Gln Leu Arg Lys Ala Glu Lys Cys Lys Leu Met
625                 630                 635                 640

Lys Met Ile Ile Val Gly Pro Pro Arg Gln Gly Lys Ser Thr Leu Leu
                645                 650                 655

Glu Ile Leu Gln Thr Gly Arg Ala Pro Gln Val Val His Gly Glu Ala
            660                 665                 670

Thr Ile Arg Thr Thr Lys Trp Glu Leu Gln Arg Pro Ala Gly Ser Arg
        675                 680                 685

Ala Lys Val Lys Asp Gly Leu Arg Ala Glu Ser Leu Trp Val Glu Ser
    690                 695                 700

Val Glu Phe Asn Val Trp Asp Ile Gly Gly Pro Ala Ser Met Ala Thr
705                 710                 715                 720

Val Asn Gln Cys Phe Phe Thr Asp Lys Ala Leu Tyr Val Val Val Trp
                725                 730                 735

Asn Leu Ala Leu Gly Glu Glu Ala Val Ala Asn Leu Gln Phe Trp Leu
            740                 745                 750

Leu Asn Ile Glu Ala Lys Ala Pro Asn Ala Val Val Leu Val Val Gly
        755                 760                 765

Thr His Leu Asp Leu Ile Glu Ala Lys Phe Arg Val Glu Arg Ile Ala
    770                 775                 780

Thr Leu Arg Ala Tyr Val Leu Ala Leu Cys Arg Ser Pro Ser Gly Ser
785                 790                 795                 800

Arg Ala Thr Gly Phe Pro Asp Ile Thr Phe Lys His Leu His Glu Ile
                805                 810                 815

Ser Cys Lys Ser Leu Glu Gly Gln Glu Gly Leu Arg Gln Leu Ile Phe
            820                 825                 830

His Val Thr Cys Ser Met Lys Asp Val Gly Ser Thr Ile Gly Cys Gln
        835                 840                 845

Arg Leu Ala Gly Arg Leu Ile Pro Arg Ser Tyr Leu Ser Leu Gln Glu
    850                 855                 860

Ala Val Leu Ala Glu Gln Gln Arg Arg Ser Arg Asp Asp Asp Val Gln
865                 870                 875                 880

Tyr Leu Thr Asp Arg Gln Leu Glu Gln Leu Val Glu Gln Thr Pro Asp
                885                 890                 895

Asn Asp Ile Lys Asp Tyr Glu Asp Leu Gln Ser Ala Ile Ser Phe Leu
            900                 905                 910

Ile Glu Thr Gly Thr Leu Leu His Phe Pro Asp Thr Ser His Gly Leu
        915                 920                 925

Arg Asn Leu Tyr Phe Leu Asp Pro Ile Trp Leu Ser Glu Cys Leu Gln
    930                 935                 940

Arg Ile Phe Asn Ile Lys Gly Ser Arg Ser Val Ala Lys Asn Gly Val
945                 950                 955                 960

Ile Arg Ala Glu Asp Leu Arg Met Leu Leu Val Gly Thr Gly Phe Thr
                965                 970                 975

Gln Gln Thr Glu Glu Gln Tyr Phe Gln Phe Leu Ala Lys Phe Glu Ile
            980                 985                 990

Ala Leu Pro Val Ala Asn Asp Ser Tyr Leu Leu Pro His Leu Leu Pro
        995                 1000                1005

Ser Lys Pro Gly Leu Asp Thr His Gly Met Arg His Pro Thr Ala Asn
    1010                1015                1020

Thr Ile Gln Arg Val Phe Lys Met Ser Phe Val Pro Val Gly Phe Trp
1025                1030                1035                1040

Gln Arg Phe Ile Ala Arg Met Leu Ile Ser Leu Ala Glu Met Asp Leu
                1045                1050                1055

Gln Leu Phe Glu Asn Lys Lys Asn Thr Lys Ser Arg Asn Arg Lys Val
            1060                1065                1070

Thr Ile Tyr Ser Phe Thr Gly Asn Gln Arg Asn Arg Cys Ser Thr Phe
        1075                1080                1085

Arg Val Lys Arg Asn Gln Thr Ile Tyr Trp Gln Glu Gly Leu Leu Val
    1090                1095                1100

Thr Phe Asp Gly Gly Tyr Leu Ser Val Glu Ser Ser Asp Val Asn Trp
1105                1110                1115                1120

Lys Lys Lys Lys Ser Gly Gly Met Lys Ile Val Cys Gln Ser Glu Val
                1125                1130                1135

Arg Asp Phe Ser Ala Met Ala Phe Ile Thr Asp His Val Asn Ser Leu
            1140                1145                1150

Ile Asp Gln Trp Phe Pro Ala Leu Thr Ala Thr Glu Ser Asp Gly Thr
        1155                1160                1165

Pro Leu Met Glu Gln Tyr Val Pro Cys Pro Val Cys Glu Thr Ala Trp
    1170                1175                1180

Ala Gln His Thr Asp Pro Ser Glu Lys Ser Glu Asp Val Gln Tyr Phe
1185                1190                1195                1200

Asp Met Glu Asp Cys Val Leu Thr Ala Ile Glu Arg Asp Phe Ile Ser
                1205                1210                1215

Cys Pro Arg His Pro Asp Leu Pro Val Pro Leu Gln Glu Leu Val Pro
            1220                1225                1230

Glu Leu Phe Met Thr Asp Phe Pro Ala Arg Leu Phe Leu Glu Asn Ser
        1235                1240                1245

Lys Leu Glu His Ser Glu Asp Glu Gly Ser Val Leu Gly Gln Gly Gly
    1250                1255                1260

Ser Gly Thr Val Ile Tyr Arg Ala Arg Tyr Gln Gly Gln Pro Val Ala
1265                1270                1275                1280

Val Lys Arg Phe His Ile Lys Lys Phe Lys Asn Phe Ala Asn Val Pro
                1285                1290                1295

Ala Asp Thr Met Leu Arg His Leu Arg Ala Thr Asp Ala Met Lys Asn
            1300                1305                1310

Phe Ser Glu Phe Arg Gln Glu Ala Ser Met Leu His Ala Leu Gln His
        1315                1320                1325

Pro Cys Ile Val Ala Leu Ile Gly Ile Ser Ile His Pro Leu Cys Phe
    1330                1335                1340

Ala Leu Glu Leu Ala Pro Leu Ser Ser Leu Asn Thr Val Leu Ser Glu
1345                1350                1355                1360

Asn Ala Arg Asp Ser Ser Phe Ile Pro Leu Gly His Met Leu Thr Gln
                1365                1370                1375

Lys Ile Ala Tyr Gln Ile Ala Ser Gly Leu Ala Tyr Leu His Lys Lys
            1380                1385                1390

Asn Ile Ile Phe Cys Asp Leu Lys Ser Asp Asn Ile Leu Val Trp Ser
        1395                1400                1405

Leu Asp Val Lys Glu His Ile Asn Ile Lys Leu Ser Asp Tyr Gly Ile
    1410                1415                1420

Ser Arg Gln Ser Phe His Glu Gly Ala Leu Gly Val Glu Gly Thr Pro
1425                1430                1435                1440

Gly Tyr Gln Ala Pro Glu Ile Arg Pro Arg Ile Val Tyr Asp Glu Lys
                1445                1450                1455

Val Asp Met Phe Ser Tyr Gly Met Val Leu Tyr Glu Leu Leu Ser Gly
            1460                1465                1470

Gln Arg Pro Ala Leu Gly His His Gln Leu Gln Ile Ala Lys Lys Leu
        1475                1480                1485

Ser Lys Gly Ile Arg Pro Val Leu Gly Gln Pro Glu Glu Val Gln Phe
    1490                1495                1500

Arg Arg Leu Gln Ala Leu Met Met Glu Cys Trp Asp Thr Lys Pro Glu
1505                1510                1515                1520

Lys Arg Pro Leu Ala Leu Ser Val Val Ser Gln Met Lys Asp Pro Thr
                1525                1530                1535

Phe Ala Thr Phe Met Tyr Glu Leu Cys Cys Gly Lys Gln Thr Ala Phe
            1540                1545                1550

Phe Ser Ser Gln Gly Gln Glu Tyr Thr Val Val Phe Trp Asp Gly Lys
        1555                1560                1565

Glu Glu Ser Arg Asn Tyr Thr Val Val Asn Thr Glu Lys Gly Leu Met
    1570                1575                1580

Glu Val Gln Arg Met Cys Cys Pro Gly Met Lys Val Ser Cys Gln Leu
1585                1590                1595                1600

Gln Val Gln Arg Ser Leu Trp Thr Ala Thr Glu Asn Ser Tyr Leu Val
                1605                1610                1615

Leu Ala Gly Leu Ala Asp Gly Leu Val Ala Val Phe Pro Val Val Arg
            1620                1625                1630

Gly Thr Pro Lys Asp Ser Cys Ser Tyr Leu Cys Ser His Thr Ala Asn
        1635                1640                1645

Arg Ser Lys Phe Ser Ile Ala Asp Glu Asp Ala Arg Gln Asn Pro Tyr
    1650                1655                1660

Pro Val Lys Ala Met Glu Val Val Asn Ser Gly Ser Glu Val Trp Tyr
1665                1670                1675                1680

Ser Asn Gly Pro Gly Leu Leu Val Ile Asp Cys Ala Ser Leu Glu Ile
                1685                1690                1695

Cys Arg Arg Leu Glu Pro Tyr Met Ala Pro Ser Met Val Thr Ser Val
            1700                1705                1710

Val Cys Ser Ser Glu Gly Arg Gly Glu Glu Val Val Trp Cys Leu Asp
        1715                1720                1725

Asp Lys Ala Asn Ser Leu Val Met Tyr His Ser Thr Thr Tyr Gln Leu
    1730                1735                1740

Cys Ala Arg Tyr Phe Cys Gly Val Pro Ser Pro Leu Arg Asp Met Phe
1745                1750                1755                1760

Pro Val Arg Pro Leu Asp Thr Glu Pro Pro Ala Ala Ser His Thr Ala
                1765                1770                1775

Asn Pro Lys Val Pro Glu Gly Asp Ser Ile Ala Asp Val Ser Ile Met
        1780                1785                1790

Tyr Ser Glu Glu Leu Gly Thr Gln Ile Leu Ile His Gln Glu Ser Leu
    1795                1800                1805

Thr Asp Tyr Cys Ser Met Ser Ser Tyr Ser Ser Ser Pro Pro Arg Gln
  1810                1815                1820

Ala Ala Arg Ser Pro Ser Ser Leu Pro Ser Ser Pro Ala Ser Ser Ser
1825                1830                1835                1840

Ser Val Pro Phe Ser Thr Asp Cys Glu Asp Ser Asp Met Leu His Thr
                1845                1850                1855

Pro Gly Ala Ala Ser Asp Arg Ser Glu His Asp Leu Thr Pro Met Asp
        1860                1865                1870

Gly Glu Thr Phe Ser Gln His Leu Gln Ala Val Lys Ile Leu Ala Val
    1875                1880                1885

Arg Asp Leu Ile Trp Val Pro Arg Arg Gly Gly Asp Val Ile Val Ile
  1890                1895                1900

Gly Leu Glu Lys Asp Ser Gly Ala Gln Arg Gly Arg Val Ile Ala Val
1905                1910                1915                1920

Leu Lys Ala Arg Glu Leu Thr Pro His Gly Val Leu Val Asp Ala Ala
                1925                1930                1935

Val Val Ala Lys Asp Thr Val Val Cys Thr Phe Glu Asn Glu Asn Thr
        1940                1945                1950

Glu Trp Cys Leu Ala Val Trp Arg Gly Trp Gly Ala Arg Glu Phe Asp
    1955                1960                1965

Ile Phe Tyr Gln Ser Tyr Glu Glu Leu Gly Arg Leu Glu Ala Cys Thr
  1970                1975                1980

Arg Lys Arg Arg
1985

<210> 7
<211> 7229
<212> DNA
<213> Homo sapiens

<400> 7
gcggccgccg aggggacgcc tcgtgacggt tcctgggaga gctggcggcg gccttgctct 60
gcgcgctctt cgcgccgccc tccccgcccg cccgcctcag gattgaggaa gtgcgtctgg 120
gcccggcccc ggcgcggggg gcagacggcg gtgggacggc caggccccgg ccccgccagt 180
gtgtccgccc ggccccgcgt cccggagcgc ccgcacccgg ccccgccgcc gcctcagagc 240
aagaaagctt tctgctcagc catggctacg agtccacgcc ttaatgcacc ccacagccag 300
cggcagtggc agtgacaaca gcgggacctg cctttgaaga tcggctgctg caagggttga 360
tggctggcat gtcgcaaaga ccccccagca tgtactggtg tgtggggccg gaggagtcag 420
ctgtgtgtcc agaacgtgcc atggagacgc ttaacggtgc cggggacacg ggcggcaagc 480
cgtccacgcg gggcggtgac cctgcagcgc ggtcccgcag gacggaaggc atccgcgccg 540
cgtacaggcg gggagaccgc ggcggcgccc gggacctgct ggaggaggcc tgcgaccagt 600
gcgcgtccca gctggaaaag ggccagcttc tgagcatccc ggcagcctat ggggatctgg 660
agatggtccg ctacctactc agcaagagac tggtggagct gcccaccgag cccacggatg 720
acaacccagc cgtggtggca gcgtattttg gacacacggc agttgtgcag gaattgcttg 780
agtccttacc aggtccctgc agtccccagc ggcttctgaa ctggatgctg gccttggctt 840

gccagcgagg gcacctgggg gttgtgaagc tcctggtcct gacgcacggg gctgacccgg 900
agagctacgc tgtcaggaag aatgagttcc ctgtcatcgt gcgcttgccc ctgtatgcgg 960
ccatcaagtc agggaatgaa gacattgcaa tattcctgct tcggcatggg gcctatttct 1020
gttcctacat cttgctggat agtcctgacc ccagcaaaca tctgctgaga aagtacttca 1080
ttgaagccag tcccttgccc agcagttatc cgggaaaaac agctctccgt gtgaaatggt 1140
cccatctcag actgccctgg gtagacctag actggctcat agacatctcc tgccagatca 1200
cggagctcga cctttctgcc aactgcctgg cgaccctccc ctcggttatc cctgggggcc 1260
tcatcaatct ccggaagctg aacctctccg acaaccacct gggggagctg cctggcgtgc 1320
agtcatcgga cgaaatcatc tgttccaggc tacttgaaat tgacatttcc agcaacaagt 1380
tgtcccacct ccctcctgga ttcttgcacc tctcaaaact tcaaaaactg acagcttcaa 1440
aaaattgttt agaaaaattg ttcgaagaag aaaatgccac taactggata ggtttacgga 1500
agctacagga acttgatata tctgacaata aattgacaga actccctgcc ctgttccttc 1560
actctttcaa gtccctcaat tctctgaatg tctccagaaa caacctgaag gtgtttccag 1620
atccctgggc ctgccctttg aaatgttgta aagcttccag aaatgccctg gaatgtctgc 1680
cagacaaaat ggctgtcttt tggaaaaatc acctgaagga tgtggatttc tcagaaaacg 1740
cactcaaaga agttcccctg ggacttttcc agcttgatgc cctcatgttc ttgaggttac 1800
aggggaacca gctggcggca cttccacctc aagagaagtg gacctgcagg cagctcaaaa 1860
ccctggatct ctccagaaac caacttggca aaaatgaaga tggactgaaa acgaagcgta 1920
ttgccttttt caccaccaga ggtcgccagc gctccgggac tgaggcagag acaactatgg 1980
agttcagtgc atctctggta accattgtgt tcctgtctaa caactgtaac ctctgtgcat 2040
acacatgtgc agcaagtgtg ctggaatttc cggccttcct aagtgagtct ttggaagtcc 2100
tttgcctgaa cgacaaccac ctcgacacag tccctccctc ggtttgccta ctgaagagct 2160
tatcagagct ctacttggga aacaaccctg gcctccggga gctccctcct gagctggggc 2220
agctgggcaa cctctggcag ctggacactg aagacctgac catcagcaat gtgcctgcag 2280
aaatccaaaa agaaggcccc aaagcaatgc tgtcttacct gcgtgctcag ctgcggaaag 2340
cggaaaagtg caagctgatg aagatgatca tcgtgggtcc cccgcgccag ggcaagtcca 2400
ccctcctgga gatcttacag acggggaggg ccccccaggt ggtgcatgga gaggccacca 2460
tcaggaccac caagtgggag ctccagaggc cggctggctc gagagccaag gtcaaggatg 2520
gtctgcgtgc agagtccctg tgggttgagt ccgtggagtt caacgtctgg gacatcgggg 2580
gaccggccag catggccact gtcaaccagt gcttcttcac ggacaaggcc ctgtacgtgg 2640
tggtctggaa cctggcgctg ggggaggagg ccgtggccaa cctccagttc tggctgctca 2700
acatcgaggc caaggcccca aacgccgtgg tgctggtggt cgggacgcac ctggatttaa 2760
ttgaagccaa gttccgtgtg gaaaggattg caacgctgcg tgcctatgtg ctggcactct 2820
gccgctcccc ctccggctcc agggccacag gcttcccaga catcaccttc aaacacttac 2880
atgagatttc ctgcaagagc ctggaaggtc aggaagggct gcgacagctg attttccacg 2940
tcacgtgcag catgaaggac gtcggcagca ccatcggctg ccagcgactg gcagggcggc 3000
tgatccccag gagctacctg agcctgcagg aggccgtgct ggcagagcag cagcgccgca 3060
gccgggacga cgacgtgcag tacctgacgg acaggcagct ggagcagctg gtggagcaga 3120
cgcccgacaa cgacatcaag gactacgagg acctgcagtc agccatcagc ttcctcatag 3180
aaaccggcac cctgctccat ttcccggaca ccagccacgg cctgaggaac ctctacttcc 3240
tcgaccctat ttggctctcc gaatgtctgc agaggatctt taatattaag ggctctcggt 3300
cagtggccaa gaatggggtg atcagagcag aagacctcag gatgctgctg gtggggactg 3360
gcttcacgca gcagacggaa gagcagtact tccagttcct ggccaagttt gagatcgccc 3420
tgcccgtcgc caatgacagc tacctcctgc cccatctcct tccatctaaa cctggcctgg 3480
acacccacgg tatgcggcac cccacagcca acaccattca gagggtattt aagatgagct 3540
tcgttcccgt tggcttctgg caaaggttta tagcacggat gctgatcagc ctggcggaga 3600
tggacctgca gctttttgaa aacaagaaga atactaaaag caggaacagg aaagtcacca 3660
tttacagttt tacaggaaac cagagaaatc gctgtagcac attcagagtg aaaagaaatc 3720
agaccatcta ttggcaggaa gggctcctgg tcacttttga tgggggctac ctcagtgtgg 3780
aatcttccga cgtgaactgg aaaaagaaga aaagcggagg aatgaaaatt gtttgccaat 3840
cagaagtgag ggacttctca gccatggctt tcatcacgga ccacgtcaat tccttgattg 3900
atcagtggtt tcccgccctg acagccacag agagcgacgg gacgccactc atggagcagt 3960
acgtgccctg cccggtctgc gagacagcct gggcccagca cacggacccc agtgagaaat 4020
cagaggatgt gcagtacttc gacatggaag actgtgtcct gacggccatc gagcgggact 4080
tcatctcctg ccccagacac ccggacctcc ccgtgccgct gcaggagctg gtccctgaac 4140
tgttcatgac cgacttcccg gccaggctct tcctggagaa cagcaagctg gagcacagcg 4200
aggacgaggg cagcgtcctg ggccagggcg gcagtggcac cgtcatctac cgggcccggt 4260
accaggccca gcctgtggcc gtcaagcgct tccacatcaa aaaattcaag aactttgcta 4320
acgtaccggc agacaccatg ctgaggcacc tgcggccacc cgatgccatg aagaacttct 4380
ccgagttccg gcaggaggcc agcatgctgc acgcgctgca gcacccctgc atcgtggcgc 4440
tcatcggcat cagcatccac ccgctctgct tcgccctgga gctcgcgccg ctcagcagcc 4500
tcaacaccgt gctgtccgag aacgccagag attcttcctt tatacccctg ggacacatgc 4560
tcacccaaaa aatagcctac cagatcgcct cgggcctggc ctacctgcac aagaaaaaca 4620

tcatcttctg tgacctgaag tcggacaaca ttctggtgtg gtcccttgac gtcaaggagc 4680
acatcaacat caagctatct gactacggga tttcgaggca gtcattccat gagggcgccc 4740
taggcgtgga gggcactcct ggctaccagg ccccagagat caggcctcgc attgtatatg 4800
atgagaaggt agatatgttc tcctatggaa tggtgctcta cgagttgctg tcaggacagc 4860
gccctgcact gggccaccac cagctccaga ttgccaagaa gctgtccaag ggcatccgcc 4920
cggttctggg gcagccggag gaagtgcagt tccggcgact gcaggcgctc atgatggagt 4980
gctgggacac taagccagag aagcgaccgc tggccctgtc ggtggtgagc cagatgaagg 5040
acccgacttt tgccaccttc atgtatgaac tgtgctgtgg gaagcagaca gccttcttct 5100
catcccaggg ccaggagtac accgtggtgt tttgggatgg aaaagaggag tccaggaact 5160
acacggtggt gaacacagag aagggcctca tggaggtgca gaggatgtgc tgccctggga 5220
tgaaggtgag ctgccagctc caggtccaga gatccctgtg gacagccacc gagaattcct 5280
acctggtctt agcgggcctc gccgatgggc ttgtggctgt gtttcccgtg gtgcggggca 5340
ccccaaagga cagctgctcc tacctgtgct cacacacagc caacaggtcc aagttcagca 5400
tcgcggatga agacgcacgg cagaacccct acccagtgaa ggccatggag gtggtcaaca 5460
gcggctctga ggtctggtac agcaatgggc cgggcctcct tgtcatcgac tgtgcctccc 5520
tggagatctg caggcggctg gagccctaca tggccccctc catggttacg tcagtcgtgt 5580
gcagctctga gggcagaggg gaggaggtcg tctggtgcct ggatgacaag gccaactcct 5640
tggtgatgta ccactccacc acctaccagc tgtgtgcccg gtacttctgc ggggtcccca 5700
gccccctcag ggacatgttt cccgtgcggc ccttggacac ggaacccccg gcagccagcc 5760
acacggccaa cccaaaggtg cctgaggggg actccatcgc ggacgtgagc atcatgtaca 5820
gtgaggagct gggcacgcag atcctgatcc accaggaatc actcactgac tactgctcca 5880
tgtcctccta ctcctcatcc ccaccccgcc aggctgccag gtccccctca agcctcccca 5940
gctccccagc aagttcttcc agtgtgcctt tctccaccga ctgcgaggac tcagacatgc 6000
tacatacgcc cggtgctgcc tccgacaggt ctgagcatga cctgaccccc atggacgggg 6060
agaccttcag ccagcacctg caggccgtga agatcctcgc cgtcagagac ctcatttggg 6120
tccccaggcg cggtggagat gttatcgtca ttggcctgga gaaggattct ggcgcccagc 6180
ggggccgagt cattgccgtc ttaaaagccc gagagctgac tccgcatggg gtgctggtgg 6240
atgctgccgt ggtggcaaag gacactgttg tgtgcacctt tgaaaatgaa aacacagagt 6300
ggtgcctggc cgtctggagg ggctggggcg ccagggagtt cgacattttc taccagtcct 6360
acgaggagct gggccggctg gaggcttgca ctcgcaagag aaggtaattc ctgtggaatg 6420
actgtcacac atcagagctg gctggcccgg ggctgcagcc tgactcctct gccatcggcc 6480
tctagttctc caaggaccta gaagacagat ggagttctcc cctgaactcc ttgctgctaa 6540
gaagtgctga gaagttactc gcctggcggt ggctccaggg ttctctggtt ctctggagca 6600
gagttctctg aataccccat cccccaactg ctgattttac agccccaggg aagacagtgg 6660
tatcaggctg ggagcggcct cctctggcct cccccatcag tttgcaggag caggggtgca 6720
ggatcctgtt ctgagctggg tcaaacaaag cagggccggg ccttcctgcc atccccaggt 6780
ctcagatgga attacactag aggccctccg ctgggaagca cttgaggtag ggcaggaggg 6840
gggctgtgac cctgcccttt tccccgccag agacctcagg ctctcagcac attccacagg 6900
ctcctgagtc cccgaggcct gggccagctt gggcaagcca agatcagatg tctctgtgtt 6960
cgggaaggtc tccgtgtggg aaagcccttg ggggatcccg ggtgaggagt gttgccccat 7020
ccagagaatg aatgagttcc tttaagtgcc accgccagca agcccagagg cacacattct 7080
gagtgcaccc gcttagcctt tacattcctc tccaccgaca aaaggaaggg gaaactcaat 7140
cagcaggact tcagaaaggg ccttgtgttt atagctttgt caagtaaatt tggacgcagc 7200
tggaaacaca ggcctgtttg ttgcacata 7229

<210> 8
<211> 2015
<212> PRT
<213> Homo sapiens

<400> 8
Met Ala Gly Met Ser Gln Arg Pro Pro Ser Met Tyr Trp Cys Val Gly
1               5                   10                  15

Pro Glu Glu Ser Ala Val Cys Pro Glu Arg Ala Met Glu Thr Leu Asn
            20                  25                  30

Gly Ala Gly Asp Thr Gly Gly Lys Pro Ser Thr Arg Gly Gly Asp Pro
        35                  40                  45

Ala Ala Arg Ser Arg Arg Thr Glu Gly Ile Arg Ala Ala Tyr Arg Arg
    50                  55                  60

Gly Asp Arg Gly Gly Ala Arg Asp Leu Leu Glu Glu Ala Cys Asp Gln
65                  70                  75                  80

Cys Ala Ser Gln Leu Glu Lys Gly Gln Leu Leu Ser Ile Pro Ala Ala
                85                  90                  95

Tyr Gly Asp Leu Glu Met Val Arg Tyr Leu Leu Ser Lys Arg Leu Val
            100                 105                 110

Glu Leu Pro Thr Glu Pro Thr Asp Asp Asn Pro Ala Val Val Ala Ala
        115                 120                 125

Tyr Phe Gly His Thr Ala Val Val Gln Glu Leu Leu Glu Ser Leu Pro
    130                 135                 140

Gly Pro Cys Ser Pro Gln Arg Leu Leu Asn Trp Met Leu Ala Leu Ala
145                 150                 155                 160

Cys Gln Arg Gly His Leu Gly Val Val Lys Leu Leu Val Leu Thr His
                165                 170                 175

Gly Ala Asp Pro Glu Ser Tyr Ala Val Arg Lys Asn Glu Phe Pro Val
            180                 185                 190

Ile Val Arg Leu Pro Leu Tyr Ala Ala Ile Lys Ser Gly Asn Glu Asp
        195                 200                 205

Ile Ala Ile Phe Leu Leu Arg His Gly Ala Tyr Phe Cys Ser Tyr Ile
    210                 215                 220

Leu Leu Asp Ser Pro Asp Pro Ser Lys His Leu Leu Arg Lys Tyr Phe
225                 230                 235                 240

Ile Glu Ala Ser Pro Leu Pro Ser Ser Tyr Pro Gly Lys Thr Ala Leu
                245                 250                 255

Arg Val Lys Trp Ser His Leu Arg Leu Pro Trp Val Asp Leu Asp Trp
            260                 265                 270

Leu Ile Asp Ile Ser Cys Gln Ile Thr Glu Leu Asp Leu Ser Ala Asn
        275                 280                 285

Cys Leu Ala Thr Leu Pro Ser Val Ile Pro Trp Gly Leu Ile Asn Leu
    290                 295                 300

Arg Lys Leu Asn Leu Ser Asp Asn His Leu Gly Glu Leu Pro Gly Val
305                 310                 315                 320

Gln Ser Ser Asp Glu Ile Ile Cys Ser Arg Leu Leu Glu Ile Asp Ile
                325                 330                 335

Ser Ser Asn Lys Leu Ser His Leu Pro Pro Gly Phe Leu His Leu Ser
            340                 345                 350

Lys Leu Gln Lys Leu Thr Ala Ser Lys Asn Cys Leu Glu Lys Leu Phe
        355                 360                 365

Glu Glu Glu Asn Ala Thr Asn Trp Ile Gly Leu Arg Lys Leu Gln Glu
    370                 375                 380

Leu Asp Ile Ser Asp Asn Lys Leu Thr Glu Leu Pro Ala Leu Phe Leu
385                 390                 395                 400

His Ser Phe Lys Ser Leu Asn Ser Leu Asn Val Ser Arg Asn Asn Leu
                405                 410                 415

Lys Val Phe Pro Asp Pro Trp Ala Cys Pro Leu Lys Cys Cys Lys Ala
            420                 425                 430

Ser Arg Asn Ala Leu Glu Cys Leu Pro Asp Lys Met Ala Val Phe Trp
        435                 440                 445

Lys Asn His Leu Lys Asp Val Asp Phe Ser Glu Asn Ala Leu Lys Glu
    450                 455                 460

Val Pro Leu Gly Leu Phe Gln Leu Asp Ala Leu Met Phe Leu Arg Leu
465                 470                 475                 480

Gln Gly Asn Gln Leu Ala Ala Leu Pro Pro Gln Glu Lys Trp Thr Cys
                485                 490                 495

Arg Gln Leu Lys Thr Leu Asp Leu Ser Arg Asn Gln Leu Gly Lys Asn
            500                 505                 510

Glu Asp Gly Leu Lys Thr Lys Arg Ile Ala Phe Phe Thr Thr Arg Gly
        515                 520                 525

Arg Gln Arg Ser Gly Thr Glu Ala Glu Thr Thr Met Glu Phe Ser Ala
    530                 535                 540

Ser Leu Val Thr Ile Val Phe Leu Ser Asn Asn Cys Asn Leu Cys Ala
545                 550                 555                 560

Tyr Thr Cys Ala Ala Ser Val Leu Glu Phe Pro Ala Phe Leu Ser Glu
                565                 570                 575

Ser Leu Glu Val Leu Cys Leu Asn Asp Asn His Leu Asp Thr Val Pro
            580                 585                 590

Pro Ser Val Cys Leu Leu Lys Ser Leu Ser Glu Leu Tyr Leu Gly Asn
        595                 600                 605

Asn Pro Gly Leu Arg Glu Leu Pro Pro Glu Leu Gly Gln Leu Gly Asn
    610                 615                 620

Leu Trp Gln Leu Asp Thr Glu Asp Leu Thr Ile Ser Asn Val Pro Ala
625                 630                 635                 640

Glu Ile Gln Lys Glu Gly Pro Lys Ala Met Leu Ser Tyr Leu Arg Ala
                645                 650                 655

Gln Leu Arg Lys Ala Glu Lys Cys Lys Leu Met Lys Met Ile Ile Val
            660                 665                 670

Gly Pro Pro Arg Gln Gly Lys Ser Thr Leu Leu Glu Ile Leu Gln Thr
        675                 680                 685

Gly Arg Ala Pro Gln Val Val His Gly Glu Ala Thr Ile Arg Thr Thr
    690                 695                 700

Lys Trp Glu Leu Gln Arg Pro Ala Gly Ser Arg Ala Lys Val Lys Asp
705                 710                 715                 720

Gly Leu Arg Ala Glu Ser Leu Trp Val Glu Ser Val Glu Phe Asn Val
                725                 730                 735

Trp Asp Ile Gly Gly Pro Ala Ser Met Ala Thr Val Asn Gln Cys Phe
            740                 745                 750

Phe Thr Asp Lys Ala Leu Tyr Val Val Val Trp Asn Leu Ala Leu Gly
        755                 760                 765

Glu Glu Ala Val Ala Asn Leu Gln Phe Trp Leu Leu Asn Ile Glu Ala
    770                 775                 780

Lys Ala Pro Asn Ala Val Val Leu Val Val Gly Thr His Leu Asp Leu
785                 790                 795                 800

Ile Glu Ala Lys Phe Arg Val Glu Arg Ile Ala Thr Leu Arg Ala Tyr
                805                 810                 815

Val Leu Ala Leu Cys Arg Ser Pro Ser Gly Ser Arg Ala Thr Gly Phe
            820                 825                 830

Pro Asp Ile Thr Phe Lys His Leu His Glu Ile Ser Cys Lys Ser Leu
        835                 840                 845

Glu Gly Gln Glu Gly Leu Arg Gln Leu Ile Phe His Val Thr Cys Ser
    850                 855                 860

Met Lys Asp Val Gly Ser Thr Ile Gly Cys Gln Arg Leu Ala Gly Arg
865                 870                 875                 880

Leu Ile Pro Arg Ser Tyr Leu Ser Leu Gln Glu Ala Val Leu Ala Glu
                885                 890                 895

Gln Gln Arg Arg Ser Arg Asp Asp Asp Val Gln Tyr Leu Thr Asp Arg
            900                 905                 910

Gln Leu Glu Gln Leu Val Glu Gln Thr Pro Asp Asn Asp Ile Lys Asp
        915                 920                 925

Tyr Glu Asp Leu Gln Ser Ala Ile Ser Phe Leu Ile Glu Thr Gly Thr
    930                 935                 940

Leu Leu His Phe Pro Asp Thr Ser His Gly Leu Arg Asn Leu Tyr Phe
945                 950                 955                 960

Leu Asp Pro Ile Trp Leu Ser Glu Cys Leu Gln Arg Ile Phe Asn Ile
                965                 970                 975

Lys Gly Ser Arg Ser Val Ala Lys Asn Gly Val Ile Arg Ala Glu Asp
            980                 985                 990

Leu Arg Met Leu Leu Val Gly Thr Gly Phe Thr Gln Gln Thr Glu Glu
        995                 1000                1005

Gln Tyr Phe Gln Phe Leu Ala Lys Phe Glu Ile Ala Leu Pro Val Ala
    1010                1015                1020

Asn Asp Ser Tyr Leu Leu Pro His Leu Leu Pro Ser Lys Pro Gly Leu
1025                1030                1035                1040

Asp Thr His Gly Met Arg His Pro Thr Ala Asn Thr Ile Gln Arg Val
                1045                1050                1055

Phe Lys Met Ser Phe Val Pro Val Gly Phe Trp Gln Arg Phe Ile Ala
            1060                1065                1070

Arg Met Leu Ile Ser Leu Ala Glu Met Asp Leu Gln Leu Phe Glu Asn
        1075                1080                1085

Lys Lys Asn Thr Lys Ser Arg Asn Arg Lys Val Thr Ile Tyr Ser Phe
    1090                1095                1100

Thr Gly Asn Gln Arg Asn Arg Cys Ser Thr Phe Arg Val Lys Arg Asn
1105                1110                1115                1120

Gln Thr Ile Tyr Trp Gln Glu Gly Leu Leu Val Thr Phe Asp Gly Gly
                1125                1130                1135

Tyr Leu Ser Val Glu Ser Ser Asp Val Asn Trp Lys Lys Lys Lys Ser
            1140                1145                1150

Gly Gly Met Lys Ile Val Cys Gln Ser Glu Val Arg Asp Phe Ser Ala
        1155                1160                1165

Met Ala Phe Ile Thr Asp His Val Asn Ser Leu Ile Asp Gln Trp Phe
    1170                1175                1180

Pro Ala Leu Thr Ala Thr Glu Ser Asp Gly Thr Pro Leu Met Glu Gln
1185                1190                1195                1200

Tyr Val Pro Cys Pro Val Cys Glu Thr Ala Trp Ala Gln His Thr Asp
                1205                1210                1215

Pro Ser Glu Lys Ser Glu Asp Val Gln Tyr Phe Asp Met Glu Asp Cys
            1220                1225                1230

Val Leu Thr Ala Ile Glu Arg Asp Phe Ile Ser Cys Pro Arg His Pro
        1235                1240                1245

Asp Leu Pro Val Pro Leu Gln Glu Leu Val Pro Glu Leu Phe Met Thr
    1250                1255                1260

Asp Phe Pro Ala Arg Leu Phe Leu Glu Asn Ser Lys Leu Glu His Ser
1265                1270                1275                1280

Glu Asp Glu Gly Ser Val Leu Gly Gln Gly Gly Ser Gly Thr Val Ile
                1285                1290                1295

Tyr Arg Ala Arg Tyr Gln Gly Gln Pro Val Ala Val Lys Arg Phe His
            1300                1305                1310

Ile Lys Lys Phe Lys Asn Phe Ala Asn Val Pro Ala Asp Thr Met Leu
        1315                1320                1325

Arg His Leu Arg Ala Thr Asp Ala Met Lys Asn Phe Ser Glu Phe Arg
    1330                1335                1340

Gln Glu Ala Ser Met Leu His Ala Leu Gln His Pro Cys Ile Val Ala
1345                1350                1355                1360

Leu Ile Gly Ile Ser Ile His Pro Leu Cys Phe Ala Leu Glu Leu Ala
                1365                1370                1375

Pro Leu Ser Ser Leu Asn Thr Val Leu Ser Glu Asn Ala Arg Asp Ser
            1380                1385                1390

Ser Phe Ile Pro Leu Gly His Met Leu Thr Gln Lys Ile Ala Tyr Gln
        1395                1400                1405

Ile Ala Ser Gly Leu Ala Tyr Leu His Lys Lys Asn Ile Ile Phe Cys
1410                1415                1420

Asp Leu Lys Ser Asp Asn Ile Leu Val Trp Ser Leu Asp Val Lys Glu
1425                1430                1435                1440

His Ile Asn Ile Lys Leu Ser Asp Tyr Gly Ile Ser Arg Gln Ser Phe
                1445                1450                1455

His Glu Gly Ala Leu Gly Val Glu Gly Thr Pro Gly Tyr Gln Ala Pro
            1460                1465                1470

Glu Ile Arg Pro Arg Ile Val Tyr Asp Glu Lys Val Asp Met Phe Ser
        1475                1480                1485

Tyr Gly Met Val Leu Tyr Glu Leu Leu Ser Gly Gln Arg Pro Ala Leu
    1490                1495                1500

Gly His His Gln Leu Gln Ile Ala Lys Lys Leu Ser Lys Gly Ile Arg
1505                1510                1515                1520

Pro Val Leu Gly Gln Pro Glu Glu Val Gln Phe Arg Arg Leu Gln Ala
                1525                1530                1535

Leu Met Met Glu Cys Trp Asp Thr Lys Pro Glu Lys Arg Pro Leu Ala
            1540                1545                1550

Leu Ser Val Val Ser Gln Met Lys Asp Pro Thr Phe Ala Thr Phe Met
        1555                1560                1565

Tyr Glu Leu Cys Cys Gly Lys Gln Thr Ala Phe Phe Ser Ser Gln Gly
    1570                1575                1580

Gln Glu Tyr Thr Val Val Phe Trp Asp Gly Lys Glu Glu Ser Arg Asn
1585                1590                1595                1600

Tyr Thr Val Val Asn Thr Glu Lys Gly Leu Met Glu Val Gln Arg Met
                1605                1610                1615

Cys Cys Pro Gly Met Lys Val Ser Cys Gln Leu Gln Val Gln Arg Ser
            1620                1625                1630

Leu Trp Thr Ala Thr Glu Asn Ser Tyr Leu Val Leu Ala Gly Leu Ala
        1635                1640                1645

Asp Gly Leu Val Ala Val Phe Pro Val Val Arg Gly Thr Pro Lys Asp
    1650                1655                1660

Ser Cys Ser Tyr Leu Cys Ser His Thr Ala Asn Arg Ser Lys Phe Ser
1665                1670                1675                1680

Ile Ala Asp Glu Asp Ala Arg Gln Asn Pro Tyr Pro Val Lys Ala Met
                1685                1690                1695

Glu Val Val Asn Ser Gly Ser Glu Val Trp Tyr Ser Asn Gly Pro Gly
            1700                1705                1710

Leu Leu Val Ile Asp Cys Ala Ser Leu Glu Ile Cys Arg Arg Leu Glu
        1715                1720                1725

Pro Tyr Met Ala Pro Ser Met Val Thr Ser Val Val Cys Ser Ser Glu
    1730                1735                1740

Gly Arg Gly Glu Glu Val Val Trp Cys Leu Asp Asp Lys Ala Asn Ser
1745                1750                1755                1760

Leu Val Met Tyr His Ser Thr Thr Tyr Gln Leu Cys Ala Arg Tyr Phe
                1765                1770                1775

Cys Gly Val Pro Ser Pro Leu Arg Asp Met Phe Pro Val Arg Pro Leu
            1780                1785                1790

Asp Thr Glu Pro Pro Ala Ala Ser His Thr Ala Asn Pro Lys Val Pro
        1795                1800                1805

Glu Gly Asp Ser Ile Ala Asp Val Ser Ile Met Tyr Ser Glu Glu Leu
    1810                1815                1820

Gly Thr Gln Ile Leu Ile His Gln Glu Ser Leu Thr Asp Tyr Cys Ser
1825                1830                1835                1840

Met Ser Ser Tyr Ser Ser Ser Pro Pro Arg Gln Ala Ala Arg Ser Pro
                1845                1850                1855

Ser Ser Leu Pro Ser Ser Pro Ala Ser Ser Ser Ser Val Pro Phe Ser
            1860                1865                1870

Thr Asp Cys Glu Asp Ser Asp Met Leu His Thr Pro Gly Ala Ala Ser
        1875                1880                1885

Asp Arg Ser Glu His Asp Leu Thr Pro Met Asp Gly Glu Thr Phe Ser
    1890                1895                1900

Gln His Leu Gln Ala Val Lys Ile Leu Ala Val Arg Asp Leu Ile Trp
1905                1910                1915                1920

Val Pro Arg Arg Gly Gly Asp Val Ile Val Ile Gly Leu Glu Lys Asp
                1925                1930                1935

Ser Gly Ala Gln Arg Gly Arg Val Ile Ala Val Leu Lys Ala Arg Glu
            1940                1945                1950

Leu Thr Pro His Gly Val Leu Val Asp Ala Ala Val Val Ala Lys Asp
        1955                1960                1965

Thr Val Val Cys Thr Phe Glu Asn Glu Asn Thr Glu Trp Cys Leu Ala
    1970                1975                1980

Val Trp Arg Gly Trp Gly Ala Arg Glu Phe Asp Ile Phe Tyr Gln Ser
1985                1990                1995                2000

Tyr Glu Glu Leu Gly Arg Leu Glu Ala Cys Thr Arg Lys Arg Arg
                2005                2010                2015


<210> 9
<211> 6294
<212> DNA
<213> Homo sapiens

<400> 9
cgaggggacg cctcgcgacg gttcctggga gagctggcgg cggccttgct ctgcgcgctc 60
ttcgcgccgc cctccccgcc cgcccgcctc aggattgagg aagtgcgtct gggcccggcc 120
ccggcgcggg gggcagacgg cggtgggacg gccaggcccc ggccccgcca gtgtgtccgc 180
ccggccccgc gtcccggagg agtcagctgt gtgtccagaa cgtgccatgg agacgcttaa 240

cggtgccggg gacacgggcg gcaagccgtc cacgcggggc ggtgaccctg cagcgcggtc 300
ccgcaggacg gaaggcatcc gcgccgcgta caggcgggga gaccgcggcg gcgcccggga 360
cctgctggag gaggcctgcg accagtgcgc gtcccagctg gaaaagggcc agcttctgag 420
catcccggca gcctatgggg atctggagat ggtccgctac ctactcagca agagactggt 480
ggagctgccc accgagccca cggatgacaa cccagccgtg gtggcagcgt attttggaca 540
cacggcagtt gtgcaaaata cgctgcccac cgagcccacg gatgacaacc cagccgtggt 600
ggcagcgtat tttggacaca cggcagttgt gcaggaattg cttgagtcct taccaggtcc 660
ctgcagtccc cagcggcttc tgaactggat gctggccttg gcttgccagc gagggcacct 720
gggggttgtg aagctcctgg tcctgacgca cggggctgac ccggagagct acgctgtcag 780
gaagaatgag ttccctgtca tcgtgcgctt gcccctgtat gcggccatca agtcagggaa 840
tgaagacatt gcaatattcc tgcttcggca tggggcctat ttctgttcct acatcttgct 900
ggatagtcct gaccccagca aacatctgct gagaaagtac ttcattgaag ccagtccctt 960
gcccagcagt tatccgggaa aaacagctct ccgtgtgaaa tggtcccatc tcagactgcc 1020
ctgggtagac ctagactggc tcatagacat ctcctgccag atcacggagc tcgacctttc 1080
tgccaactgc ctggcgaccc tcccctcggt tatcccctgg ggcctcatca atctccggaa 1140
gctgaacctc tccgacaacc acctggggga gctgcctggc gtgcagtcat cggacgaaat 1200
catctgttcc aggctacttg aaattgacat ttccagcaac aagttgtccc acctccctcc 1260
tggattcttg cacctctcaa aacttcaaaa actgacagct tcaaaaaatt gtttagaaaa 1320
attgttcgaa gaagaaaatg ccactaactg gataggttta cggaagctac aggaacttga 1380
tatatctgac aataaattga cagaactccc tgccctgttc cttcactctt tcaagtccct 1440
caattctctg aatgtctcca gaaacaacct gaaggtgttt ccagatccct gggcctgccc 1500
tttgaaatgt tgtaaagctt ccagaaatgc cctggaatgt ctgccagaca aaatggctgt 1560
cttttggaaa aatcacctga aggatgtgga tttctcagaa aacgcactca aagaagttcc 1620
cctgggactt ttccagcttg atgccctcat gttcttgagg ttacagggga accagctggc 1680
ggcacttcca cctcaagaga agtggacctg caggcagctc aaaaccctgg atctctccag 1740
aaaccaactt ggcaaaaatg aagatggact gaaaacgaag cgtattgcct ttttcaccac 1800
cagaggtcgc cagcgctccg ggactgaggc agcaagtgtg ctggaatttc cggccttcct 1860
aagtgagtct ttggaagtcc tttgcctgaa cgacaaccac ctcgacacag tccctccctc 1920
ggtttgccta ctgaagagct tatcagagct ctacttggga aacaaccctg gcctccggga 1980
gctccctcct gagctggggc agctgggcaa cctctggcag ctggacactg aagacctgac 2040
catcagcaat gtgcctgcag aaatccaaaa agaaggcccc aaagcaatgc tgtcttacct 2100
gcgtgctcag ctgcggaaag cggaaaagtg caagctgatg aagatgatca tcgtgggtcc 2160
cccgcgccag ggcaagtcca ccctcctgga gatcttacag acggggaggg ccccccaggt 2220
ggtgcatgga gaggccacca tcaggaccac caagtgggag ctccagaggc cggctggctc 2280
aagagccaag gttgagtccg tggagttcaa cgtctgggac atcgggggac cggccagcat 2340
ggccactgtc aaccagtgct tcttcacgga caaggccctg tacgtggtgg tctggaacct 2400
ggcgctgggg gaggaggccg tggccaacct ccagttctgg ctgctcaaca tcgaggccaa 2460
ggccccaaac gccgtggtgc tggtggtcgg gacgcacctg gatttaattg aagccaagtt 2520
ccgtgtggaa aggattgcaa cgctgcgtgc ctatgtgctg gcactctgcc gctccccctc 2580
cggctccagg gccacaggct tcccagacat caccttcaaa cacttacatg agatttcctg 2640
caagagcctg gaaggtcagg aagggctgcg acagctgatt ttccacgtca cgtgcagcat 2700
gaaggacgtc ggcagcacca tcggctgcca acgactggca gggcggctga tccccaggag 2760
ctacctgagc ctgcaggagg ccgtgctggc agagcagcag cgccgcagcc gggacgacga 2820
cgtgcagtac ctgacggaca rgcagctgga gcagctggtg gagcagacgc ccgacaacga 2880
catcaaggac tacgaggacc tgcagtcagc catcagcttc ctcatagaaa ccggcaccct 2940
gctccatttc ccggacacca gccacggcct gaggaacctc tacttcctcg accctatttg 3000
gctctccgaa tgtctgcaga ggatctttaa tattaagggc tctcgatcag tggccaagaa 3060
tggggtgatc agagcagaag acctcaggat gctgctggtg gggactggct tcacgcagca 3120
gacggaagag cagtacttcc agttcctggc caagtttgag atcgccctgc ccgtcgccaa 3180
tgacagctac ctcctgcccc atctccttcc atctaaacct ggcctggaca cccacggtat 3240
gcggcacccc acagccaaca ccattcagag ggtatttaag atgagcttcg ttcccgttgg 3300
cttctggcaa aggtttatag cacggatgct gatcagcctg gcggagatgg acctgcagct 3360
ttttgaaaac aagaagaata ctaaaagcag gaacaggaaa gtcaccattt acagttttac 3420
aggaaaccag agaaatcgct gtagcacatt cagagtgaaa agaaatcaga ccatctattg 3480
gcaggaaggg ctcctggtca cttttgatgg gggctacctc agtgtggaat cttccgacgt 3540
gaactggaaa aagaagaaaa gcggaggaat gaaaattgtt tgccaatcag aagtgaggga 3600
cttctcagcc atggctttca tcacggacca cgtcaattcc ttgattgatc agtggtttcc 3660
cgccctgacg gccacagaga gcgacggcac gccactcatg gagcagtacg tgccctgccc 3720
ggtctgcgag acagcctggg cccagcacac ggaccccagt gagaaatcag aggatgtgca 3780
gtacttcgac atggaagact gtgtcctgac ggccatcgag cgggacttca tctcctgccc 3840
cagacacccg gacctccccg tgccgctgca ggagctggtc cctgaactgt tcatgaccga 3900
cttcccggcc aggctcttcc tggagaacag caagctggag cacagcgagg acgagggcag 3960
cgtcctgggc cagggcggca gtggcaccgt catctaccgg gcccggtacc agggccagcc 4020

tgtggccgtc aagcgcttcc acatcaaaaa attcaagaac tttgctaacg taccggcaga 4080
caccatgctg aggcacctgc gggccaccga tgccatgaag aacttctccg agttccggca 4140
ggaggccagc atgctgcacg cgctgcagca cccctgcatc gtggcgctca tcggcatcag 4200
catccacccg ctctgcttcg ccctggagct cgcgccgctc agcagcctca acaccgtgct 4260
gtccgagaac gccagagatt cttcctttat acccctggga cacatgctca cccaaaaaat 4320
agcctaccag atcgcctcgg gcctggccta cctgcacaag aaaaacatca tcttctgtga 4380
cctgaagtcg gacaacattc tggtgtggtc ccttgacgtc aaggagcaca tcaacatcaa 4440
gctatctgac tacgggattt cgaggcagtc attccatgag ggcgccctag gcgtggaggg 4500
cactcctggc taccaggccc cagagatcag gcctcgcatt gtatatgatg agaaggtaga 4560
tatgttctcc tatggaatgg tgctctacga gttgctgtca ggacagcgcc ctgcactggg 4620
ccaccaccag ctccagattg ccaagaagct gtccaagggc atccgcccgg ttctgggcca 4680
gccggaggaa gtgcagttcc ggcgactgca ggcgctcatg atggagtgct gggacactaa 4740
gccagagaag cgaccgctgg ccctgtcggt ggtgagccag atgaaggacc cgacttttgc 4800
caccttcatg tatgaactgt gctgtgggaa gcagacagcc ttcttctcat cccagggcca 4860
ggagtacact gtggtgtttt gggatggaaa agaggagtcc aggaactaca cggtggtgaa 4920
cacagagaag ggcctcatgg aggtgcagag gatgtgctgc cctgggatga aggtgagctg 4980
ccagctccag gtccagagat ccctgtggac agccaccgag gaccagaaaa tctacatcta 5040
caccctcaag ggcatgtgcc ccttaaacac accccaacag gccttggata ctccagctgt 5100
cgtcacctgc ttcttggccg tgcctgttat taaaaagaat tcctacctgg tcttagcggg 5160
cctcgccgat gggcttgtgg ctgtgtttcc cgtggtgcgg ggcaccccaa aggacagctg 5220
ctcctacctg tgctcacaca cagccaacag gtccaagttc agcatcgcgg atgaagacgc 5280
acggcagaac ccctacccag tgaaggccat ggaggtggtc aacagcggct ctgaggtctg 5340
gtacagcaat gggccgggcc tccttgtcat cgactgtgcc tccctggaga tctgcaggcg 5400
gctggagccc tacatggccc cctccatggt tacgtcagtc gtgtgcagct ctgagggcag 5460
aggggaggag gtcgtctggt gcctggatga caaggccaac tccttggtga tgtaccactc 5520
caccacctac cagytgtgtg cccggtactt ctgcggggtc cccagccccc tcagggacat 5580
gtttcccgtg cggcccttgg acacggaacc cccggcagcc agccacacgg ccaacccaaa 5640
ggtgcctgag ggggactcca tcgcggacgt gagcatcatg tacagtgagg agctgggcac 5700
gcagatcctg atccaccagg aatcactcac tgactactgc tccatgtcct cctactcctc 5760
atccccaccc cgccaggctg ccaggtcccc ctcaagcctc cccagctccc cagcaagttc 5820
ttccagtgtg cctttctcca ccgactgcga ggactcagac atgctacata cgcccggtgc 5880
tgcctccgac aggtctgagc atgacctgac ccccatggac ggggagacct tcagccagca 5940
cctgcaggcc gtgaagatcc tcgccgtcag agacctcatt tgggtcccca ggcgcggtgg 6000
agatgttatc gtcattggcc tggagaagga ttctggcgcc cagcggggcc gagtcattgc 6060
cgtcttaaaa gcccgagagc tgactccgca tggggtgctg gtggatgctg ccgtggtggc 6120
aaaggacact gttgtgtgca cctttgaaaa tgaaaacaca gagtggtgcc tggccgtctg 6180
gaggggctgg ggcgccaggg agttcgacat tttctaccag tcctacgagg agctgggccg 6240
gctggaggct tgcactcgca agagaaggta attcctgaag ggcgaatcgc ggcc 6294

<210> 10
<211> 2089
<212> PRT
<213> Homo sapiens

<400> 10
Glu Gly Thr Pro Arg Asp Gly Ser Trp Glu Ser Trp Arg Arg Pro Cys
1               5                   10                  15

Ser Ala Arg Ser Ser Arg Arg Pro Pro Arg Pro Pro Ala Ser Gly Leu
            20                  25                  30

Arg Lys Cys Val Trp Ala Arg Pro Arg Arg Gly Gly Gln Thr Ala Val
        35                  40                  45

Gly Arg Pro Gly Pro Gly Pro Ala Ser Val Ser Ala Arg Pro Arg Val
    50                  55                  60

Pro Glu Glu Ser Ala Val Cys Pro Glu Arg Ala Met Glu Thr Leu Asn
65                  70                  75                  80

Gly Ala Gly Asp Thr Gly Gly Lys Pro Ser Thr Arg Gly Gly Asp Pro
                85                  90                  95

Ala Ala Arg Ser Arg Arg Thr Glu Gly Ile Arg Ala Ala Tyr Arg Arg
            100                 105                 110

Gly Asp Arg Gly Gly Ala Arg Asp Leu Leu Glu Glu Ala Cys Asp Gln
        115                 120                 125

Cys Ala Ser Gln Leu Glu Lys Gly Gln Leu Leu Ser Ile Pro Ala Ala
    130                 135                 140

Tyr Gly Asp Leu Glu Met Val Arg Tyr Leu Leu Ser Lys Arg Leu Val
145                 150                 155                 160

Glu Leu Pro Thr Glu Pro Thr Asp Asp Asn Pro Ala Val Val Ala Ala
                165                 170                 175

Tyr Phe Gly His Thr Ala Val Val Gln Asn Thr Leu Pro Thr Glu Pro
            180                 185                 190

Thr Asp Asp Asn Pro Ala Val Val Ala Ala Tyr Phe Gly His Thr Ala
        195                 200                 205

Val Val Gln Glu Leu Leu Glu Ser Leu Pro Gly Pro Cys Ser Pro Gln
    210                 215                 220

Arg Leu Leu Asn Trp Met Leu Ala Leu Ala Cys Gln Arg Gly His Leu
225                 230                 235                 240

Gly Val Val Lys Leu Leu Val Leu Thr His Gly Ala Asp Pro Glu Ser
                245                 250                 255

Tyr Ala Val Arg Lys Asn Glu Phe Pro Val Ile Val Arg Leu Pro Leu
            260                 265                 270

Tyr Ala Ala Ile Lys Ser Gly Asn Glu Asp Ile Ala Ile Phe Leu Leu
        275                 280                 285

Arg His Gly Ala Tyr Phe Cys Ser Tyr Ile Leu Leu Asp Ser Pro Asp
    290                 295                 300

Pro Ser Lys His Leu Leu Arg Lys Tyr Phe Ile Glu Ala Ser Pro Leu
305                 310                 315                 320

Pro Ser Ser Tyr Pro Gly Lys Thr Ala Leu Arg Val Lys Trp Ser His
                325                 330                 335

Leu Arg Leu Pro Trp Val Asp Leu Asp Trp Leu Ile Asp Ile Ser Cys
            340                 345                 350

Gln Ile Thr Glu Leu Asp Leu Ser Ala Asn Cys Leu Ala Thr Leu Pro
        355                 360                 365

Ser Val Ile Pro Trp Gly Leu Ile Asn Leu Arg Lys Leu Asn Leu Ser
    370                 375                 380

Asp Asn His Leu Gly Glu Leu Pro Gly Val Gln Ser Ser Asp Glu Ile
385                 390                 395                 400

Ile Cys Ser Arg Leu Leu Glu Ile Asp Ile Ser Ser Asn Lys Leu Ser
                405                 410                 415

His Leu Pro Pro Gly Phe Leu His Leu Ser Lys Leu Gln Lys Leu Thr
            420                 425                 430

Ala Ser Lys Asn Cys Leu Glu Lys Leu Phe Glu Glu Glu Asn Ala Thr
        435                 440                 445

Asn Trp Ile Gly Leu Arg Lys Leu Gln Glu Leu Asp Ile Ser Asp Asn
    450                 455                 460

Lys Leu Thr Glu Leu Pro Ala Leu Phe Leu His Ser Phe Lys Ser Leu
465                 470                 475                 480

Asn Ser Leu Asn Val Ser Arg Asn Asn Leu Lys Val Phe Pro Asp Pro
                485                 490                 495

Trp Ala Cys Pro Leu Lys Cys Cys Lys Ala Ser Arg Asn Ala Leu Glu
            500                 505                 510

Cys Leu Pro Asp Lys Met Ala Val Phe Trp Lys Asn His Leu Lys Asp
        515                 520                 525

Val Asp Phe Ser Glu Asn Ala Leu Lys Glu Val Pro Leu Gly Leu Phe
    530                 535                 540

Gln Leu Asp Ala Leu Met Phe Leu Arg Leu Gln Gly Asn Gln Leu Ala
545                 550                 555                 560

Ala Leu Pro Pro Gln Glu Lys Trp Thr Cys Arg Gln Leu Lys Thr Leu
                565                 570                 575

Asp Leu Ser Arg Asn Gln Leu Gly Lys Asn Glu Asp Gly Leu Lys Thr
            580                 585                 590

Lys Arg Ile Ala Phe Phe Thr Thr Arg Gly Arg Gln Arg Ser Gly Thr
        595                 600                 605

Glu Ala Ala Ser Val Leu Glu Phe Pro Ala Phe Leu Ser Glu Ser Leu
    610                 615                 620

Glu Val Leu Cys Leu Asn Asp Asn His Leu Asp Thr Val Pro Pro Ser
625                 630                 635                 640

Val Cys Leu Leu Lys Ser Leu Ser Glu Leu Tyr Leu Gly Asn Asn Pro
                645                 650                 655

Gly Leu Arg Glu Leu Pro Pro Glu Leu Gly Gln Leu Gly Asn Leu Trp
            660                 665                 670

Gln Leu Asp Thr Glu Asp Leu Thr Ile Ser Asn Val Pro Ala Glu Ile
        675                 680                 685

Gln Lys Glu Gly Pro Lys Ala Met Leu Ser Tyr Leu Arg Ala Gln Leu
    690                 695                 700

Arg Lys Ala Glu Lys Cys Lys Leu Met Lys Met Ile Ile Val Gly Pro
705                 710                 715                 720

Pro Arg Gln Gly Lys Ser Thr Leu Leu Glu Ile Leu Gln Thr Gly Arg
                725                 730                 735

Ala Pro Gln Val Val His Gly Glu Ala Thr Ile Arg Thr Thr Lys Trp
            740                 745                 750

Glu Leu Gln Arg Pro Ala Gly Ser Arg Ala Lys Val Glu Ser Val Glu
        755                 760                 765

Phe Asn Val Trp Asp Ile Gly Gly Pro Ala Ser Met Ala Thr Val Asn
    770                 775                 780

Gln Cys Phe Phe Thr Asp Lys Ala Leu Tyr Val Val Val Trp Asn Leu
785                 790                 795                 800

Ala Leu Gly Glu Glu Ala Val Ala Asn Leu Gln Phe Trp Leu Leu Asn
                805                 810                 815

Ile Glu Ala Lys Ala Pro Asn Ala Val Val Leu Val Val Gly Thr His
            820                 825                 830

Leu Asp Leu Ile Glu Ala Lys Phe Arg Val Glu Arg Ile Ala Thr Leu
        835                 840                 845

Arg Ala Tyr Val Leu Ala Leu Cys Arg Ser Pro Ser Gly Ser Arg Ala
    850                 855                 860

Thr Gly Phe Pro Asp Ile Thr Phe Lys His Leu His Glu Ile Ser Cys
865                 870                 875                 880

Lys Ser Leu Glu Gly Gln Glu Gly Leu Arg Gln Leu Ile Phe His Val
                885                 890                 895

Thr Cys Ser Met Lys Asp Val Gly Ser Thr Ile Gly Cys Gln Arg Leu
            900                 905                 910

Ala Gly Arg Leu Ile Pro Arg Ser Tyr Leu Ser Leu Gln Glu Ala Val
        915                 920                 925

Leu Ala Glu Gln Gln Arg Arg Ser Arg Asp Asp Asp Val Gln Tyr Leu
    930                 935                 940

Thr Asp Xaa Gln Leu Glu Gln Leu Val Glu Gln Thr Pro Asp Asn Asp
945                 950                 955                 960

Ile Lys Asp Tyr Glu Asp Leu Gln Ser Ala Ile Ser Phe Leu Ile Glu
                965                 970                 975

Thr Gly Thr Leu Leu His Phe Pro Asp Thr Ser His Gly Leu Arg Asn
            980                 985                 990

Leu Tyr Phe Leu Asp Pro Ile Trp Leu Ser Glu Cys Leu Gln Arg Ile
        995                 1000                1005

Phe Asn Ile Lys Gly Ser Arg Ser Val Ala Lys Asn Gly Val Ile Arg
    1010                1015                1020

Ala Glu Asp Leu Arg Met Leu Leu Val Gly Thr Gly Phe Thr Gln Gln
1025                1030                1035                1040

Thr Glu Glu Gln Tyr Phe Gln Phe Leu Ala Lys Phe Glu Ile Ala Leu
                1045                1050                1055

Pro Val Ala Asn Asp Ser Tyr Leu Leu Pro His Leu Leu Pro Ser Lys
            1060                1065                1070

Pro Gly Leu Asp Thr His Gly Met Arg His Pro Thr Ala Asn Thr Ile
        1075                1080                1085

Gln Arg Val Phe Lys Met Ser Phe Val Pro Val Gly Phe Trp Gln Arg
    1090                1095                1100

Phe Ile Ala Arg Met Leu Ile Ser Leu Ala Glu Met Asp Leu Gln Leu
1105                1110                1115                1120

Phe Glu Asn Lys Lys Asn Thr Lys Ser Arg Asn Arg Lys Val Thr Ile
                1125                1130                1135

Tyr Ser Phe Thr Gly Asn Gln Arg Asn Arg Cys Ser Thr Phe Arg Val
            1140                1145                1150

Lys Arg Asn Gln Thr Ile Tyr Trp Gln Glu Gly Leu Leu Val Thr Phe
        1155                1160                1165

Asp Gly Gly Tyr Leu Ser Val Glu Ser Ser Asp Val Asn Trp Lys Lys
    1170                1175                1180

Lys Lys Ser Gly Gly Met Lys Ile Val Cys Gln Ser Glu Val Arg Asp
1185                1190                1195                1200

Phe Ser Ala Met Ala Phe Ile Thr Asp His Val Asn Ser Leu Ile Asp
                1205                1210                1215

Gln Trp Phe Pro Ala Leu Thr Ala Thr Glu Ser Asp Gly Thr Pro Leu
            1220                1225                1230

Met Glu Gln Tyr Val Pro Cys Pro Val Cys Glu Thr Ala Trp Ala Gln
        1235                1240                1245

His Thr Asp Pro Ser Glu Lys Ser Glu Asp Val Gln Tyr Phe Asp Met
    1250                1255                1260

Glu Asp Cys Val Leu Thr Ala Ile Glu Arg Asp Phe Ile Ser Cys Pro
1265                1270                1275                1280

Arg His Pro Asp Leu Pro Val Pro Leu Gln Glu Leu Val Pro Glu Leu
                1285                1290                1295

Phe Met Thr Asp Phe Pro Ala Arg Leu Phe Leu Glu Asn Ser Lys Leu
            1300                1305                1310

Glu His Ser Glu Asp Glu Gly Ser Val Leu Gly Gln Gly Gly Ser Gly
        1315                1320                1325

Thr Val Ile Tyr Arg Ala Arg Tyr Gln Gly Gln Pro Val Ala Val Lys
    1330                1335                1340

Arg Phe His Ile Lys Lys Phe Lys Asn Phe Ala Asn Val Pro Ala Asp
1345                1350                1355                1360

Thr Met Leu Arg His Leu Arg Ala Thr Asp Ala Met Lys Asn Phe Ser
                1365                1370                1375

Glu Phe Arg Gln Glu Ala Ser Met Leu His Ala Leu Gln His Pro Cys
            1380                1385                1390

Ile Val Ala Leu Ile Gly Ile Ser Ile His Pro Leu Cys Phe Ala Leu
        1395                1400                1405

Glu Leu Ala Pro Leu Ser Ser Leu Asn Thr Val Leu Ser Glu Asn Ala
    1410                1415                1420

Arg Asp Ser Ser Phe Ile Pro Leu Gly His Met Leu Thr Gln Lys Ile
1425                1430                1435                1440

Ala Tyr Gln Ile Ala Ser Gly Leu Ala Tyr Leu His Lys Lys Asn Ile
                1445                1450                1455

Ile Phe Cys Asp Leu Lys Ser Asp Asn Ile Leu Val Trp Ser Leu Asp
            1460                1465                1470

Val Lys Glu His Ile Asn Ile Lys Leu Ser Asp Tyr Gly Ile Ser Arg
        1475                1480                1485

Gln Ser Phe His Glu Gly Ala Leu Gly Val Glu Gly Thr Pro Gly Tyr
    1490                1495                1500

Gln Ala Pro Glu Ile Arg Pro Arg Ile Val Tyr Asp Glu Lys Val Asp
1505                1510                1515                1520

Met Phe Ser Tyr Gly Met Val Leu Tyr Glu Leu Leu Ser Gly Gln Arg
                1525                1530                1535

Pro Ala Leu Gly His His Gln Leu Gln Ile Ala Lys Lys Leu Ser Lys
            1540                1545                1550

Gly Ile Arg Pro Val Leu Gly Gln Pro Glu Glu Val Gln Phe Arg Arg
        1555                1560                1565

Leu Gln Ala Leu Met Met Glu Cys Trp Asp Thr Lys Pro Glu Lys Arg
    1570                1575                1580

Pro Leu Ala Leu Ser Val Val Ser Gln Met Lys Asp Pro Thr Phe Ala
1585                1590                1595                1600

Thr Phe Met Tyr Glu Leu Cys Cys Gly Lys Gln Thr Ala Phe Phe Ser
                1605                1610                1615

Ser Gln Gly Gln Glu Tyr Thr Val Val Phe Trp Asp Gly Lys Glu Glu
            1620                1625                1630

Ser Arg Asn Tyr Thr Val Val Asn Thr Glu Lys Gly Leu Met Glu Val
        1635                1640                1645

Gln Arg Met Cys Cys Pro Gly Met Lys Val Ser Cys Gln Leu Gln Val
    1650                1655                1660

Gln Arg Ser Leu Trp Thr Ala Thr Glu Asp Gln Lys Ile Tyr Ile Tyr
1665                1670                1675                1680

Thr Leu Lys Gly Met Cys Pro Leu Asn Thr Pro Gln Gln Ala Leu Asp
                1685                1690                1695

Thr Pro Ala Val Val Thr Cys Phe Leu Ala Val Pro Val Ile Lys Lys
            1700                1705                1710

Asn Ser Tyr Leu Val Leu Ala Gly Leu Ala Asp Gly Leu Val Ala Val
        1715                1720                1725

Phe Pro Val Val Arg Gly Thr Pro Lys Asp Ser Cys Ser Tyr Leu Cys
    1730                1735                1740

Ser His Thr Ala Asn Arg Ser Lys Phe Ser Ile Ala Asp Glu Asp Ala
1745                1750                1755                1760

Arg Gln Asn Pro Tyr Pro Val Lys Ala Met Glu Val Val Asn Ser Gly
                1765                1770                1775

Ser Glu Val Trp Tyr Ser Asn Gly Pro Gly Leu Leu Val Ile Asp Cys
        1780                1785                1790

Ala Ser Leu Glu Ile Cys Arg Arg Leu Glu Pro Tyr Met Ala Pro Ser
    1795                1800                1805

Met Val Thr Ser Val Val Cys Ser Ser Glu Gly Arg Gly Glu Glu Val
  1810                1815                1820

Val Trp Cys Leu Asp Asp Lys Ala Asn Ser Leu Val Met Tyr His Ser
1825                1830                1835                1840

Thr Thr Tyr Gln Leu Cys Ala Arg Tyr Phe Cys Gly Val Pro Ser Pro
                1845                1850                1855

Leu Arg Asp Met Phe Pro Val Arg Pro Leu Asp Thr Glu Pro Pro Ala
        1860                1865                1870

Ala Ser His Thr Ala Asn Pro Lys Val Pro Glu Gly Asp Ser Ile Ala
    1875                1880                1885

Asp Val Ser Ile Met Tyr Ser Glu Glu Leu Gly Thr Gln Ile Leu Ile
  1890                1895                1900

His Gln Glu Ser Leu Thr Asp Tyr Cys Ser Met Ser Ser Tyr Ser Ser
1905                1910                1915                1920

Ser Pro Pro Arg Gln Ala Ala Arg Ser Pro Ser Ser Leu Pro Ser Ser
                1925                1930                1935

Pro Ala Ser Ser Ser Ser Val Pro Phe Ser Thr Asp Cys Glu Asp Ser
        1940                1945                1950

Asp Met Leu His Thr Pro Gly Ala Ala Ser Asp Arg Ser Glu His Asp
    1955                1960                1965

Leu Thr Pro Met Asp Gly Glu Thr Phe Ser Gln His Leu Gln Ala Val
  1970                1975                1980

Lys Ile Leu Ala Val Arg Asp Leu Ile Trp Val Pro Arg Arg Gly Gly
1985                1990                1995                2000

Asp Val Ile Val Ile Gly Leu Glu Lys Asp Ser Gly Ala Gln Arg Gly
                2005                2010                2015

Arg Val Ile Ala Val Leu Lys Ala Arg Glu Leu Thr Pro His Gly Val
        2020                2025                2030

Leu Val Asp Ala Ala Val Val Ala Lys Asp Thr Val Val Cys Thr Phe
    2035                2040                2045

Glu Asn Glu Asn Thr Glu Trp Cys Leu Ala Val Trp Arg Gly Trp Gly
  2050                2055                2060

Ala Arg Glu Phe Asp Ile Phe Tyr Gln Ser Tyr Glu Glu Leu Gly Arg
2065                2070                2075                2080

Leu Glu Ala Cys Thr Arg Lys Arg Arg
                2085

<210> 11

<211> 6216
<212> DNA
<213> Homo sapiens

<400> 11
cgaggggacg cctcgcgacg gttcctggga gagctggcgg cggccttgct ctgcgcgctc 60
ttcgcgccgc cctccccgcc cgcccgcctc aggattgagg aagtgcgtct gggcccggcc 120
ccggcgcggg gggcagacgg cggtgggacg gccaggcccc ggccccgcca gtgtgtccgc 180
ccggccccgc gtcccggagg agtcagctgt gtgtccagaa cgtgccatgg agacgcttaa 240
cggtgccggg gacacgggcg gcaagccgtc cacgcggggc ggtgaccctg cagcgcggtc 300
ccgcaggacg gaaggcatcc gcgccgcgta caggcgggga gaccgcggcg gcgcccggga 360
cctgctggag gaggcctgcg accagtgcgc gtcccagctg gaaaagggcc agcttctgag 420
catcccggca gcctatgggg atctggagat ggtccgctac ctactcagca agagactggt 480
ggagctgccc accgagccca cggatgacaa cccagccgtg gtggcagcgt attttggaca 540
cacggcagtt gtgcaggaat tgcttgagtc cttaccaggt ccctgcagtc cccagcggct 600
tctgaactgg atgctggcct tggcttgcca gcgagggcac ctgggggttg tgaagctcct 660
ggtcctgacg cacggggctg acccggagag ctacgctgtc aggaagaatg agttccctgt 720
catcgtgcgc ttgcccctgt atgcggccat caagtcaggg aatgaagaca ttgcaatatt 780
cctgcttcgg catggggcct atttctgttc ctacatcttg ctggatagtc ctgaccccag 840
caaacatctg ctgagaaagt acttcattga agccagtccc ttgcccagca gttatccggg 900
aaaaacagct ctccgtgtga aatggtccca tctcagactg ccctgggtag acctagactg 960
gctcatagac atctcctgcc agatcacgga gctcgacctt tctgccaact gcctggcgac 1020
cctcccctcg gttatcccct ggggcctcat caatctccgg aagctgaacc tctccgacaa 1080
ccacctgggg gagctgcctg gcgtgcagtc atcggacgaa atcatctgtt ccaggctact 1140
tgaaattgac atttccagca acaagttgtc ccacctccct cctggattct tgcacctctc 1200
aaaacttcaa aaactgacag cttcaaaaaa ttgtttagaa aaattgttcg aagaagaaaa 1260
tgccactaac tggataggtt tacggaagct acaggaactt gatatatctg acaataaatt 1320
gacagaactc cctgccctgt tccttcactc tttcaagtcc ctcaattctc tgaatgtctc 1380
cagaaacaac ctgaaggtgt ttccagatcc ctgggcctgc cctttgaaat gttgtaaagc 1440
ttccagaaat gccctggaat gtctgccaga caaaatggct gtcttttgga aaaatcacct 1500
gaaggatgtg gatttctcag aaaacgcact caaagaagtt cccctgggac ttttccagct 1560
tgatgccctc atgttcttga ggttacaggg gaaccagctg gcggcacttc cacctcaaga 1620
gaagtggacc tgcaggcagc tcaaaaccct ggatctctcc agaaaccaac ttggcaaaaa 1680
tgaagatgga ctgaaaacga agcgtattgc ctttttcacc accagaggtc gccagcgctc 1740
cgggactgag gcagcaagtg tgctggaatt tccggccttc ctaagtgagt ctttggaagt 1800
cctttgcctg aacgacaacc acctcgacac agtccctccc tcggtttgcc tactgaagag 1860
cttatcagag ctctacttgg gaaacaaccc tggcctccgg gagctccctc ctgagctggg 1920
gcagctgggc aacctctggc agctggacac tgaagacctg accatcagca atgtgcctgc 1980
agaaatccaa aaagaaggcc ccaaagcaat gctgtcttac ctgcgtgctc agctgcggaa 2040
agcggaaaag tgcaagctga tgaagatgat catcgtgggt cccccgcgcc agggcaagtc 2100
caccctcctg gagatcttac agacggggag ggccccccag gtggtgcatg gagaggccac 2160
catcaggacc accaagtggg agctccagag gccggctggc tcaagagcca aggttgagtc 2220
cgtggagttc aacgtctggg acatcggggg accggccagc atggccactg tcaaccagtg 2280
cttcttcacg gacaaggccc tgtacgtggt ggtctggaac ctggcgctgg gggaggaggc 2340
cgtggccaac ctccagttct ggctgctcaa catcgaggcc aaggccccaa acgccgtggt 2400
gctggtggtc gggacgcacc tggatttaat tgaagccaag ttccgtgtgg aaaggattgc 2460
aacgctgcgt gcctatgtgc tggcactctg ccgctccccc tccggctcca gggccacagg 2520
cttcccagac atcaccttca aacacttaca tgagatttcc tgcaagagcc tggaaggtca 2580
ggaagggctg cgacagctga ttttccacgt cacgtgcagc atgaaggacg tcggcagcac 2640
catcggctgc caacgactgg cagggcggct gatccccagg agctacctga gcctgcagga 2700
ggccgtgctg gcagagcagc agcgccgcag ccgggacgac gacgtgcagt acctgacgga 2760
cargcagctg gagcagctgg tggagcagac gcccgacaac gacatcaagg actacgagga 2820
cctgcagtca gccatcagct tcctcataga aaccggcacc ctgctccatt tcccggacac 2880
cagccacggc ctgaggaacc tctacttcct cgaccctatt tggctctccg aatgtctgca 2940
gaggatcttt aatattaagg gctctcgatc agtggccaag aatggggtga tcagagcaga 3000
agacctcagg atgctgctgg tggggactgg cttcacgcag cagacggaag agcagtactt 3060
ccagttcctg gccaagtttg agatcgccct gcccgtcgcc aatgacagct acctcctgcc 3120
ccatctcctt ccatctaaac ctggcctgga cacccacggt atgcggcacc ccacagccaa 3180
caccattcag agggtattta agatgagctt cgttcccgtt ggcttctggc aaaggtttat 3240
agcacggatg ctgatcagcc tggcggagat ggacctgcag ctttttgaaa acaagaagaa 3300
tactaaaagc aggaacagga aagtcaccat ttacagtttt acaggaaacc agagaaatcg 3360
ctgtagcaca ttcagagtga aaagaaatca gaccatctat tggcaggaag ggctcctggt 3420
cacttttgat gggggctacc tcagtgtgga atcttccgac gtgaactgga aaaagaagaa 3480

aagcggagga atgaaaattg tttgccaatc agaagtgagg gacttctcag ccatggcttt 3540
catcacggac cacgtcaatt ccttgattga tcagtggttt cccgccctga cggccacaga 3600
gagcgacggg acgccactca tggagcagta cgtgccctgc ccggtctgcg agacagcctg 3660
ggcccagcac acggacccca gtgagaaatc agaggatgtg cagtacttcg acatggaaga 3720
ctgtgtcctg acggccatcg agcgggactt catctcctgc cccagacacc cggacctccc 3780
cgtgccgctg caggagctgg tccctgaact gttcatgacc gacttcccgg ccaggctctt 3840
cctggagaac agcaagctgg agcacagcga ggacgagggc agcgtcctgg gccagggcgg 3900
cagtggcacc gtcatctacc gggcccggta ccagggccag cctgtggccg tcaagcgctt 3960
ccacatcaaa aaattcaaga actttgctaa cgtaccggca gacaccatgc tgaggcacct 4020
gcgggccacc gatgccatga agaacttctc cgagttccgg caggaggcca gcatgctgca 4080
cgcgctgcag cacccctgca tcgtggcgct catcggcatc agcatccacc cgctctgctt 4140
cgccctggag ctcgcgccgc tcagcagcct caacaccgtg ctgtccgaga acgccagaga 4200
ttcttccttt atacccctgg gacacatgct cacccaaaaa atagcctacc agatcgcctc 4260
gggcctggcc tacctgcaca agaaaaacat catcttctgt gacctgaagt cggacaacat 4320
tctggtgtgg tcccttgacg tcaaggagca catcaacatc aagctatctg actacgggat 4380
ttcgaggcag tcattccatg agggcgccct aggcgtggag ggcactcctg gctaccaggc 4440
cccagagatc aggcctcgca ttgtatatga tgagaaggta gatatgttct cctatggaat 4500
ggtgctctac gagttgctgt caggacagcg ccctgcactg ggccaccacc agctccagat 4560
tgccaagaag ctgtccaagg gcatccgccc ggttctgggg cagccggagg aagtgcagtt 4620
ccggcgactg caggcgctca tgatggagtg ctgggacact aagccagaga agcgaccgct 4680
ggccctgtcg gtggtgagcc agatgaagga cccgactttt gccaccttca tgtatgaact 4740
gtgctgtggg aagcagacag ccttcttctc atcccagggc caggagtaca ctgtggtgtt 4800
ttgggatgga aaagaggagt ccaggaacta cacggtggtg aacacagaga agggcctcat 4860
ggaggtgcag aggatgtgct gccctgggat gaaggtgagc tgccagctcc aggtccagag 4920
atccctgtgg acagccaccg aggaccagaa aatctacatc tacaccctca agggcatgtg 4980
ccccttaaac acaccccaac aggccttgga tactccagct gtcgtcacct gcttcttggc 5040
cgtgcctgtt attaaaaaga attcctacct ggtcttagcg ggcctcgccg atgggcttgt 5100
ggctgtgttt cccgtggtgc ggggcacccc aaaggacagc tgctcctacc tgtgctcaca 5160
cacagccaac aggtccaagt tcagcatcgc ggatgaagac gcacggcaga acccctaccc 5220
agtgaaggcc atggaggtgg tcaacagcgg ctctgaggtc tggtacagca atgggccggg 5280
cctccttgtc atcgactgtg cctccctgga gatctgcagg cggctggagc cctacatggc 5340
cccctccatg gttacgtcag tcgtgtgcag ctctgagggc agaggggagg aggtcgtctg 5400
gtgcctggat gacaaggcca actccttggt gatgtaccac tccaccacct accagytgtg 5460
tgcccggtac ttctgcgggg tccccagccc cctcagggac atgtttcccg tgcggccctt 5520
ggacacggaa cccccggcag ccagccacac ggccaaccca aaggtgcctg agggggactc 5580
catcgcggac gtgagcatca tgtacagtga ggagctgggc acgcagatcc tgatccacca 5640
ggaatcactc actgactact gctccatgtc ctcctactcc tcatccccac cccgccaggc 5700
tgccaggtcc ccctcaagcc tccccagctc cccagcaagt tcttccagtg tgcctttctc 5760
caccgactgc gaggactcag acatgctaca tacgcccggt gctgcctccg acaggtctga 5820
gcatgacctg acccccatgg acggggagac cttcagccag cacctgcagg ccgtgaagat 5880
cctcgccgtc agagacctca tttgggtccc caggcgcggt ggagatgtta tcgtcattgg 5940
cctggagaag gattctggcg cccagcgggg ccgagtcatt gccgtcttaa aagcccgaga 6000
gctgactccg catggggtgc tggtggatgc tgccgtggtg gcaaaggaca ctgttgtgtg 6060
cacctttgaa aatgaaaaca cagagtggtg cctggccgtc tggaggggct ggggcgccag 6120
ggagttcgac attttctacc agtcctacga ggagctgggc cggctggagg cttgcactcg 6180
caagagaagg taattcctga agggcgaatc gcggcc 6216

<210> 12
<211> 2063
<212> PRT
<213> Homo sapiens

<400> 12
Glu Gly Thr Pro Arg Asp Gly Ser Trp Glu Ser Trp Arg Arg Pro Cys
1               5                   10                  15

Ser Ala Arg Ser Ser Arg Arg Pro Pro Arg Pro Pro Ala Ser Gly Leu
            20                  25                  30

Arg Lys Cys Val Trp Ala Arg Pro Arg Arg Gly Gly Gln Thr Ala Val
        35                  40                  45

Gly Arg Pro Gly Pro Gly Pro Ala Ser Val Ser Ala Arg Pro Arg Val
    50                  55                  60

Pro Glu Glu Ser Ala Val Cys Pro Glu Arg Ala Met Glu Thr Leu Asn
65                  70                  75                  80

Gly Ala Gly Asp Thr Gly Gly Lys Pro Ser Thr Arg Gly Gly Asp Pro
                85                  90                  95

Ala Ala Arg Ser Arg Arg Thr Glu Gly Ile Arg Ala Ala Tyr Arg Arg
            100                 105                 110

Gly Asp Arg Gly Gly Ala Arg Asp Leu Leu Glu Glu Ala Cys Asp Gln
        115                 120                 125

Cys Ala Ser Gln Leu Glu Lys Gly Gln Leu Leu Ser Ile Pro Ala Ala
    130                 135                 140

Tyr Gly Asp Leu Glu Met Val Arg Tyr Leu Leu Ser Lys Arg Leu Val
145                 150                 155                 160

Glu Leu Pro Thr Glu Pro Thr Asp Asp Asn Pro Ala Val Val Ala Ala
                165                 170                 175

Tyr Phe Gly His Thr Ala Val Val Gln Glu Leu Leu Glu Ser Leu Pro
            180                 185                 190

Gly Pro Cys Ser Pro Gln Arg Leu Leu Asn Trp Met Leu Ala Leu Ala
        195                 200                 205

Cys Gln Arg Gly His Leu Gly Val Val Lys Leu Leu Val Leu Thr His
    210                 215                 220

Gly Ala Asp Pro Glu Ser Tyr Ala Val Arg Lys Asn Glu Phe Pro Val
225                 230                 235                 240

Ile Val Arg Leu Pro Leu Tyr Ala Ala Ile Lys Ser Gly Asn Glu Asp
                245                 250                 255

Ile Ala Ile Phe Leu Leu Arg His Gly Ala Tyr Phe Cys Ser Tyr Ile
            260                 265                 270

Leu Leu Asp Ser Pro Asp Pro Ser Lys His Leu Leu Arg Lys Tyr Phe
        275                 280                 285

Ile Glu Ala Ser Pro Leu Pro Ser Ser Tyr Pro Gly Lys Thr Ala Leu
    290                 295                 300

Arg Val Lys Trp Ser His Leu Arg Leu Pro Trp Val Asp Leu Asp Trp
305                 310                 315                 320

Leu Ile Asp Ile Ser Cys Gln Ile Thr Glu Leu Asp Leu Ser Ala Asn
                325                 330                 335

Cys Leu Ala Thr Leu Pro Ser Val Ile Pro Trp Gly Leu Ile Asn Leu
            340                 345                 350

Arg Lys Leu Asn Leu Ser Asp Asn His Leu Gly Glu Leu Pro Gly Val
        355                 360                 365

Gln Ser Ser Asp Glu Ile Ile Cys Ser Arg Leu Leu Glu Ile Asp Ile
    370                 375                 380

Ser Ser Asn Lys Leu Ser His Leu Pro Pro Gly Phe Leu His Leu Ser
385                 390                 395                 400

Lys Leu Gln Lys Leu Thr Ala Ser Lys Asn Cys Leu Glu Lys Leu Phe
                405                 410                 415

Glu Glu Glu Asn Ala Thr Asn Trp Ile Gly Leu Arg Lys Leu Gln Glu
            420                 425                 430

Leu Asp Ile Ser Asp Asn Lys Leu Thr Glu Leu Pro Ala Leu Phe Leu
        435                 440                 445

His Ser Phe Lys Ser Leu Asn Ser Leu Asn Val Ser Arg Asn Asn Leu
    450                 455                 460

Lys Val Phe Pro Asp Pro Trp Ala Cys Pro Leu Lys Cys Cys Lys Ala
465                 470                 475                 480

Ser Arg Asn Ala Leu Glu Cys Leu Pro Asp Lys Met Ala Val Phe Trp
                485                 490                 495

Lys Asn His Leu Lys Asp Val Asp Phe Ser Glu Asn Ala Leu Lys Glu
            500                 505                 510

Val Pro Leu Gly Leu Phe Gln Leu Asp Ala Leu Met Phe Leu Arg Leu
        515                 520                 525

Gln Gly Asn Gln Leu Ala Ala Leu Pro Pro Gln Glu Lys Trp Thr Cys
    530                 535                 540

Arg Gln Leu Lys Thr Leu Asp Leu Ser Arg Asn Gln Leu Gly Lys Asn
545                 550                 555                 560

Glu Asp Gly Leu Lys Thr Lys Arg Ile Ala Phe Phe Thr Thr Arg Gly
                565                 570                 575

Arg Gln Arg Ser Gly Thr Glu Ala Ala Ser Val Leu Glu Phe Pro Ala
            580                 585                 590

Phe Leu Ser Glu Ser Leu Glu Val Leu Cys Leu Asn Asp Asn His Leu
        595                 600                 605

Asp Thr Val Pro Pro Ser Val Cys Leu Leu Lys Ser Leu Ser Glu Leu
    610                 615                 620

Tyr Leu Gly Asn Asn Pro Gly Leu Arg Glu Leu Pro Pro Glu Leu Gly
625                 630                 635                 640

Gln Leu Gly Asn Leu Trp Gln Leu Asp Thr Glu Asp Leu Thr Ile Ser
                645                 650                 655

Asn Val Pro Ala Glu Ile Gln Lys Glu Gly Pro Lys Ala Met Leu Ser
            660                 665                 670

Tyr Leu Arg Ala Gln Leu Arg Lys Ala Glu Lys Cys Lys Leu Met Lys
        675                 680                 685

Met Ile Ile Val Gly Pro Pro Arg Gln Gly Lys Ser Thr Leu Leu Glu
    690                 695                 700

Ile Leu Gln Thr Gly Arg Ala Pro Gln Val Val His Gly Glu Ala Thr
705                 710                 715                 720

Ile Arg Thr Thr Lys Trp Glu Leu Gln Arg Pro Ala Gly Ser Arg Ala
                725                 730                 735

Lys Val Glu Ser Val Glu Phe Asn Val Trp Asp Ile Gly Gly Pro Ala
            740                 745                 750

Ser Met Ala Thr Val Asn Gln Cys Phe Phe Thr Asp Lys Ala Leu Tyr
        755                 760                 765

Val Val Val Trp Asn Leu Ala Leu Gly Glu Glu Ala Val Ala Asn Leu
    770                 775                 780

Gln Phe Trp Leu Leu Asn Ile Glu Ala Lys Ala Pro Asn Ala Val Val
785                 790                 795                 800

Leu Val Val Gly Thr His Leu Asp Leu Ile Glu Ala Lys Phe Arg Val
                805                 810                 815

Glu Arg Ile Ala Thr Leu Arg Ala Tyr Val Leu Ala Leu Cys Arg Ser
            820                 825                 830

Pro Ser Gly Ser Arg Ala Thr Gly Phe Pro Asp Ile Thr Phe Lys His
        835                 840                 845

Leu His Glu Ile Ser Cys Lys Ser Leu Glu Gly Gln Glu Gly Leu Arg
    850                 855                 860

Gln Leu Ile Phe His Val Thr Cys Ser Met Lys Asp Val Gly Ser Thr
865                 870                 875                 880

Ile Gly Cys Gln Arg Leu Ala Gly Arg Leu Ile Pro Arg Ser Tyr Leu
                885                 890                 895

Ser Leu Gln Glu Ala Val Leu Ala Glu Gln Gln Arg Arg Ser Arg Asp
            900                 905                 910

Asp Asp Val Gln Tyr Leu Thr Asp Xaa Gln Leu Glu Gln Leu Val Glu
        915                 920                 925

Gln Thr Pro Asp Asn Asp Ile Lys Asp Tyr Glu Asp Leu Gln Ser Ala
    930                 935                 940

Ile Ser Phe Leu Ile Glu Thr Gly Thr Leu Leu His Phe Pro Asp Thr
945                 950                 955                 960

Ser His Gly Leu Arg Asn Leu Tyr Phe Leu Asp Pro Ile Trp Leu Ser
                965                 970                 975

Glu Cys Leu Gln Arg Ile Phe Asn Ile Lys Gly Ser Arg Ser Val Ala
            980                 985                 990

Lys Asn Gly Val Ile Arg Ala Glu Asp Leu Arg Met Leu Leu Val Gly
        995                 1000                1005

Thr Gly Phe Thr Gln Gln Thr Glu Glu Gln Tyr Phe Gln Phe Leu Ala
    1010                1015                1020

Lys Phe Glu Ile Ala Leu Pro Val Ala Asn Asp Ser Tyr Leu Leu Pro
1025                1030                1035                1040

His Leu Leu Pro Ser Lys Pro Gly Leu Asp Thr His Gly Met Arg His
                1045                1050                1055

Pro Thr Ala Asn Thr Ile Gln Arg Val Phe Lys Met Ser Phe Val Pro
            1060                1065                1070

Val Gly Phe Trp Gln Arg Phe Ile Ala Arg Met Leu Ile Ser Leu Ala
        1075                1080                1085

Glu Met Asp Leu Gln Leu Phe Glu Asn Lys Lys Asn Thr Lys Ser Arg
    1090                1095                1100

Asn Arg Lys Val Thr Ile Tyr Ser Phe Thr Gly Asn Gln Arg Asn Arg
1105                1110                1115                1120

Cys Ser Thr Phe Arg Val Lys Arg Asn Gln Thr Ile Tyr Trp Gln Glu
                1125                1130                1135

Gly Leu Leu Val Thr Phe Asp Gly Gly Tyr Leu Ser Val Glu Ser Ser
            1140                1145                1150

Asp Val Asn Trp Lys Lys Lys Lys Ser Gly Gly Met Lys Ile Val Cys
        1155                1160                1165

Gln Ser Glu Val Arg Asp Phe Ser Ala Met Ala Phe Ile Thr Asp His
    1170                1175                1180

Val Asn Ser Leu Ile Asp Gln Trp Phe Pro Ala Leu Thr Ala Thr Glu
1185                1190                1195                1200

Ser Asp Gly Thr Pro Leu Met Glu Gln Tyr Val Pro Cys Pro Val Cys
                1205                1210                1215

Glu Thr Ala Trp Ala Gln His Thr Asp Pro Ser Glu Lys Ser Glu Asp
            1220                1225                1230

Val Gln Tyr Phe Asp Met Glu Asp Cys Val Leu Thr Ala Ile Glu Arg
        1235                1240                1245

Asp Phe Ile Ser Cys Pro Arg His Pro Asp Leu Pro Val Pro Leu Gln
    1250                1255                1260

Glu Leu Val Pro Glu Leu Phe Met Thr Asp Phe Pro Ala Arg Leu Phe
1265                1270                1275                1280

Leu Glu Asn Ser Lys Leu Glu His Ser Glu Asp Glu Gly Ser Val Leu
                1285                1290                1295

Gly Gln Gly Gly Ser Gly Thr Val Ile Tyr Arg Ala Arg Tyr Gln Gly
            1300                1305                1310

Gln Pro Val Ala Val Lys Arg Phe His Ile Lys Lys Phe Lys Asn Phe
        1315                1320                1325

Ala Asn Val Pro Ala Asp Thr Met Leu Arg His Leu Arg Ala Thr Asp
    1330                1335                1340

Ala Met Lys Asn Phe Ser Glu Phe Arg Gln Glu Ala Ser Met Leu His
1345                1350                1355                1360

Ala Leu Gln His Pro Cys Ile Val Ala Leu Ile Gly Ile Ser Ile His
                1365                1370                1375

Pro Leu Cys Phe Ala Leu Glu Leu Ala Pro Leu Ser Ser Leu Asn Thr
            1380                1385                1390

Val Leu Ser Glu Asn Ala Arg Asp Ser Ser Phe Ile Pro Leu Gly His
        1395                1400                1405

Met Leu Thr Gln Lys Ile Ala Tyr Gln Ile Ala Ser Gly Leu Ala Tyr
    1410                1415                1420

Leu His Lys Lys Asn Ile Ile Phe Cys Asp Leu Lys Ser Asp Asn Ile
1425                1430                1435                1440

Leu Val Trp Ser Leu Asp Val Lys Glu His Ile Asn Ile Lys Leu Ser
                1445                1450                1455

Asp Tyr Gly Ile Ser Arg Gln Ser Phe His Glu Gly Ala Leu Gly Val
            1460                1465                1470

Glu Gly Thr Pro Gly Tyr Gln Ala Pro Glu Ile Arg Pro Arg Ile Val
        1475                1480                1485

Tyr Asp Glu Lys Val Asp Met Phe Ser Tyr Gly Met Val Leu Tyr Glu
    1490                1495                1500

Leu Leu Ser Gly Gln Arg Pro Ala Leu Gly His His Gln Leu Gln Ile
1505                1510                1515                1520

Ala Lys Lys Leu Ser Lys Gly Ile Arg Pro Val Leu Gly Gln Pro Glu
                1525                1530                1535

Glu Val Gln Phe Arg Arg Leu Gln Ala Leu Met Met Glu Cys Trp Asp
            1540                1545                1550

Thr Lys Pro Glu Lys Arg Pro Leu Ala Leu Ser Val Val Ser Gln Met
        1555                1560                1565

Lys Asp Pro Thr Phe Ala Thr Phe Met Tyr Glu Leu Cys Cys Gly Lys
    1570                1575                1580

Gln Thr Ala Phe Phe Ser Ser Gln Gly Gln Glu Tyr Thr Val Val Phe
1585                1590                1595                1600

Trp Asp Gly Lys Glu Glu Ser Arg Asn Tyr Thr Val Val Asn Thr Glu
                1605                1610                1615

Lys Gly Leu Met Glu Val Gln Arg Met Cys Cys Pro Gly Met Lys Val
            1620                1625                1630

Ser Cys Gln Leu Gln Val Gln Arg Ser Leu Trp Thr Ala Thr Glu Asp
        1635                1640                1645

Gln Lys Ile Tyr Ile Tyr Thr Leu Lys Gly Met Cys Pro Leu Asn Thr
    1650                1655                1660

Pro Gln Gln Ala Leu Asp Thr Pro Ala Val Val Thr Cys Phe Leu Ala
1665                1670                1675                1680

Val Pro Val Ile Lys Lys Asn Ser Tyr Leu Val Leu Ala Gly Leu Ala
                1685                1690                1695

Asp Gly Leu Val Ala Val Phe Pro Val Val Arg Gly Thr Pro Lys Asp
            1700                1705                1710

Ser Cys Ser Tyr Leu Cys Ser His Thr Ala Asn Arg Ser Lys Phe Ser
        1715                1720                1725

Ile Ala Asp Glu Asp Ala Arg Gln Asn Pro Tyr Pro Val Lys Ala Met
    1730                1735                1740

Glu Val Val Asn Ser Gly Ser Glu Val Trp Tyr Ser Asn Gly Pro Gly
1745                1750                1755                1760

Leu Leu Val Ile Asp Cys Ala Ser Leu Glu Ile Cys Arg Arg Leu Glu
                1765                1770                1775

Pro Tyr Met Ala Pro Ser Met Val Thr Ser Val Val Cys Ser Ser Glu
            1780                1785                1790

Gly Arg Gly Glu Glu Val Val Trp Cys Leu Asp Asp Lys Ala Asn Ser
        1795                1800                1805

Leu Val Met Tyr His Ser Thr Thr Tyr Gln Leu Cys Ala Arg Tyr Phe
    1810                1815                1820

Cys Gly Val Pro Ser Pro Leu Arg Asp Met Phe Pro Val Arg Pro Leu
1825                1830                1835                1840

Asp Thr Glu Pro Pro Ala Ala Ser His Thr Ala Asn Pro Lys Val Pro
                1845                1850                1855

Glu Gly Asp Ser Ile Ala Asp Val Ser Ile Met Tyr Ser Glu Glu Leu
            1860                1865                1870

Gly Thr Gln Ile Leu Ile His Gln Glu Ser Leu Thr Asp Tyr Cys Ser
        1875                1880                1885

Met Ser Ser Tyr Ser Ser Ser Pro Pro Arg Gln Ala Ala Arg Ser Pro
    1890                1895                1900

Ser Ser Leu Pro Ser Ser Pro Ala Ser Ser Ser Ser Val Pro Phe Ser
1905                1910                1915                1920

Thr Asp Cys Glu Asp Ser Asp Met Leu His Thr Pro Gly Ala Ala Ser
                1925                1930                1935

Asp Arg Ser Glu His Asp Leu Thr Pro Met Asp Gly Glu Thr Phe Ser
            1940                1945                1950

Gln His Leu Gln Ala Val Lys Ile Leu Ala Val Arg Asp Leu Ile Trp
        1955                1960                1965

Val Pro Arg Arg Gly Gly Asp Val Ile Val Ile Gly Leu Glu Lys Asp
    1970                1975                1980

Ser Gly Ala Gln Arg Gly Arg Val Ile Ala Val Leu Lys Ala Arg Glu
1985                1990                1995                2000

Leu Thr Pro His Gly Val Leu Val Asp Ala Ala Val Val Ala Lys Asp
                2005                2010                2015

Thr Val Val Cys Thr Phe Glu Asn Glu Asn Thr Glu Trp Cys Leu Ala
            2020                2025                2030

Val Trp Arg Gly Trp Gly Ala Arg Glu Phe Asp Ile Phe Tyr Gln Ser
        2035                2040                2045

Tyr Glu Glu Leu Gly Arg Leu Glu Ala Cys Thr Arg Lys Arg Arg
    2050                2055                2060

<210> 13
<211> 6508
<212> DNA
<213> Homo sapiens

<400> 13
gcggccgccg aggggacgcc tcgtgacggt tcctgggaga gctggcggcg gccttgctct 60
gcgcgctctt cgcgccgccc tccccgcccg cccgcctcag gattgaggaa gtgcgtctgg 120
gcccggcccc ggcgcggggg gcagacggcg gtgggacggc caggccccgg ccccgccagt 180
gtgtccgccc ggccccgcgt cccggagcgc ccgcacccgg ccccgccgcc gcctcagagc 240
aagaaagctt tctgctcagc catggctacg agtccacgcc ttaatgcacc ccacagccag 300
cggcagtggc agtgacaaca gcgggacctg cctttgaaga tcggctgctg caagggttga 360
tggctggcat gtcgcaaaga ccccccagca tgtactggtg tgtggggccg gaggagtcag 420
ctgtgtgtcc agaacgtgcc atggagacgc ttaacggtgc cggggacacg ggcggcaagc 480
cgtccacgcg gggcggtgac cctgcagcgc ggtcccgcag gacggaaggc atccgcgccg 540
cgtacaggcg gggagaccgc ggcggcgccc gggacctgct ggaggaggcc tgcgaccagt 600
gcgcgtccca gctggaaaag ggccagcttc tgagcatccc ggcagcctat ggggatctgg 660
agatggtccg ctacctactc agcaagagac tggtggagct gcccaccgag cccacggatg 720
acaacccagc cgtggtggca gcgtattttg gacacacggc agttgtgcaa aatacgctgc 780
ccaccgagcc cacggatgac aacccagccg tggtggcagc gtattttgga cacacggcag 840
ttgtgcagga attgcttgag tccttaccag gtccctgcag tccccagcgg cttctgaact 900
ggatgctggc cttggcttgc cagcgagggc acctgggggt tgtgaagctc ctggtcctga 960
cgcacggggc tgacccggag agctacgctg tcaggaagaa tgagttccct gtcatcgtgc 1020
gcttgcccct gtatgcggcc atcaagtcag ggaatgaaga cattgcaata ttcctgcttc 1080
ggcatggggc ctatttctgt tcctacatct tgctggatag tcctgacccc agcaaacatc 1140
tgctgagaaa gtacttcatt gaagccagtc ccttgcccag cagttatccg ggaaaaacag 1200
ctctccgtgt gaaatggtcc catctcagac tgccctgggt agacctagac tggctcatag 1260
acatctcctg ccagatcacg gagctcgacc tttctgccaa ctgcctggcg accctcccct 1320
cggttatccc ctggggcctc atcaatctcc ggaagctgaa cctctccgac aaccacctgg 1380
gggagctgcc tggcgtgcag tcatcggacg aaatcatctg ttccaggcta cttgaaattg 1440
acatttccag caacaagttg tcccacctcc ctcctggatt cttgcacctc tcaaaacttc 1500
aaaaactgac agcttcaaaa aattgtttag aaaaattgtt cgaagaagaa aatgccacta 1560
actggatagg tttacggaag ctacaggaac ttgatatatc tgacaataaa ttgacagaac 1620
tccctgccct gttccttcac tctttcaagt ccctcaattc tctgaatgtc tccagaaaca 1680
acctgaaggt gtttccagat ccctgggcct gccctttgaa atgttgtaaa gcttccagaa 1740
atgccctgga atgtctgcca gacaaaatgg ctgtcttttg gaaaaatcac ctgaaggatg 1800
tggatttctc agaaaacgca ctcaaagaag ttcccctggg acttttccag cttgatgccc 1860
tcatgttctt gaggttacag gggaaccagc tggcggcact tccacctcaa gagaagtgga 1920
cctgcaggca gctcaaaacc ctggatctct ccagaaacca acttggcaaa aatgaagatg 1980
gactgaaaac gaagcgtatt gcctttttca ccaccagagg tcgccagcgc tccgggactg 2040
aggcagcaag tgtgctggaa tttccggcct tcctaagtga gtctttggaa gtcctttgcc 2100
tgaacgacaa ccacctcgac acagtccctc cctcggtttg cctactgaag agcttatcag 2160
agctctactt gggaaacaac cctggcctcc gggagctccc tcctgagctg gggcagctgg 2220
gcaacctctg gcagctggac actgaagacc tgaccatcag caatgtgcct gcagaaatcc 2280
aaaaagaagg ccccaaagca atgctgtctt acctgcgtgc tcagctgcgg aaagcggaaa 2340
agtgcaagct gatgaagatg atcatcgtgg gtcccccgcg ccagggcaag tccaccctcc 2400
tggagatctt acagacgggg agggcccccc aggtggtgca tggagaggcc accatcagga 2460
ccaccaagtg ggagctccag aggccggctg gctcaagagc caaggttgag tccgtggagt 2520
tcaacgtctg ggacatcggg ggaccggcca gcatggccac tgtcaaccag tgcttcttca 2580
cggacaaggc cctgtacgtg gtggtctgga acctggcgct gggggaggag gccgtggcca 2640
acctccagtt ctggctgctc aacatcgagg ccaaggcccc aaacgccgtg gtgctggtgg 2700
tcgggacgca cctggattta attgaagcca agttccgtgt ggaaaggatt gcaacgctgc 2760
gtgcctatgt gctggcactc tgccgctccc cctccggctc cagggccaca ggcttcccag 2820
acatcacctt caaacactta catgagattt cctgcaagag cctggaaggt caggaagggc 2880
tgcgacagct gattttccac gtcacgtgca gcatgaagga cgtcggcagc accatcggct 2940
gccaacgact ggcagggcgg ctgatcccca ggagctacct gagcctgcag gaggccgtgc 3000
tggcagagca gcagcgccgc agccgggacg acgacgtgca gtacctgacg gacargcagc 3060
tggagcagct ggtggagcag acgcccgaca acgacatcaa ggactacgag gacctgcagt 3120
cagccatcag cttcctcata gaaaccggca ccctgctcca tttcccggac accagccacg 3180
gcctgaggaa cctctacttc ctcgacccta tttggctctc cgaatgtctg cagaggatct 3240
ttaatattaa gggctctcga tcagtggcca agaatggggt gatcagagca gaagacctca 3300
ggatgctgct ggtggggact ggcttcacgc agcagacgga agagcagtac ttccagttcc 3360

tggccaagtt tgagatcgcc ctgcccgtcg ccaatgacag ctacctcctg ccccatctcc 3420
ttccatctaa acctggcctg gacacccacg gtatgcggca ccccacagcc aacaccattc 3480
agagggtatt taagatgagc ttcgttcccg ttggcttctg gcaaaggttt atagcacgga 3540
tgctgatcag cctggcggag atggacctgc agctttttga aaacaagaag aatactaaaa 3600
gcaggaacag gaaagtcacc atttacagtt ttacaggaaa ccagagaaat cgctgtagca 3660
cattcagagt gaaaagaaat cagaccatct attggcagga agggctcctg gtcacttttg 3720
atgggggcta cctcagtgtg gaatcttccg acgtgaactg gaaaaagaag aaaagcggag 3780
gaatgaaaat tgtttgccaa tcagaagtga gggacttctc agccatggct ttcatcacgg 3840
accacgtcaa ttccttgatt gatcagtggt ttcccgccct gacggccaca gagagcgacg 3900
ggacgccact catggagcag tacgtgccct gcccggtctg cgagacagcc tgggcccagc 3960
acacggaccc cagtgagaaa tcagaggatg tgcagtactt cgacatggaa gactgtgtcc 4020
tgacggccat cgagcgggac ttcatctcct gccccagaca cccggacctc cccgtgccgc 4080
tgcaggagct ggtccctgaa ctgttcatga ccgacttccc ggccaggctc ttcctggaga 4140
acagcaagct ggagcacagc gaggacgagg gcagcgtcct gggccagggc ggcagtggca 4200
ccgtcatcta ccgggcccgg taccagggcc agcctgtggc cgtcaagcgc ttccacatca 4260
aaaaattcaa gaactttgct aacgtaccgg cagacaccat gctgaggcac ctgcgggcca 4320
ccgatgccat gaagaacttc tccgagttcc ggcaggaggc cagcatgctg cacgcgctgc 4380
agcacccctg catcgtggcg ctcatcggca tcagcatcca cccgctctgc ttcgccctgg 4440
agctcgcgcc gctcagcagc ctcaacaccg tgctgtccga gaacgccaga gattcttcct 4500
ttatacccct gggacacatg ctcacccaaa aaatagccta ccagatcgcc tcgggcctgg 4560
cctacctgca caagaaaaac atcatcttct gtgacctgaa gtcggacaac attctggtgt 4620
ggtcccttga cgtcaaggag cacatcaaca tcaagctatc tgactacggg atttcgaggc 4680
agtcattcca tgagggcgcc ctaggcgtgg agggcactcc tggctaccag gccccagaga 4740
tcaggcctcg cattgtatat gatgagaagg tagatatgtt ctcctatgga atggtgctct 4800
acgagttgct gtcaggacag cgccctgcac tgggccacca ccagctccag attgccaaga 4860
agctgtccaa gggcatccgc ccggttctgg ggcagccgga ggaagtgcag ttccggcgac 4920
tgcaggcgct catgatggag tgctgggaca ctaagccaga gaagcgaccg ctggccctgt 4980
cggtggtgag ccagatgaag gacccgactt ttgccacctt catgtatgaa ctgtgctgtg 5040
ggaagcagac agccttcttc tcatcccagg gccaggagta cactgtggtg ttttgggatg 5100
gaaaagagga gtccaggaac tacacggtgg tgaacacaga gaagggcctc atggaggtgc 5160
agaggatgtg ctgccctggg atgaaggtga gctgccagct ccaggtccag agatccctgt 5220
ggacagccac cgaggaccag aaaatctaca tctacaccct caagggcatg tgccccttaa 5280
acacacccca acaggccttg gatactccag ctgtcgtcac ctgcttcttg gccgtgcctg 5340
ttattaaaaa gaattcctac ctggtcttag cgggcctcgc cgatgggctt gtggctgtgt 5400
ttcccgtggt gcggggcacc ccaaaggaca gctgctccta cctgtgctca cacacagcca 5460
acaggtccaa gttcagcatc gcggatgaag acgcacggca gaacccctac ccagtgaagg 5520
ccatggaggt ggtcaacagc ggctctgagg tctggtacag caatgggccg ggcctccttg 5580
tcatcgactg tgcctccctg gagatctgca ggcggctgga gccctacatg gccccctcca 5640
tggttacgtc agtcgtgtgc agctctgagg gcagagggga ggaggtcgtc tggtgcctgg 5700
atgacaaggc caactccttg gtgatgtacc actccaccac ctaccagytg tgtgcccggt 5760
acttctgcgg ggtccccagc cccctcaggg acatgtttcc cgtgcggccc ttggacacgg 5820
aacccccggc agccagccac acggccaacc caaaggtgcc tgagggggac tccatcgcgg 5880
acgtgagcat catgtacagt gaggagctgg gcacgcagat cctgatccac caggaatcac 5940
tcactgacta ctgctccatg tcctcctact cctcatcccc accccgccag gctgccaggt 6000
ccccctcaag cctccccagc tccccagcaa gttcttccag tgtgcctttc tccaccgact 6060
gcgaggactc agacatgcta catacgcccg gtgctgcctc cgacaggtct gagcatgacc 6120
tgacccccat ggacggggag accttcagcc agcacctgca ggccgtgaag atcctcgccg 6180
tcagagacct catttgggtc cccaggcgcg gtggagatgt tatcgtcatt ggcctggaga 6240
aggattctgg cgcccagcgg ggccgagtca ttgccgtctt aaaagcccga gagctgactc 6300
cgcatggggt gctggtggat gctgccgtgg tggcaaagga cactgttgtg tgcacctttg 6360
aaaatgaaaa cacagagtgg tgcctggccg tctggagggg ctggggcgcc agggagttcg 6420
acattttcta ccagtcctac gaggagctgg gccggctgga ggcttgcact cgcaagagaa 6480
ggtaattcct gaagggcgaa tcgcggcc 6508

<210> 14
<211> 2158
<212> PRT
<213> Homo sapiens

<400> 14
Gly Arg Arg Gly Asp Ala Ser Arg Phe Leu Gly Glu Leu Ala Ala Ala
1               5                   10                  15

Leu Leu Cys Ala Leu Phe Ala Pro Pro Ser Pro Pro Ala Arg Leu Arg
            20                  25                  30

Ile Glu Glu Val Arg Leu Gly Pro Ala Pro Ala Arg Gly Ala Asp Gly
        35                  40                  45

Gly Gly Thr Ala Arg Pro Arg Pro Arg Gln Cys Val Arg Pro Ala Pro
    50                  55                  60

Arg Pro Gly Ala Pro Ala Pro Gly Pro Ala Ala Ala Ser Glu Gln Glu
65                  70                  75                  80

Ser Phe Leu Leu Ser His Gly Tyr Glu Ser Thr Pro Cys Thr Pro Gln
                85                  90                  95

Pro Ala Ala Val Ala Val Thr Thr Ala Gly Pro Ala Phe Glu Asp Arg
            100                 105                 110

Leu Leu Gln Gly Leu Met Ala Gly Met Ser Gln Arg Pro Pro Ser Met
        115                 120                 125

Tyr Trp Cys Val Gly Pro Glu Glu Ser Ala Val Cys Pro Glu Arg Ala
    130                 135                 140

Met Glu Thr Leu Asn Gly Ala Gly Asp Thr Gly Gly Lys Pro Ser Thr
145                 150                 155                 160

Arg Gly Gly Asp Pro Ala Ala Arg Ser Arg Arg Thr Glu Gly Ile Arg
                165                 170                 175

Ala Ala Tyr Arg Arg Gly Asp Arg Gly Gly Ala Arg Asp Leu Leu Glu
            180                 185                 190

Glu Ala Cys Asp Gln Cys Ala Ser Gln Leu Glu Lys Gly Gln Leu Leu
        195                 200                 205

Ser Ile Pro Ala Ala Tyr Gly Asp Leu Glu Met Val Arg Tyr Leu Leu
    210                 215                 220

Ser Lys Arg Leu Val Glu Leu Pro Thr Glu Pro Thr Asp Asp Asn Pro
225                 230                 235                 240

Ala Val Val Ala Ala Tyr Phe Gly His Thr Ala Val Val Gln Asn Thr
                245                 250                 255

Leu Pro Thr Glu Pro Thr Asp Asp Asn Pro Ala Val Val Ala Ala Tyr
            260                 265                 270

Phe Gly His Thr Ala Val Val Gln Glu Leu Leu Glu Ser Leu Pro Gly
        275                 280                 285

Pro Cys Ser Pro Gln Arg Leu Leu Asn Trp Met Leu Ala Leu Ala Cys
    290                 295                 300

Gln Arg Gly His Leu Gly Val Val Lys Leu Leu Val Leu Thr His Gly
305                 310                 315                 320

Ala Asp Pro Glu Ser Tyr Ala Val Arg Lys Asn Glu Phe Pro Val Ile
                325                 330                 335

Val Arg Leu Pro Leu Tyr Ala Ala Ile Lys Ser Gly Asn Glu Asp Ile
            340                 345                 350

Ala Ile Phe Leu Leu Arg His Gly Ala Tyr Phe Cys Ser Tyr Ile Leu
        355                 360                 365

Leu Asp Ser Pro Asp Pro Ser Lys His Leu Leu Arg Lys Tyr Phe Ile
    370                 375                 380

Glu Ala Ser Pro Leu Pro Ser Ser Tyr Pro Gly Lys Thr Ala Leu Arg
385                 390                 395                 400

Val Lys Trp Ser His Leu Arg Leu Pro Trp Val Asp Leu Asp Trp Leu
                405                 410                 415

Ile Asp Ile Ser Cys Gln Ile Thr Glu Leu Asp Leu Ser Ala Asn Cys
            420                 425                 430

Leu Ala Thr Leu Pro Ser Val Ile Pro Trp Gly Leu Ile Asn Leu Arg
        435                 440                 445

Lys Leu Asn Leu Ser Asp Asn His Leu Gly Glu Leu Pro Gly Val Gln
    450                 455                 460

Ser Ser Asp Glu Ile Ile Cys Ser Arg Leu Leu Glu Ile Asp Ile Ser
465                 470                 475                 480

Ser Asn Lys Leu Ser His Leu Pro Pro Gly Phe Leu His Leu Ser Lys
                485                 490                 495

Leu Gln Lys Leu Thr Ala Ser Lys Asn Cys Leu Glu Lys Leu Phe Glu
            500                 505                 510

Glu Glu Asn Ala Thr Asn Trp Ile Gly Leu Arg Lys Leu Gln Glu Leu
        515                 520                 525

Asp Ile Ser Asp Asn Lys Leu Thr Glu Leu Pro Ala Leu Phe Leu His
    530                 535                 540

Ser Phe Lys Ser Leu Asn Ser Leu Asn Val Ser Arg Asn Asn Leu Lys
545                 550                 555                 560

Val Phe Pro Asp Pro Trp Ala Cys Pro Leu Lys Cys Cys Lys Ala Ser
                565                 570                 575

Arg Asn Ala Leu Glu Cys Leu Pro Asp Lys Met Ala Val Phe Trp Lys
            580                 585                 590

Asn His Leu Lys Asp Val Asp Phe Ser Glu Asn Ala Leu Lys Glu Val
        595                 600                 605

Pro Leu Gly Leu Phe Gln Leu Asp Ala Leu Met Phe Leu Arg Leu Gln
    610                 615                 620

Gly Asn Gln Leu Ala Ala Leu Pro Pro Gln Glu Lys Trp Thr Cys Arg
625                 630                 635                 640

Gln Leu Lys Thr Leu Asp Leu Ser Arg Asn Gln Leu Gly Lys Asn Glu
                645                 650                 655

Asp Gly Leu Lys Thr Lys Arg Ile Ala Phe Phe Thr Thr Arg Gly Arg
            660                 665                 670

Gln Arg Ser Gly Thr Glu Ala Ala Ser Val Leu Glu Phe Pro Ala Phe
        675                 680                 685

Leu Ser Glu Ser Leu Glu Val Leu Cys Leu Asn Asp Asn His Leu Asp
    690                 695                 700

Thr Val Pro Pro Ser Val Cys Leu Leu Lys Ser Leu Ser Glu Leu Tyr
705                 710                 715                 720

Leu Gly Asn Asn Pro Gly Leu Arg Glu Leu Pro Pro Glu Leu Gly Gln
                725                 730                 735

Leu Gly Asn Leu Trp Gln Leu Asp Thr Glu Asp Leu Thr Ile Ser Asn
            740                 745                 750

Val Pro Ala Glu Ile Gln Lys Glu Gly Pro Lys Ala Met Leu Ser Tyr
        755                 760                 765

Leu Arg Ala Gln Leu Arg Lys Ala Glu Lys Cys Lys Leu Met Lys Met
    770                 775                 780

Ile Ile Val Gly Pro Pro Arg Gln Gly Lys Ser Thr Leu Leu Glu Ile
785                 790                 795                 800

Leu Gln Thr Gly Arg Ala Pro Gln Val Val His Gly Glu Ala Thr Ile
                805                 810                 815

Arg Thr Thr Lys Trp Glu Leu Gln Arg Pro Ala Gly Ser Arg Ala Lys
            820                 825                 830

Val Glu Ser Val Glu Phe Asn Val Trp Asp Ile Gly Gly Pro Ala Ser
        835                 840                 845

Met Ala Thr Val Asn Gln Cys Phe Phe Thr Asp Lys Ala Leu Tyr Val
    850                 855                 860

Val Val Trp Asn Leu Ala Leu Gly Glu Glu Ala Val Ala Asn Leu Gln
865                 870                 875                 880

Phe Trp Leu Leu Asn Ile Glu Ala Lys Ala Pro Asn Ala Val Val Leu
                885                 890                 895

Val Val Gly Thr His Leu Asp Leu Ile Glu Ala Lys Phe Arg Val Glu
            900                 905                 910

Arg Ile Ala Thr Leu Arg Ala Tyr Val Leu Ala Leu Cys Arg Ser Pro
        915                 920                 925

Ser Gly Ser Arg Ala Thr Gly Phe Pro Asp Ile Thr Phe Lys His Leu
    930                 935                 940

His Glu Ile Ser Cys Lys Ser Leu Glu Gly Gln Glu Gly Leu Arg Gln
945                 950                 955                 960

Leu Ile Phe His Val Thr Cys Ser Met Lys Asp Val Gly Ser Thr Ile
                965                 970                 975

Gly Cys Gln Arg Leu Ala Gly Arg Leu Ile Pro Arg Ser Tyr Leu Ser
            980                 985                 990

Leu Gln Glu Ala Val Leu Ala Glu Gln Gln Arg Arg Ser Arg Asp Asp
        995                 1000                1005

Asp Val Gln Tyr Leu Thr Asp Xaa Gln Leu Glu Gln Leu Val Glu Gln
    1010                1015                1020

Thr Pro Asp Asn Asp Ile Lys Asp Tyr Glu Asp Leu Gln Ser Ala Ile
1025                1030                1035                1040

Ser Phe Leu Ile Glu Thr Gly Thr Leu Leu His Phe Pro Asp Thr Ser
                1045                1050                1055

His Gly Leu Arg Asn Leu Tyr Phe Leu Asp Pro Ile Trp Leu Ser Glu
            1060                1065                1070

Cys Leu Gln Arg Ile Phe Asn Ile Lys Gly Ser Arg Ser Val Ala Lys
        1075                1080                1085

Asn Gly Val Ile Arg Ala Glu Asp Leu Arg Met Leu Leu Val Gly Thr
    1090                1095                1100

Gly Phe Thr Gln Gln Thr Glu Glu Gln Tyr Phe Gln Phe Leu Ala Lys
1105                1110                1115                1120

Phe Glu Ile Ala Leu Pro Val Ala Asn Asp Ser Tyr Leu Leu Pro His
                1125                1130                1135

Leu Leu Pro Ser Lys Pro Gly Leu Asp Thr His Gly Met Arg His Pro
            1140                1145                1150

Thr Ala Asn Thr Ile Gln Arg Val Phe Lys Met Ser Phe Val Pro Val
        1155                1160                1165

Gly Phe Trp Gln Arg Phe Ile Ala Arg Met Leu Ile Ser Leu Ala Glu
    1170                1175                1180

Met Asp Leu Gln Leu Phe Glu Asn Lys Lys Asn Thr Lys Ser Arg Asn
1185                1190                1195                1200

Arg Lys Val Thr Ile Tyr Ser Phe Thr Gly Asn Gln Arg Asn Arg Cys
                1205                1210                1215

Ser Thr Phe Arg Val Lys Arg Asn Gln Thr Ile Tyr Trp Gln Glu Gly
            1220                1225                1230

Leu Leu Val Thr Phe Asp Gly Gly Tyr Leu Ser Val Glu Ser Ser Asp
        1235                1240                1245

Val Asn Trp Lys Lys Lys Lys Ser Gly Gly Met Lys Ile Val Cys Gln
    1250                1255                1260

Ser Glu Val Arg Asp Phe Ser Ala Met Ala Phe Ile Thr Asp His Val
1265                1270                1275                1280

Asn Ser Leu Ile Asp Gln Trp Phe Pro Ala Leu Thr Ala Thr Glu Ser
                1285                1290                1295

Asp Gly Thr Pro Leu Met Glu Gln Tyr Val Pro Cys Pro Val Cys Glu
            1300                1305                1310

Thr Ala Trp Ala Gln His Thr Asp Pro Ser Glu Lys Ser Glu Asp Val
        1315                1320                1325

Gln Tyr Phe Asp Met Glu Asp Cys Val Leu Thr Ala Ile Glu Arg Asp
    1330                1335                1340

Phe Ile Ser Cys Pro Arg His Pro Asp Leu Pro Val Pro Leu Gln Glu
1345                1350                1355                1360

Leu Val Pro Glu Leu Phe Met Thr Asp Phe Pro Ala Arg Leu Phe Leu
            1365                1370                1375

Glu Asn Ser Lys Leu Glu His Ser Glu Asp Glu Gly Ser Val Leu Gly
        1380                1385                1390

Gln Gly Gly Ser Gly Thr Val Ile Tyr Arg Ala Arg Tyr Gln Gly Gln
    1395                1400                1405

Pro Val Ala Val Lys Arg Phe His Ile Lys Lys Phe Lys Asn Phe Ala
  1410                1415                1420

Asn Val Pro Ala Asp Thr Met Leu Arg His Leu Arg Ala Thr Asp Ala
1425                1430                1435                1440

Met Lys Asn Phe Ser Glu Phe Arg Gln Glu Ala Ser Met Leu His Ala
            1445                1450                1455

Leu Gln His Pro Cys Ile Val Ala Leu Ile Gly Ile Ser Ile His Pro
        1460                1465                1470

Leu Cys Phe Ala Leu Glu Leu Ala Pro Leu Ser Ser Leu Asn Thr Val
    1475                1480                1485

Leu Ser Glu Asn Ala Arg Asp Ser Ser Phe Ile Pro Leu Gly His Met
  1490                1495                1500

Leu Thr Gln Lys Ile Ala Tyr Gln Ile Ala Ser Gly Leu Ala Tyr Leu
1505                1510                1515                1520

His Lys Lys Asn Ile Ile Phe Cys Asp Leu Lys Ser Asp Asn Ile Leu
            1525                1530                1535

Val Trp Ser Leu Asp Val Lys Glu His Ile Asn Ile Lys Leu Ser Asp
        1540                1545                1550

Tyr Gly Ile Ser Arg Gln Ser Phe His Glu Gly Ala Leu Gly Val Glu
    1555                1560                1565

Gly Thr Pro Gly Tyr Gln Ala Pro Glu Ile Arg Pro Arg Ile Val Tyr
  1570                1575                1580

Asp Glu Lys Val Asp Met Phe Ser Tyr Gly Met Val Leu Tyr Glu Leu
1585                1590                1595                1600

Leu Ser Gly Gln Arg Pro Ala Leu Gly His His Gln Leu Gln Ile Ala
            1605                1610                1615

Lys Lys Leu Ser Lys Gly Ile Arg Pro Val Leu Gly Gln Pro Glu Glu
        1620                1625                1630

Val Gln Phe Arg Arg Leu Gln Ala Leu Met Met Glu Cys Trp Asp Thr
    1635                1640                1645

Lys Pro Glu Lys Arg Pro Leu Ala Leu Ser Val Val Ser Gln Met Lys
  1650                1655                1660

Asp Pro Thr Phe Ala Thr Phe Met Tyr Glu Leu Cys Cys Gly Lys Gln
1665                1670                1675                1680

Thr Ala Phe Phe Ser Ser Gln Gly Gln Glu Tyr Thr Val Val Phe Trp
            1685                1690                1695

Asp Gly Lys Glu Glu Ser Arg Asn Tyr Thr Val Val Asn Thr Glu Lys
            1700                1705                1710

Gly Leu Met Glu Val Gln Arg Met Cys Cys Pro Gly Met Lys Val Ser
        1715                1720                1725

Cys Gln Leu Gln Val Gln Arg Ser Leu Trp Thr Ala Thr Glu Asp Gln
    1730                1735                1740

Lys Ile Tyr Ile Tyr Thr Leu Lys Gly Met Cys Pro Leu Asn Thr Pro
1745                1750                1755                1760

Gln Gln Ala Leu Asp Thr Pro Ala Val Val Thr Cys Phe Leu Ala Val
                1765                1770                1775

Pro Val Ile Lys Lys Asn Ser Tyr Leu Val Leu Ala Gly Leu Ala Asp
            1780                1785                1790

Gly Leu Val Ala Val Phe Pro Val Val Arg Gly Thr Pro Lys Asp Ser
        1795                1800                1805

Cys Ser Tyr Leu Cys Ser His Thr Ala Asn Arg Ser Lys Phe Ser Ile
    1810                1815                1820

Ala Asp Glu Asp Ala Arg Gln Asn Pro Tyr Pro Val Lys Ala Met Glu
1825                1830                1835                1840

Val Val Asn Ser Gly Ser Glu Val Trp Tyr Ser Asn Gly Pro Gly Leu
                1845                1850                1855

Leu Val Ile Asp Cys Ala Ser Leu Glu Ile Cys Arg Arg Leu Glu Pro
            1860                1865                1870

Tyr Met Ala Pro Ser Met Val Thr Ser Val Val Cys Ser Ser Glu Gly
        1875                1880                1885

Arg Gly Glu Glu Val Val Trp Cys Leu Asp Asp Lys Ala Asn Ser Leu
    1890                1895                1900

Val Met Tyr His Ser Thr Thr Tyr Gln Leu Cys Ala Arg Tyr Phe Cys
1905                1910                1915                1920

Gly Val Pro Ser Pro Leu Arg Asp Met Phe Pro Val Arg Pro Leu Asp
                1925                1930                1935

Thr Glu Pro Pro Ala Ala Ser His Thr Ala Asn Pro Lys Val Pro Glu
            1940                1945                1950

Gly Asp Ser Ile Ala Asp Val Ser Ile Met Tyr Ser Glu Glu Leu Gly
        1955                1960                1965

Thr Gln Ile Leu Ile His Gln Glu Ser Leu Thr Asp Tyr Cys Ser Met
    1970                1975                1980

Ser Ser Tyr Ser Ser Ser Pro Pro Arg Gln Ala Ala Arg Ser Pro Ser
1985                1990                1995                2000

Ser Leu Pro Ser Ser Pro Ala Ser Ser Ser Ser Val Pro Phe Ser Thr
                2005                2010                2015

Asp Cys Glu Asp Ser Asp Met Leu His Thr Pro Gly Ala Ala Ser Asp
            2020                2025                2030

Arg Ser Glu His Asp Leu Thr Pro Met Asp Gly Glu Thr Phe Ser Gln
        2035                2040                2045

His Leu Gln Ala Val Lys Ile Leu Ala Val Arg Asp Leu Ile Trp Val
    2050                2055                2060

Pro Arg Arg Gly Gly Asp Val Ile Val Ile Gly Leu Glu Lys Asp Ser
2065                2070                2075                2080

Gly Ala Gln Arg Gly Arg Val Ile Ala Val Leu Lys Ala Arg Glu Leu
                2085                2090                2095

Thr Pro His Gly Val Leu Val Asp Ala Ala Val Val Ala Lys Asp Thr
            2100                2105                2110

Val Val Cys Thr Phe Glu Asn Glu Asn Thr Glu Trp Cys Leu Ala Val
        2115                2120                2125

Trp Arg Gly Trp Gly Ala Arg Glu Phe Asp Ile Phe Tyr Gln Ser Tyr
    2130                2135                2140

Glu Glu Leu Gly Arg Leu Glu Ala Cys Thr Arg Lys Arg Arg
2145                2150                2155

<210> 15
<211> 6430
<212> DNA
<213> Homo sapiens

<400> 15
gcggccgccg aggggacgcc tcgtgacggt tcctgggaga gctggcggcg gccttgctct 60
gcgcgctctt cgcgccgccc tccccgcccg cccgcctcag gattgaggaa gtgcgtctgg 120
gcccggcccc ggcgcggggg gcagacggcg gtgggacggc caggccccgg ccccgccagt 180
gtgtccgccc ggccccgcgt cccggagcgc ccgcacccgg ccccgccgcc gcctcagagc 240
aagaaagctt tctgctcagc catggctacg agtccacgcc ttaatgcacc ccacagccag 300
cggcagtggc agtgacaaca gcgggacctg cctttgaaga tcggctgctg caagggttga 360
tggctggcat gtcgcaaaga ccccccagca tgtactggtg tgtggggccg gaggagtcag 420
ctgtgtgtcc agaacgtgcc atggagacgc ttaacggtgc cggggacacg ggcggcaagc 480
cgtccacgcg gggcggtgac cctgcagcgc ggtcccgcag gacggaaggc atccgcgccg 540
cgtacaggcg gggagaccgc ggcggcgccc gggacctgct ggaggaggcc tgcgaccagt 600
gcgcgtccca gctggaaaag ggccagcttc tgagcatccc ggcagcctat ggggatctgg 660
agatggtccg ctacctactc agcaagagac tggtggagct gcccaccgag cccacggatg 720
acaacccagc cgtggtggca gcgtattttg gacacacggc agttgtgcag gaattgcttg 780
agtccttacc aggtccctgc agtccccagc ggcttctgaa ctggatgctg gccttggctt 840
gccagcgagg gcacctgggg gttgtgaagc tcctggtcct gacgcacggg gctgacccgg 900
agagctacgc tgtcaggaag aatgagttcc ctgtcatcgt gcgcttgccc ctgtatgcgg 960
ccatcaagtc agggaatgaa gacattgcaa tattcctgct tcggcatggg gcctatttct 1020
gttcctacat cttgctggat agtcctgacc ccagcaaaca tctgctgaga aagtacttca 1080
ttgaagccag tcccttgccc agcagttatc cgggaaaaac agctctccgt gtgaaatggt 1140
cccatctcag actgccctgg gtagacctag actggctcat agacatctcc tgccagatca 1200
cggagctcga cctttctgcc aactgcctgg cgaccctccc ctcggttatc ccctggggcc 1260
tcatcaatct ccggaagctg aacctctccg acaaccacct gggggagctg cctggcgtgc 1320
agtcatcgga cgaaatcatc tgttccaggc tacttgaaat tgacatttcc agcaacaagt 1380
tgtcccacct ccctcctgga ttcttgcacc tctcaaaact tcaaaaactg acagcttcaa 1440
aaaattgttt agaaaaattg ttcgaagaag aaaatgccac taactggata ggtttacgga 1500
agctacagga acttgatata tctgacaata aattgacaga actccctgcc ctgttccttc 1560
actctttcaa gtccctcaat tctctgaatg tctccagaaa caacctgaag gtgtttccag 1620
atccctgggc ctgccctttg aaatgttgta aagcttccag aaatgccctg gaatgtctgc 1680
cagacaaaat ggctgtcttt tggaaaaatc acctgaagga tgtggatttc tcagaaaacg 1740
cactcaaaga agttcccctg ggacttttcc agcttgatgc cctcatgttc ttgaggttac 1800
aggggaacca gctggcggca cttccacctc aagagaagtg gacctgcagg cagctcaaaa 1860

ccctggatct ctccagaaac caacttggca aaaatgaaga tggactgaaa acgaagcgta 1920
ttgccttttt caccaccaga ggtcgccagc gctccgggac tgaggcagca agtgtgctgg 1980
aatttccggc cttcctaagt gagtctttgg aagtcctttg cctgaacgac aaccacctcg 2040
acacagtccc tccctcggtt tgcctactga agagcttatc agagctctac ttgggaaaca 2100
accctggcct ccgggagctc cctcctgagc tggggcagct gggcaacctc tggcagctgg 2160
acactgaaga cctgaccatc agcaatgtgc ctgcagaaat ccaaaaagaa ggccccaaag 2220
caatgctgtc ttacctgcgt gctcagctgc ggaaagcgga aaagtgcaag ctgatgaaga 2280
tgatcatcgt gggtcccccg cgccagggca agtccaccct cctggagatc ttacagacgg 2340
ggagggcccc ccaggtggtg catggagagg ccaccatcag gaccaccaag tgggagctcc 2400
agaggccggc tggctcaaga gccaaggttg agtccgtgga gttcaacgtc tgggacatcg 2460
ggggaccggc cagcatggcc actgtcaacc agtgcttctt cacggacaag gccctgtacg 2520
tggtggtctg gaacctggcg ctgggggagg aggccgtggc caacctccag ttctggctgc 2580
tcaacatcga ggccaaggcc ccaaacgccg tggtgctggt ggtcgggacg cacctggatt 2640
taattgaagc caagttccgt gtggaaagga ttgcaacgct gcgtgcctat gtgctggcac 2700
tctgccgctc cccctccggc tccagggcca caggcttccc agacatcacc ttcaaacact 2760
tacatgagat ttcctgcaag agcctggaag gtcaggaagg gctgcgacag ctgattttcc 2820
acgtcacgtg cagcatgaag gacgtcggca gcaccatcgg ctgccaacga ctggcagggc 2880
ggctgatccc caggagctac ctgagcctgc aggaggccgt gctggcagag cagcagcgcc 2940
gcagccggga cgacgacgtg cagtacctga cggacargca gctggagcag ctggtggagc 3000
agacgcccga caacgacatc aaggactacg aggacctgca gtcagccatc agcttcctca 3060
tagaaaccgg caccctgctc catttcccgg acaccagcca cggcctgagg aacctctact 3120
tcctcgaccc tatttggctc tccgaatgtc tgcagaggat ctttaatatt aagggctctc 3180
gatcagtggc caagaatggg gtgatcagag cagaagacct caggatgctg ctggtgggga 3240
ctggcttcac gcagcagacg gaagagcagt acttccagtt cctggccaag tttgagatcg 3300
ccctgcccgt cgccaatgac agctacctcc tgccccatct ccttccatct aaacctggcc 3360
tggacaccca cggtatgcgg caccccacag ccaacaccat tcagagggta tttaagatga 3420
gcttcgttcc cgttggcttc tggcaaaggt ttatagcacg gatgctgatc agcctggcgg 3480
agatggacct gcagcttttt gaaaacaaga agaatactaa aagcaggaac aggaaagtca 3540
ccatttacag ttttacagga aaccagagaa atcgctgtag cacattcaga gtgaaaagaa 3600
atcagaccat ctattggcag gaagggctcc tggtcacttt tgatgggggc tacctcagtg 3660
tggaatcttc cgacgtgaac tggaaaaaga agaaaagcgg aggaatgaaa attgtttgcc 3720
aatcagaagt gagggacttc tcagccatgg ctttcatcac ggaccacgtc aattccttga 3780
ttgatcagtg gtttcccgcc ctgacggcca cagagagcga cgggacgcca ctcatggagc 3840
agtacgtgcc ctgcccggtc tgcgagacag cctgggccca gcacacggac cccagtgaga 3900
aatcagagga tgtgcagtac ttcgacatgg aagactgtgt cctgacggcc atcgagcggg 3960
acttcatctc ctgccccaga cacccggacc tccccgtgcc gctgcaggag ctggtccctg 4020
aactgttcat gaccgacttc ccggccaggc tcttcctgga gaacagcaag ctggagcaca 4080
gcgaggacga gggcagcgtc ctgggccagg gcggcagtgg caccgtcatc taccgggccc 4140
ggtaccaggg ccagcctgtg gccgtcaagc gcttccacat caaaaaattc aagaactttg 4200
ctaacgtacc ggcagacacc atgctgaggc acctgcgggc caccgatgcc atgaagaact 4260
tctccgagtt ccggcaggag gccagcatgc tgcacgcgct gcagcacccc tgcatcgtgg 4320
cgctcatcgg catcagcatc cacccgctct gcttcgccct ggagctcgcg ccgctcagca 4380
gcctcaacac cgtgctgtcc gagaacgcca gagattcttc ctttataccc ctgggacaca 4440
tgctcaccca aaaaatagcc taccagatcg cctcgggcct ggcctacctg cacaagaaaa 4500
acatcatctt ctgtgacctg aagtcggaca acattctggt gtggtccctt gacgtcaagg 4560
agcacatcaa catcaagcta tctgactacg ggatttcgag gcagtcattc catgagggcg 4620
ccctaggcgt ggagggcact cctggctacc aggccccaga gatcaggcct cgcattgtat 4680
atgatgagaa ggtagatatg ttctcctatg gaatggtgct ctacgagttg ctgtcaggac 4740
agcgccctgc actgggccac caccagctcc agattgccaa gaagctgtcc aagggcatcc 4800
gcccggttct ggggcagccg gaggaagtgc agttccggcg actgcaggcg ctcatgatgg 4860
agtgctggga cactaagcca gagaagcgac cgctggccct gtcggtggtg agccagatga 4920
aggacccgac ttttgccacc ttcatgtatg aactgtgctg tgggaagcag acagccttct 4980
tctcatccca gggccaggag tacactgtgg tgttttggga tggaaaagag gagtccagga 5040
actacacggt ggtgaacaca gagaagggcc tcatggaggt gcagaggatg tgctgccctg 5100
ggatgaaggt gagctgccag ctccaggtcc agagatccct gtggacagcc accgaggacc 5160
agaaaatcta catctacacc ctcaagggca tgtgcccctt aaacacaccc caacaggcct 5220
tggatactcc agctgtcgtc acctgcttct tggccgtgcc tgttattaaa aagaattcct 5280
acctggtctt agcgggcctc gccgatgggc ttgtggctgt gtttcccgtg gtgcggggca 5340
ccccaaagga cagctgctcc tacctgtgct cacacacagc caacaggtcc aagttcagca 5400
tcgcggatga agacgcacgg cagaacccct acccagtgaa ggccatggag gtggtcaaca 5460
gcggctctga ggtctggtac agcaatgggc cgggcctcct tgtcatcgac tgtgcctccc 5520
tggagatctg caggcggctg gagccctaca tggccccctc catggttacg tcagtcgtgt 5580
gcagctctga gggcagaggg gaggaggtcg tctggtgcct ggatgacaag gccaactcct 5640

tggtgatgta ccactccacc acctaccagy tgtgtgcccg gtacttctgc ggggtcccca 5700
gccccctcag ggacatgttt cccgtgcggc ccttggacac ggaacccccg gcagccagcc 5760
acacggccaa cccaaaggtg cctgaggggg actccatcgc ggacgtgagc atcatgtaca 5820
gtgaggagct gggcacgcag atcctgatcc accaggaatc actcactgac tactgctcca 5880
tgtcctccta ctcctcatcc ccaccccgcc aggctgccag gtccccctca agcctcccca 5940
gctccccagc aagttcttcc agtgtgcctt tctccaccga ctgcgaggac tcagacatgc 6000
tacatacgcc cggtgctgcc tccgacaggt ctgagcatga cctgaccccc atggacgggg 6060
agaccttcag ccagcacctg caggccgtga agatcctcgc cgtcagagac ctcatttggg 6120
tccccaggcg cggtggagat gttatcgtca ttggcctgga gaaggattct ggcgcccagc 6180
ggggccgagt cattgccgtc ttaaaagccc gagagctgac tccgcatggg gtgctggtgg 6240
atgctgccgt ggtggcaaag gacactgttg tgtgcacctt tgaaaatgaa aacacagagt 6300
ggtgcctggc cgtctggagg ggctggggcg ccagggagtt cgacattttc taccagtcct 6360
acgaggagct gggccggctg gaggcttgca ctcgcaagag aaggtaattc ctgaagggcg 6420
aatcgcggcc 6430

<210> 16
<211> 2132
<212> PRT
<213> Homo sapiens

<400> 16
Gly Arg Arg Gly Asp Ala Ser Arg Phe Leu Gly Glu Leu Ala Ala Ala
1               5                   10                  15

Leu Leu Cys Ala Leu Phe Ala Pro Pro Ser Pro Pro Ala Arg Leu Arg
            20                  25                  30

Ile Glu Glu Val Arg Leu Gly Pro Ala Pro Ala Arg Gly Ala Asp Gly
        35                  40                  45

Gly Gly Thr Ala Arg Pro Arg Pro Arg Gln Cys Val Arg Pro Ala Pro
    50                  55                  60

Arg Pro Gly Ala Pro Ala Pro Gly Pro Ala Ala Ala Ser Glu Gln Glu
65                  70                  75                  80

Ser Phe Leu Leu Ser His Gly Tyr Glu Ser Thr Pro Cys Thr Pro Gln
                85                  90                  95

Pro Ala Ala Val Ala Val Thr Thr Ala Gly Pro Ala Phe Glu Asp Arg
            100                 105                 110

Leu Leu Gln Gly Leu Met Ala Gly Met Ser Gln Arg Pro Pro Ser Met
        115                 120                 125

Tyr Trp Cys Val Gly Pro Glu Glu Ser Ala Val Cys Pro Glu Arg Ala
    130                 135                 140

Met Glu Thr Leu Asn Gly Ala Gly Asp Thr Gly Gly Lys Pro Ser Thr
145                 150                 155                 160

Arg Gly Gly Asp Pro Ala Ala Arg Ser Arg Arg Thr Glu Gly Ile Arg
                165                 170                 175

Ala Ala Tyr Arg Arg Gly Asp Arg Gly Gly Ala Arg Asp Leu Leu Glu
            180                 185                 190

Glu Ala Cys Asp Gln Cys Ala Ser Gln Leu Glu Lys Gly Gln Leu Leu
        195                 200                 205

Ser Ile Pro Ala Ala Tyr Gly Asp Leu Glu Met Val Arg Tyr Leu Leu
    210                 215                 220

Ser Lys Arg Leu Val Glu Leu Pro Thr Glu Pro Thr Asp Asp Asn Pro
225                 230                 235                 240

Ala Val Val Ala Ala Tyr Phe Gly His Thr Ala Val Val Gln Glu Leu
                245                 250                 255

Leu Glu Ser Leu Pro Gly Pro Cys Ser Pro Gln Arg Leu Leu Asn Trp
            260                 265                 270

Met Leu Ala Leu Ala Cys Gln Arg Gly His Leu Gly Val Val Lys Leu
        275                 280                 285

Leu Val Leu Thr His Gly Ala Asp Pro Glu Ser Tyr Ala Val Arg Lys
    290                 295                 300

Asn Glu Phe Pro Val Ile Val Arg Leu Pro Leu Tyr Ala Ala Ile Lys
305                 310                 315                 320

Ser Gly Asn Glu Asp Ile Ala Ile Phe Leu Leu Arg His Gly Ala Tyr
                325                 330                 335

Phe Cys Ser Tyr Ile Leu Leu Asp Ser Pro Asp Pro Ser Lys His Leu
            340                 345                 350

Leu Arg Lys Tyr Phe Ile Glu Ala Ser Pro Leu Pro Ser Ser Tyr Pro
        355                 360                 365

Gly Lys Thr Ala Leu Arg Val Lys Trp Ser His Leu Arg Leu Pro Trp
    370                 375                 380

Val Asp Leu Asp Trp Leu Ile Asp Ile Ser Cys Gln Ile Thr Glu Leu
385                 390                 395                 400

Asp Leu Ser Ala Asn Cys Leu Ala Thr Leu Pro Ser Val Ile Pro Trp
                405                 410                 415

Gly Leu Ile Asn Leu Arg Lys Leu Asn Leu Ser Asp Asn His Leu Gly
            420                 425                 430

Glu Leu Pro Gly Val Gln Ser Ser Asp Glu Ile Ile Cys Ser Arg Leu
        435                 440                 445

Leu Glu Ile Asp Ile Ser Ser Asn Lys Leu Ser His Leu Pro Pro Gly
    450                 455                 460

Phe Leu His Leu Ser Lys Leu Gln Lys Leu Thr Ala Ser Lys Asn Cys
465                 470                 475                 480

Leu Glu Lys Leu Phe Glu Glu Glu Asn Ala Thr Asn Trp Ile Gly Leu
                485                 490                 495

Arg Lys Leu Gln Glu Leu Asp Ile Ser Asp Asn Lys Leu Thr Glu Leu
            500                 505                 510

Pro Ala Leu Phe Leu His Ser Phe Lys Ser Leu Asn Ser Leu Asn Val
        515                 520                 525

Ser Arg Asn Asn Leu Lys Val Phe Pro Asp Pro Trp Ala Cys Pro Leu
    530                 535                 540

Lys Cys Cys Lys Ala Ser Arg Asn Ala Leu Glu Cys Leu Pro Asp Lys
545                 550                 555                 560

Met Ala Val Phe Trp Lys Asn His Leu Lys Asp Val Asp Phe Ser Glu
                565                 570                 575

Asn Ala Leu Lys Glu Val Pro Leu Gly Leu Phe Gln Leu Asp Ala Leu
            580                 585                 590

Met Phe Leu Arg Leu Gln Gly Asn Gln Leu Ala Ala Leu Pro Pro Gln
        595                 600                 605

Glu Lys Trp Thr Cys Arg Gln Leu Lys Thr Leu Asp Leu Ser Arg Asn
    610                 615                 620

Gln Leu Gly Lys Asn Glu Asp Gly Leu Lys Thr Lys Arg Ile Ala Phe
625                 630                 635                 640

Phe Thr Thr Arg Gly Arg Gln Arg Ser Gly Thr Glu Ala Ala Ser Val
                645                 650                 655

Leu Glu Phe Pro Ala Phe Leu Ser Glu Ser Leu Glu Val Leu Cys Leu
            660                 665                 670

Asn Asp Asn His Leu Asp Thr Val Pro Pro Ser Val Cys Leu Leu Lys
        675                 680                 685

Ser Leu Ser Glu Leu Tyr Leu Gly Asn Asn Pro Gly Leu Arg Glu Leu
    690                 695                 700

Pro Pro Glu Leu Gly Gln Leu Gly Asn Leu Trp Gln Leu Asp Thr Glu
705                 710                 715                 720

Asp Leu Thr Ile Ser Asn Val Pro Ala Glu Ile Gln Lys Glu Gly Pro
                725                 730                 735

Lys Ala Met Leu Ser Tyr Leu Arg Ala Gln Leu Arg Lys Ala Glu Lys
            740                 745                 750

Cys Lys Leu Met Lys Met Ile Ile Val Gly Pro Pro Arg Gln Gly Lys
        755                 760                 765

Ser Thr Leu Leu Glu Ile Leu Gln Thr Gly Arg Ala Pro Gln Val Val
    770                 775                 780

His Gly Glu Ala Thr Ile Arg Thr Thr Lys Trp Glu Leu Gln Arg Pro
785                 790                 795                 800

Ala Gly Ser Arg Ala Lys Val Glu Ser Val Glu Phe Asn Val Trp Asp
                805                 810                 815

Ile Gly Gly Pro Ala Ser Met Ala Thr Val Asn Gln Cys Phe Phe Thr
            820                 825                 830

Asp Lys Ala Leu Tyr Val Val Val Trp Asn Leu Ala Leu Gly Glu Glu
        835                 840                 845

Ala Val Ala Asn Leu Gln Phe Trp Leu Leu Asn Ile Glu Ala Lys Ala
    850                 855                 860

Pro Asn Ala Val Val Leu Val Val Gly Thr His Leu Asp Leu Ile Glu
865                 870                 875                 880

Ala Lys Phe Arg Val Glu Arg Ile Ala Thr Leu Arg Ala Tyr Val Leu
                885                 890                 895

Ala Leu Cys Arg Ser Pro Ser Gly Ser Arg Ala Thr Gly Phe Pro Asp
            900                 905                 910

Ile Thr Phe Lys His Leu His Glu Ile Ser Cys Lys Ser Leu Glu Gly
        915                 920                 925

Gln Glu Gly Leu Arg Gln Leu Ile Phe His Val Thr Cys Ser Met Lys
    930                 935                 940

Asp Val Gly Ser Thr Ile Gly Cys Gln Arg Leu Ala Gly Arg Leu Ile
945                 950                 955                 960

Pro Arg Ser Tyr Leu Ser Leu Gln Glu Ala Val Leu Ala Glu Gln Gln
                965                 970                 975

Arg Arg Ser Arg Asp Asp Asp Val Gln Tyr Leu Thr Asp Xaa Gln Leu
            980                 985                 990

Glu Gln Leu Val Glu Gln Thr Pro Asp Asn Asp Ile Lys Asp Tyr Glu
        995                 1000                1005

Asp Leu Gln Ser Ala Ile Ser Phe Leu Ile Glu Thr Gly Thr Leu Leu
    1010                1015                1020

His Phe Pro Asp Thr Ser His Gly Leu Arg Asn Leu Tyr Phe Leu Asp
1025                1030                1035                1040

Pro Ile Trp Leu Ser Glu Cys Leu Gln Arg Ile Phe Asn Ile Lys Gly
                1045                1050                1055

Ser Arg Ser Val Ala Lys Asn Gly Val Ile Arg Ala Glu Asp Leu Arg
            1060                1065                1070

Met Leu Leu Val Gly Thr Gly Phe Thr Gln Gln Thr Glu Glu Gln Tyr
        1075                1080                1085

Phe Gln Phe Leu Ala Lys Phe Glu Ile Ala Leu Pro Val Ala Asn Asp
    1090                1095                1100

Ser Tyr Leu Leu Pro His Leu Leu Pro Ser Lys Pro Gly Leu Asp Thr
1105                1110                1115                1120

His Gly Met Arg His Pro Thr Ala Asn Thr Ile Gln Arg Val Phe Lys
                1125                1130                1135

Met Ser Phe Val Pro Val Gly Phe Trp Gln Arg Phe Ile Ala Arg Met
            1140                1145                1150

Leu Ile Ser Leu Ala Glu Met Asp Leu Gln Leu Phe Glu Asn Lys Lys
        1155                1160                1165

Asn Thr Lys Ser Arg Asn Arg Lys Val Thr Ile Tyr Ser Phe Thr Gly
    1170                1175                1180

Asn Gln Arg Asn Arg Cys Ser Thr Phe Arg Val Lys Arg Asn Gln Thr
1185                1190                1195                1200

Ile Tyr Trp Gln Glu Gly Leu Leu Val Thr Phe Asp Gly Gly Tyr Leu
                1205                1210                1215

Ser Val Glu Ser Ser Asp Val Asn Trp Lys Lys Lys Lys Ser Gly Gly
            1220                1225                1230

Met Lys Ile Val Cys Gln Ser Glu Val Arg Asp Phe Ser Ala Met Ala
        1235                1240                1245

Phe Ile Thr Asp His Val Asn Ser Leu Ile Asp Gln Trp Phe Pro Ala
    1250                1255                1260

Leu Thr Ala Thr Glu Ser Asp Gly Thr Pro Leu Met Glu Gln Tyr Val
1265                1270                1275                1280

Pro Cys Pro Val Cys Glu Thr Ala Trp Ala Gln His Thr Asp Pro Ser
                1285                1290                1295

Glu Lys Ser Glu Asp Val Gln Tyr Phe Asp Met Glu Asp Cys Val Leu
            1300                1305                1310

Thr Ala Ile Glu Arg Asp Phe Ile Ser Cys Pro Arg His Pro Asp Leu
        1315                1320                1325

Pro Val Pro Leu Gln Glu Leu Val Pro Glu Leu Phe Met Thr Asp Phe
    1330                1335                1340

Pro Ala Arg Leu Phe Leu Glu Asn Ser Lys Leu Glu His Ser Glu Asp
1345                1350                1355                1360

Glu Gly Ser Val Leu Gly Gln Gly Gly Ser Gly Thr Val Ile Tyr Arg
                1365                1370                1375

Ala Arg Tyr Gln Gly Gln Pro Val Ala Val Lys Arg Phe His Ile Lys
            1380                1385                1390

Lys Phe Lys Asn Phe Ala Asn Val Pro Ala Asp Thr Met Leu Arg His
        1395                1400                1405

Leu Arg Ala Thr Asp Ala Met Lys Asn Phe Ser Glu Phe Arg Gln Glu
    1410                1415                1420

Ala Ser Met Leu His Ala Leu Gln His Pro Cys Ile Val Ala Leu Ile
1425                1430                1435                1440

Gly Ile Ser Ile His Pro Leu Cys Phe Ala Leu Glu Leu Ala Pro Leu
                1445                1450                1455

Ser Ser Leu Asn Thr Val Leu Ser Glu Asn Ala Arg Asp Ser Ser Phe
            1460                1465                1470

Ile Pro Leu Gly His Met Leu Thr Gln Lys Ile Ala Tyr Gln Ile Ala
        1475                1480                1485

Ser Gly Leu Ala Tyr Leu His Lys Lys Asn Ile Ile Phe Cys Asp Leu
    1490                1495                1500

Lys Ser Asp Asn Ile Leu Val Trp Ser Leu Asp Val Lys Glu His Ile
1505                1510                1515                1520

Asn Ile Lys Leu Ser Asp Tyr Gly Ile Ser Arg Gln Ser Phe His Glu
                1525                1530                1535

Gly Ala Leu Gly Val Glu Gly Thr Pro Gly Tyr Gln Ala Pro Glu Ile
            1540                1545                1550

Arg Pro Arg Ile Val Tyr Asp Glu Lys Val Asp Met Phe Ser Tyr Gly
        1555                1560                1565

Met Val Leu Tyr Glu Leu Leu Ser Gly Gln Arg Pro Ala Leu Gly His
1570                1575                1580

His Gln Leu Gln Ile Ala Lys Lys Leu Ser Lys Gly Ile Arg Pro Val
1585                1590                1595                1600

Leu Gly Gln Pro Glu Glu Val Gln Phe Arg Arg Leu Gln Ala Leu Met
                1605                1610                1615

Met Glu Cys Trp Asp Thr Lys Pro Glu Lys Arg Pro Leu Ala Leu Ser
            1620                1625                1630

Val Val Ser Gln Met Lys Asp Pro Thr Phe Ala Thr Phe Met Tyr Glu
        1635                1640                1645

Leu Cys Cys Gly Lys Gln Thr Ala Phe Phe Ser Ser Gln Gly Gln Glu
    1650                1655                1660

Tyr Thr Val Val Phe Trp Asp Gly Lys Glu Glu Ser Arg Asn Tyr Thr
1665                1670                1675                1680

Val Val Asn Thr Glu Lys Gly Leu Met Glu Val Gln Arg Met Cys Cys
                1685                1690                1695

Pro Gly Met Lys Val Ser Cys Gln Leu Gln Val Gln Arg Ser Leu Trp
            1700                1705                1710

Thr Ala Thr Glu Asp Gln Lys Ile Tyr Ile Tyr Thr Leu Lys Gly Met
        1715                1720                1725

Cys Pro Leu Asn Thr Pro Gln Gln Ala Leu Asp Thr Pro Ala Val Val
    1730                1735                1740

Thr Cys Phe Leu Ala Val Pro Val Ile Lys Lys Asn Ser Tyr Leu Val
1745                1750                1755                1760

Leu Ala Gly Leu Ala Asp Gly Leu Val Ala Val Phe Pro Val Val Arg
                1765                1770                1775

Gly Thr Pro Lys Asp Ser Cys Ser Tyr Leu Cys Ser His Thr Ala Asn
            1780                1785                1790

Arg Ser Lys Phe Ser Ile Ala Asp Glu Asp Ala Arg Gln Asn Pro Tyr
        1795                1800                1805

Pro Val Lys Ala Met Glu Val Val Asn Ser Gly Ser Glu Val Trp Tyr
    1810                1815                1820

Ser Asn Gly Pro Gly Leu Leu Val Ile Asp Cys Ala Ser Leu Glu Ile
1825                1830                1835                1840

Cys Arg Arg Leu Glu Pro Tyr Met Ala Pro Ser Met Val Thr Ser Val
                1845                1850                1855

Val Cys Ser Ser Glu Gly Arg Gly Glu Glu Val Val Trp Cys Leu Asp
            1860                1865                1870

Asp Lys Ala Asn Ser Leu Val Met Tyr His Ser Thr Thr Tyr Gln Leu
        1875                1880                1885

Cys Ala Arg Tyr Phe Cys Gly Val Pro Ser Pro Leu Arg Asp Met Phe
    1890                1895                1900

Pro Val Arg Pro Leu Asp Thr Glu Pro Pro Ala Ala Ser His Thr Ala
1905                1910                1915                1920

Asn Pro Lys Val Pro Glu Gly Asp Ser Ile Ala Asp Val Ser Ile Met
                1925                1930                1935

Tyr Ser Glu Glu Leu Gly Thr Gln Ile Leu Ile His Gln Glu Ser Leu
            1940                1945                1950

Thr Asp Tyr Cys Ser Met Ser Ser Tyr Ser Ser Ser Pro Pro Arg Gln
        1955                1960                1965

Ala Ala Arg Ser Pro Ser Ser Leu Pro Ser Ser Pro Ala Ser Ser Ser
    1970                1975                1980

Ser Val Pro Phe Ser Thr Asp Cys Glu Asp Ser Asp Met Leu His Thr
1985                1990                1995                2000

Pro Gly Ala Ala Ser Asp Arg Ser Glu His Asp Leu Thr Pro Met Asp
                2005                2010                2015

Gly Glu Thr Phe Ser Gln His Leu Gln Ala Val Lys Ile Leu Ala Val
            2020                2025                2030

Arg Asp Leu Ile Trp Val Pro Arg Arg Gly Gly Asp Val Ile Val Ile
        2035                2040                2045

Gly Leu Glu Lys Asp Ser Gly Ala Gln Arg Gly Arg Val Ile Ala Val
    2050                2055                2060

Leu Lys Ala Arg Glu Leu Thr Pro His Gly Val Leu Val Asp Ala Ala
2065                2070                2075                2080

Val Val Ala Lys Asp Thr Val Val Cys Thr Phe Glu Asn Glu Asn Thr
                2085                2090                2095

Glu Trp Cys Leu Ala Val Trp Arg Gly Trp Gly Ala Arg Glu Phe Asp
            2100                2105                2110

Ile Phe Tyr Gln Ser Tyr Glu Glu Leu Gly Arg Leu Glu Ala Cys Thr
        2115                2120                2125

Arg Lys Arg Arg
    2130

## Beschreibung

Die Erfindung betrifft Nukleinsäuren, die für einen Membranrezeptor für Steroide und Sterole kodieren, Polypeptide und ihre Verwendung.

Nach dem klassischen Modell der Steroidhormon-Wirkung binden die Steroide an intrazelluläre Rezeptoren, die als Liganden-aktivierte Transkriptionsfaktoren die Genexpression regulieren. Es ist üblich, die direkte Wirkung der Steroidhormone über ihre kernständigen Rezeptoren auf die Transkription von Zielgenen als genomische Effekte zu bezeichnen. Genomische Effekte sind durch ihren zeitlich verzögerten Eintritt, d.h. mehr als 5 Minuten bis zu mehreren Stunden nach Hormongabe, gekennzeichnet (McEwen et al. 1978 in : Reichlin et al. Eds., The Hypothalamus, Raven Press p 255ff).

Im Gegensatz zu den genomischen Wirkungen werden seit vielen Jahren "schnelle", nichtgenomische Effekte beobachtet, die innerhalb weniger Sekunden oder Minuten nach Steroidhormongabe auftreten und ihre Ursache nicht in einer Regulation der Transkription haben können. Biochemische Indikatoren für diese Effekte sind Veränderungen des Membranpotentials und /oder die Beeinflussung von lonenkanälen in der Zellmembran (Watson et al. 1999, Proc Soc Exp Biol Med 220, 9ff). Diese Effekte könnten über membranständige Rezeptoren vermittelt werden. Häufig können Steroideffekte, die auf die Existenz eines Membranrezeptors hinweisen, auch durch Steroidderivate ausgelöst werden, die durch Kopplung an ein sperriges Trägermolekül (beispielsweise Serumalbumin) nicht mehr durch die Plasmamembran hindurchtreten können, und, fluoreszenzmikroskopisch sichtbar gemacht, an die Zelloberfläche binden. Diese Ergebnisse, ebenso wie Bindungsstudien mit Zellmembranen und Immunfärbungen, deuten auf die Existenz eines membranständigen Steroidrezeptors hin.

Ein Beispiel für eine nichtgenomische Wirkung ist die Modulation des γ-aminobutyric acid (GABA)-Rezeptors durch Progesteron. Dieser Signalweg ist für die Vermittlung der anesthetischen Wirkung von Progesteron verantworlich (Covey DF et al. 2000 J Pharmacol Exp Ther 293, 1009). Ein weiteres Beispiel für eine nichtgenomische Progesteronwirkung ist die Wiederaufnahme der Meiose in Amphibien-Oocyten. Die progesteroninduzierte Wiederaufnahme der Meiose umfaßt die Aktivierung von cAMP-abhängigen Proteinkinasen und die Aktivierung von MAPK-Signalwegen (Palmer A et al. 2000 Prog Cell Cycle Res 4, 131ff) und führt zum "germinal vesicle breakdown", GVBD (Masui Y et al. 1971, J Exp Zool 177, 129ff). Während einige Befunde dafür sprechen, daß die Effekte über einen Membranrezeptor für Progesteron (mPR) vermittelt werden könnten, sprechen neueste Untersuchungen dafür, daß der kernständige PR mit Tyrosinkinasesignalwegen interagieren könnte (Tian J et al. 2000, Proc Natl Acad Sci USA 97, 14358ff; Bayaa M et al. 2000, Proc Natl Acad Sci USA 97, 12607).
In einer großen Zahl von Publikationen sind nichtgenomische Estrogeneffekte für sehr viele Gewebe und Kulturzellen beschrieben. Innerhalb weniger Minuten löst die Gabe von Estrogen eine Reihe von Effekten aus: Intrazelluläre Ca²⁺ "spikes" (Mendoza C., Soler A., Tesarik J. 1995, Biochem Biophys Res Commun 210: 518-523), Stimulation der Adenylatcylase (Aronica SM, Kraus WL, Katzenellenbogen BS 1994, Proc Natl Acad Sci 91: 8517-21), Aktivierung von NO-Synthetase und Phospholipase C (Le Mellay V, Grosse B, Lieberherr M 1997, J Biol Chem 272: 11902-11907).

Trotz der umfangreichen Literatur zu nichtgenomischen Effekten von Steroidhormonen konnte ein membranständiger Rezeptor für Steroide in Säugetieren bisher nicht eindeutig identifiziert werden. Sollte solch ein Rezeptor existieren, würde er einen ganz neuen Weg für die Entwicklung von Medikamenten eröffnen. Daher ist die Bereitstellung eines solchen Rezeptors Aufgabe diese Erfindung.

### Gelöst wurde das Problem durch Bereitstellung einer Nukleinsäure umfassend

a. die in Seq ID NO 1, NO 3, NO 5, NO 7, NO 9, NO 11, NO 13 oder NO15 dargestellte Nukleotidsequenz,
b. eine den Sequenzen aus a. im Rahmen der Degeneration des genetischen Codes entsprechende Nukleotidsequenz oder
c. eine mit den Sequenzen aus a. oder b. unter stringenten Bedingungen hybridisierende Nukleotidsequenz

Der Begriff "Hybridisierung unter stringenten Bedingungen" gemäß der vorliegenden Erfindung wird bei Sambrook et al. (Molecular Cloning, A Laboratory Manual, Cold Spring Harbor Laboratory Press, 1989) definiert. Ein stringente Hybridisierung liegt beispielsweise vor, wenn nach dem Waschen für 1 h mit 1 x SSC und 0,1%SDS bei 50°C, vorzugsweise bei 55°C, besonders bevorzugt bei 62°C und am meisten bevorzugt bei 68°C, insbesondere für 1h in 0,2 x SSC und 0,1 % SDS bei 55°C, vorzugsweise bei 62°C und am meisten bevorzugt bei 68°C noch ein Hybridisierungssignal beobachtet wird. Die Nukleinsäuren, die unter diesen Bedingungen mit der in Seq.lD NO 1, 3, 5, 7, 9, 11, 13 und/oder 15 gezeigten Nukleinsäure oder einer dieser Sequenz im Rahmen der Degeneration des genetischen Codes entsprechende Nukleotidsequenz hybridisiert, sind auch Gegenstand der vorliegenden Erfindung.

Nukleinsäuren können einzel- oder doppelsträngige DNA, z.B. cDNA, oder RNA, z.B. mRNA, cRNA, pre-mRNA darstellen.

Die in SEQ ID NO1, 3, 5, 7, 9, 11, 13 oder 15 dargestellten Nukleinsäuren kodieren für einen Membranrezeptor für Steroide und Sterole. Die acht Nukleinsäuren sind Splicevarianten desselben Gens. Die in SEQ ID NO 3, 7, 13 und 15 dargestellten Varianten haben jeweils eine Insertion im 5' Bereich der Sequenz.
Die in Seq ID NO 5, 7, 11 und 15 dargestellten Sequenzen enthalten jeweils eine Deletion zwischen zwei Ankyrin-Domänen.
Die in Seq ID NO 9, 11, 13 und 15 dargestellten Sequenzen enthalten jeweils eine Deletion im Bereich eines Leucin-rich-repeats, eine weitere Deletion in der Island-Domäne und eine Insertion im C-terminalen Bereich.
Die Kinase-Domäne ist für alle Splice-Varianten gleich.

Bevorzugt sind die Nukleinsäuren, die einen Protein kodierenden Abschnitt der in Seq ID NO 1, 3, 5, 7, 9, 11, 13 oder 15 dargestellten Nukleinsäuresequenz umfaßen. Ein Protein kodierender Abschnitt der in Seq ID NO 1 dargestellten Sequenz liegt im Bereich von Nukleotid 227 bis 6271, ein kodierender Bereich der in SEQ ID NO 3 dargestellten Sequenz liegt im Bereich von Nukleotid 360 bis 6482, der in Seq ID NO 5 dargestellten Sequenz im Bereich von Nukleotid 227-6993, der in Seq ID NO 7 dargestellten Sequenz im Bereich von Nukleotid 360-6408, der in Seq ID NO 9 dargestellten Sequenz im Bereich von Nukleotid 227-6271, der in Seq ID NO 11 dargestellten Sequenz im Bereich von Nukleotid 227-6193, der in Seq ID NO 13 dargestellten Sequenz im Bereich von Nukleotid 360-6485 und der in Seq ID NO 15 dargestellten Sequenz im Bereich von Nukleotid 360-6407.

Gegenstand der Erfindung sind ferner Nukleinsäuren, die für ein Polypeptid mit der in Seq ID NO2, 4, 6, 8, 10, 12, 14 oder 16 dargestellten Aminosäuresequenz kodieren.

Die erfindungsgemäßen Nukleinsäuren sind aus Säugern, z.B. humanen Zellen oder aus einer cDNA-Bibliothek oder einer genomischen Bibliothek, die z.B. aus menschlichen Zellen gewonnen wird, erhältlich. Sie können nach bekannten Techniken unter Verwendung kurzer Abschnitte der in Seq ID NO 1, 3, 5, 7, 9,11, 13 oder 15 gezeigten Nukleinsäuresequenz als Hybridisierungssonden oder Amplifikationsprimer isoliert werden.

Die Erfindung betrifft weiterhin Polypeptide, die von einer erfindungsgemäßen Nukleinsäure kodiert werden. Diese Polypeptide haben die Funktion eines Steroidrezeptors.

Weiterhin sind Gegenstand der Erfindung Polypeptide, die die in Seq ID NO 2, 4, 6, 8, 10, 12, 14 oder 16 dargestellte Aminosäuresequenz umfassen.
Die erfindungsgemäßen Polypeptide können rekombinante Polypeptide, natürliche, isolierte Polypeptide oder synthetische Polypeptide sein.
Die erfindungsgemäßen Polypeptide enthalten verschiedene Domänen: mehrere (15 in Seq ID NO 10, 12, 14, 16 und 16 in Seq ID NO 2, 4, 6, 8) sogenannte "Leucin-rich-repeats" (LRR), Ankyrin-Domänen (ANK, 5 in Seq ID NO 2, 4, 10, 13 und 4 in Seq ID NO 6, 8, 12, 16) und eine Kinase-Domäne. Diese Polypeptide sind membranassoziiert. Die Kinase-Domäne phosphoryliert nach Bindung eines Steroids oder Sterols Serin- und Threonin-Reste von Proteinen und setzt damit eine intrazelluläre Signalkaskade in Gang. Die erfindungsgemäßen Polypeptide enthalten ferner eine sogenannte "Island" Domäne, die 600 Aminosäuren umfaßt und sich zwischen der jeweils vorletzten Leucin-rich-repeat Domäne und der letzten Leucin-rich-repeat Domäne des erfindungsgemäßen Polypeptids befindet. Diese Domäne ist an der Steroid- oder Sterolbindung beteiligt.

Die Erfindung betrifft auch Polypeptide, die die Kinase-Domäne der erfindungsgemäßen Polypeptide oder Teile davon umfassen. Die Kinase-Domäne befindet sich etwa im Bereich von Aminosäure 1270 bis 1565, wobei sich die Numerierung auf die in Seq ID No2 dargestellte Sequenz bezieht.

Gegenstand der Erfindung sind auch Polypeptide, die die Island Domäne der erfindungsgemäßen Polypeptide umfassen. Diese enthält eine Steroid- bzw. Sterolbindungsstelle.

Die erfindungsgemäßen Polypeptide nach Seq ID NO 2, 4 6, 8, 10, 12, 14 und 16 werden bevorzugt in Geweben exprimiert, von denen bekannt ist, dass sie Steroidhormon-sensitiv sind. Beispiele sind Ovarien, Eileiter, Prostata und Blut.

Die erfindungsgemäßen Polypeptide oder Teilbereiche davon (Peptide) können zur Herstellung von Antikörpern verwendet werden. Zur Herstellung polyklonaler Antikörper können die Polyeptide oder Peptide z.B. an KLH (Keyhole Limpet Hemocyanin) gebunden werden und Tieren, z.B. Kaninchen, gespritzt werden. Sie können auch zur Herstellung monoklonaler Antikörper verwendet werden. Zur Antikörperherstellung kann ein erfindungsgemäßes Polypeptid oder Peptid oder eine Mischung mehrerer erfindungsgemäßer Peptide verwendet werden. Die Herstellung der Antikörper erfolgt dabei nach Standardverfahren wie sie z.B. in Kohler, G. und Milstein, C., Nature 1975, 256, 495-497 und Nelson, P. N. et al., Mol. Pathol. 2000, 53, 111-117 beschrieben sind.

Gegenstand der Erfindung sind auch die Antikörper, die gegen ein erfindungsgemäßes Polypeptid gerichtet sind.

Die erfindungsgemäßen Antikörper können zur Detektion der erfindungsgemäßen Polypeptide verwendet werden. Dies kann z.B. durch Immunhistochemie erfolgen. Die erfindungsgemäßen Antikörper können auch in anderen Immuntests wie z.B. einem ELISA (enzyme linked immunosorbent assay) oder in Radioimmunotests eingesetzt werden. So kann die Konzentration an erfindungsgemäßen Polypeptid in Gewebe-oder Zellextrakten nachgewiesen werden.

Der Nachweis der Expression des erfindungsgemäßen Polypeptids kann auch über den Nachweis von mRNA in den Zellen erfolgen. Gegenstand der Erfindung ist daher auch die Verwendung einer Sonde mit Nukleinsäuresequenzen, die komplementär zu den Nukleinsäuresequenzen sind, die für die erfindungsgemäßen Peptide kodieren, zur Herstellung eines Reagenz zum Nachweis der Gegenwart von erfindungsgemäßer mRNA in Zellen. Eine Sonde ist ein kurzes DNA-Stück mit mindestens 14 Nukleotiden. Die erfindungsgemäßen Sonden können z.B. in einer Northern Blot Analyse verwendet werden. Diese Methode ist z.B. in Sambrook, J. et al., 1989, Cold Spring Harbor Laboratory Press, beschrieben. Andere Methoden zum Nachweis der RNA sind In Situ Hybridisierung, RNAse Protection Assay oder PCR.

Weiterhin sind Gegenstand der Erfindung Vektoren die mindestens eine Kopie einer erfindungsgemäßen Nukleinsäure enthalten. Vektoren können prokaryontische oder eukaryontische Vektoren sein. Beispiele für Vektoren sind pPRO (Clontech), pBAD (Invitrogen), pSG5 (Stratagene), pCl (Promega), pIRES (Clontech), pBAC (Clontech), pMET (Invitrogen), pBlueBac (Invitrogen). In diese Vektoren kann mit den dem Fachmann bekannten Methoden die erfindungsgemäßen Nukleinsäuren eingefügt werden. Die erfindungsgemäßen Nukleinsäuren befinden sich vorzugsweise in Verbindung mit Expressionssignalen wie z.B. Promotor und Enhancer auf dem Vektor.

Die Erfindung betrifft ferner Zellen, die mit einer erfindungsgemäßen Nukleinsäuresequenz oder mit einem erfindungsgemäßen Vektor transfiziert sind. Als Zellen können z.B. *E. coli,* Hefe, *Pichia*, Sf9, COS, CV-1 oder BHK verwendet werden. Diese Zellen können für die Produktion der erfindungsgemäßen Polypeptide oder für Testsysteme verwendet werden.

Ein Gegenstand der Erfindung ist die Verwendung der erfindungsgemäßen Polypeptide oder der dafür kodierenden Nukleinsäuren als Zielsubstanz zur Herstellung eines Mittels zur Behandlung von Sterol- oder Steroidhormon-abhängigen Erkrankungen.

### Insbesondere erfasst die Erfindung die Verwendung

a. einer erfindunggemäßen Nukleinsäure,
b. eines erfindungsgemäßen Polypeptids, oder
c. einer erfindungsgemäßen Zelle
zur Identifizierung von Effektoren eines erfindungsgemäßen Polypeptids. Effektoren sind Substanzen, die auf das erfindungsgemäße Polypeptid inhibitorisch oder aktivierend wirken, und die in der Lage sind, die Steroidrezeptor-Funktion der erfindungsgemäßen Polypeptide zu beeinflußen.

Die Erfindung betrifft weiterhin ein Testsystem zur Identifizierung von Effektoren eines erfindungsgemäßen Polypeptids, wobei ein erfindungsgemäßes Polypeptid als ganzes oder Teilsequenzen davon mit einem Steroid, einem Steroidanalogon oder einem Sterol inkubiert wird und die Menge an gebundenem Steroid, Steroidanalogon oder Sterol detektiert wird. Als Teilsequenz kann z.B. der Bereich, der die "Leucin-rich-repeats" und die Island-Domäne umfaßt, oder kürzere Stücke davon verwendet werden. Die Bindung der Effektoren kann z.B. gemessen werden, indem markierte Substanzen verwendet werden und die Markierung (z.B. Radioaktivität oder Fluoreszenz) gemessen wird. Es kann aber auch eine Verdrängungsreaktion gemessen werden, indem ein bindendes markiertes Steroid verwendet wird und nach Zugabe der zu testenden Substanzen die Verdrängung der Markierung vom Rezeptor gemessen wird.

Dieser Bindungstest kann auch mit einer erfindungsgemäßen Zelle durchgeführt werden, die das erfindungsgemäße Polypeptid enthält. Neben der Bindung der zu testenden Substanzen kann auch ein intrazellulärer Effekt gemessen werden wie z.B. eine Änderung der Ca²⁺ Konzentration. Diese Variante des Testsystems gibt dadurch auch Hinweise auf eine funktionelle Aktivität des Effektors.

Gegenstand der Erfindung ist ferner ein Testsystem zur Identifizierung von Inhibitoren der Kinasefunktion des erfindungsgemäßen Polypeptids wobei ein erfindungsgemäßes Polypeptid oder seine Kinasedomäne mit den zu testenden Substanzen in Kontakt gebracht wird und die Kinaseaktivität bestimmt wird. Dabei wird die Kinaseaktivität mit und ohne zu testende Substanz bestimmt und das Ausmaß der Hemmung bestimmt.

Die Effektoren der Sterol- oder Steroidrezeptorfunktion des erfindungsgemäßen Polypeptids können zur Behandlung von Sterol- oder Steroidhormon-abhängigen Krankheiten eingesetzt werden. Beispiele hierfür sind peri- und postmenopausale Beschwerden, Erkrankungen des Urogenitaltrakts, ovarielle Dysfunktion, Osteoporose, benigne Prostatahyperplasie, Herzkreislauferkrankungen, Gefäßerkrankungen, neurodegenerative Erkrankungen, enzündliche Erkrankungen und Erkrankungen des Immunsystems. Außerdem können diese Effektoren zur Behandlung von weiblicher und männlicher Infertilität eingesetzt werden.

Es wurde gefunden, dass das erfindungsgemäße Polypeptid die körpereigene Substanz FF-MAS (Meiose-aktivierende Substanz aus follikulärer Flüssigkeit) bindet. FF-MAS induziert die Meiose der weiblichen Keimzellen und ist daher ein wesentlicher Faktor der weiblichen Fertilität. Mit Hilfe eines erfindungsgemäßen Testsystems können Substanzen identifiziert werden, die den Effekt von FF-MAS auf den Rezeptor hemmen. Diese Substanzen hemmen die Fertilität und können daher als Mittel für die Fertilitätskontrolle eingesetzt werden. Es können mit dem erfindungsgemäßen Testsystem auch Substanzen identifiziert werden, die den Rezeptor aktivieren und daher analog wie FF-MAS wirken. Diese Substanzen wirken fertilitätsfördernd und können entweder Frauen, die unter Fertilitätsstörungen leiden, appliziert werden oder sie können *in vitro* zu einer Oocytenkultur gegeben werden. Die so präparierten Oocyten werden dann für die *in vitro* Fertilisation verwendet.

Weiterhin betrifft die Erfindung ein Verfahren zur Bereitstellung eines pharmazeutischen Mittels, wobei
a. Substanzen mit einem erfindungsgemäßenTestsystem in Kontakt gebracht werden,
b. die Wirkung der Substanzen auf das Testsystem im Vergleich zu Kontrollen gemessen wird,
c. eine Substanz, die in Schritt b. eine Modulation der Aktivität der erfindungsgemäßen Polypeptide zeigt, identifiziert wird
d. und die in Schritt c. identifizierte Substanz mit in der Pharmazie üblichen Formulierungsstoffen gemischt wird.
Unter Aktivität des erfindungsgemäßen Polypeptids ist die Steroid-bindende Eigenschaft und/oder die Kinaseaktivität des Polypeptids zu verstehen.
Eine Substanz, die durch ein erfindungsgemäßes Verfahren identifiziert wird, kann gegebenenfalls bezüglich metabolischer Stabilität, Aktivität in einem erfindungsgemäßen Testsystem und/oder Bioverfügbarkeit optimiert werden. Dafür können in der Chemie gängige Methoden verwendet werden.

### Beschreibung der Abbildungen

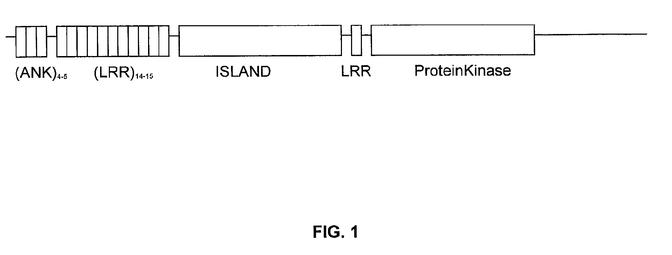

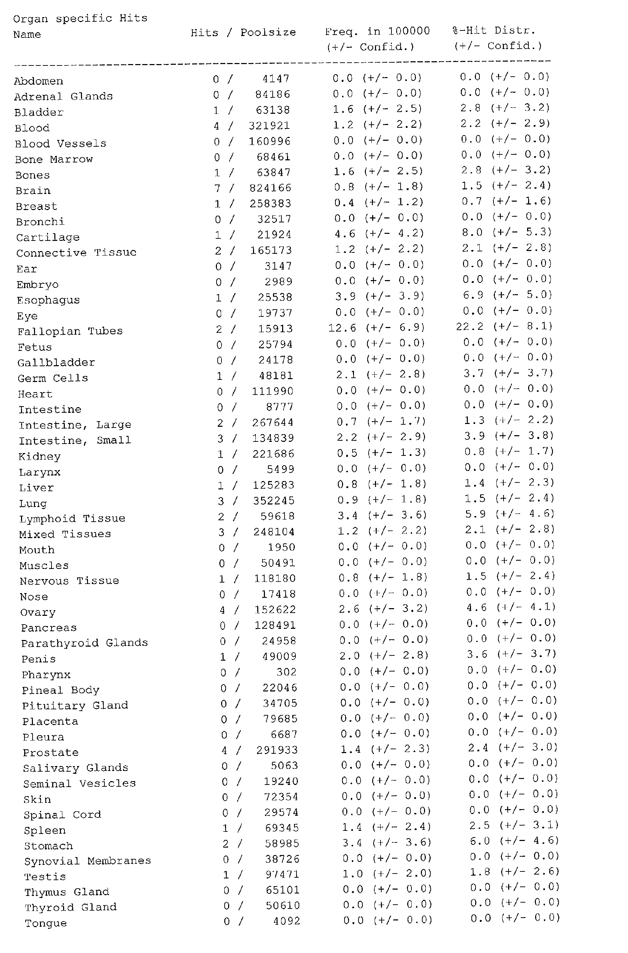

FIG.1 zeigt die Domänenstruktur der erfindungsgemäßen Polypeptide. Die Struktur beginnt links mit dem N-Terminus.

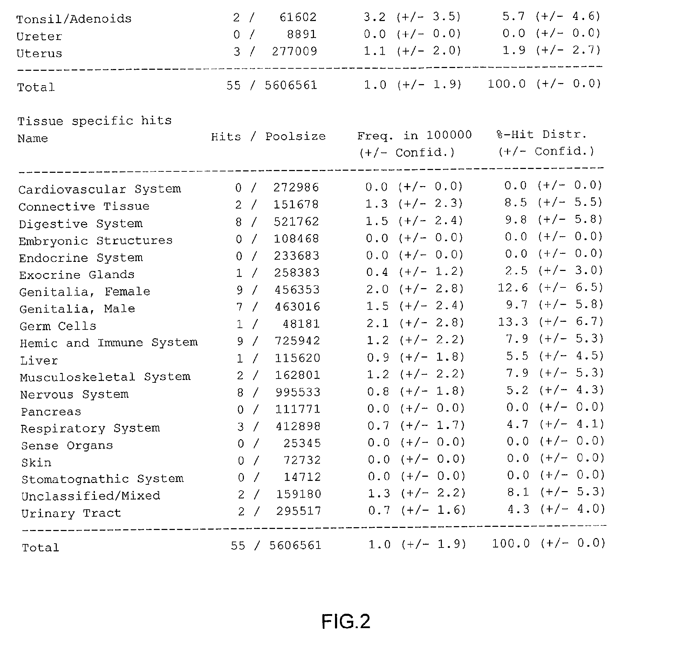

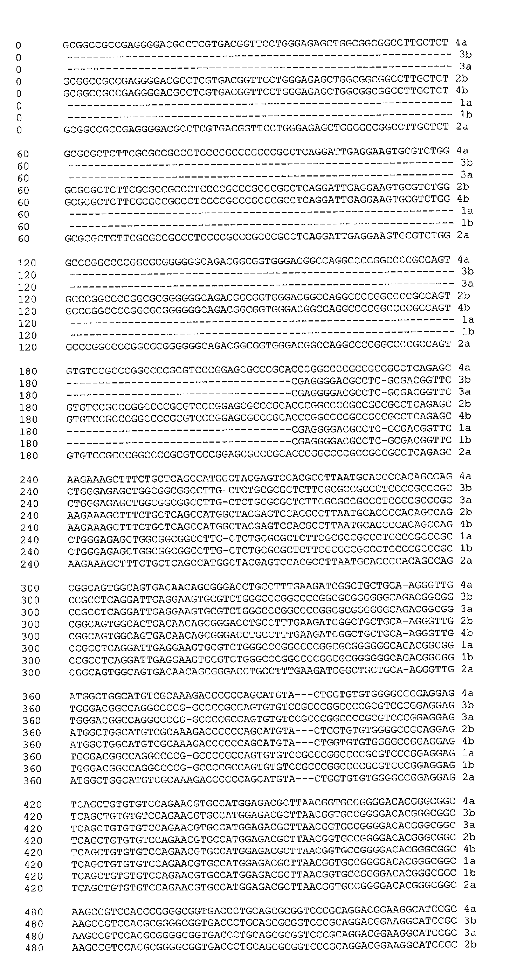

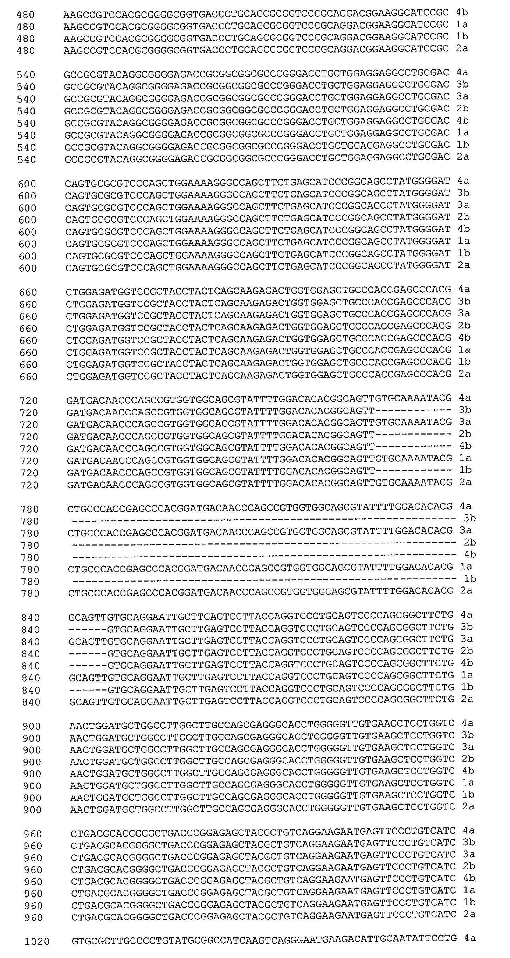

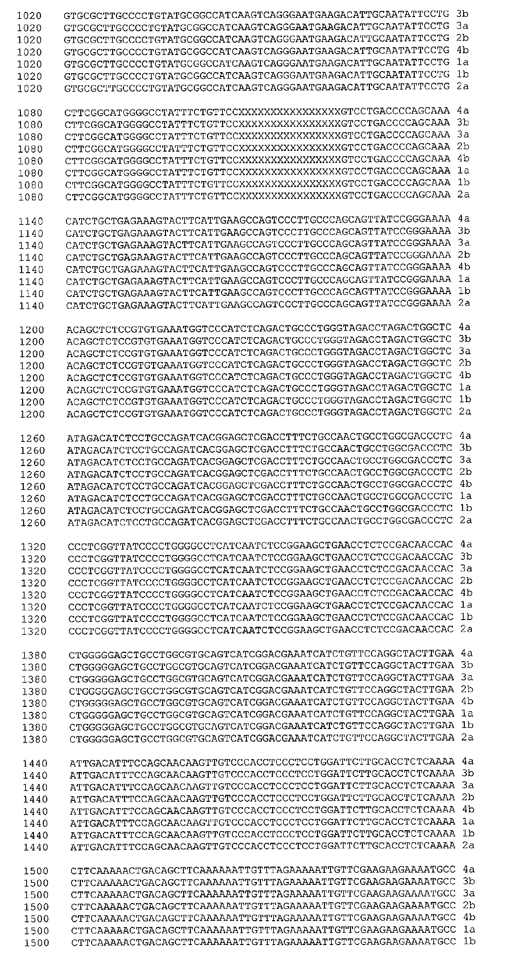

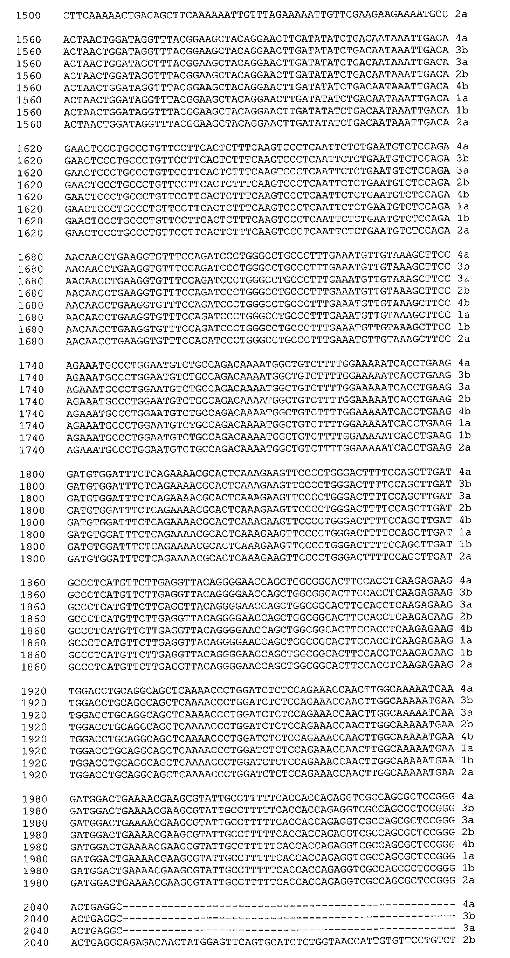

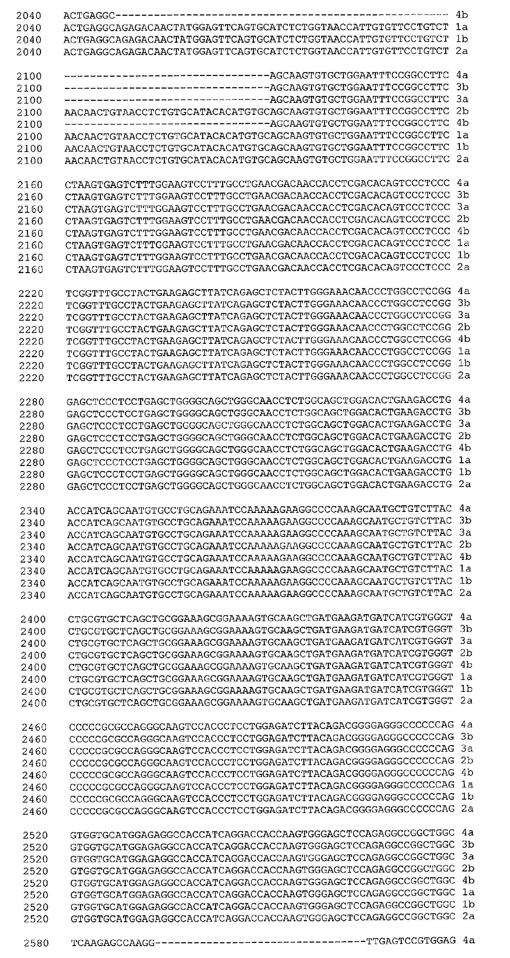

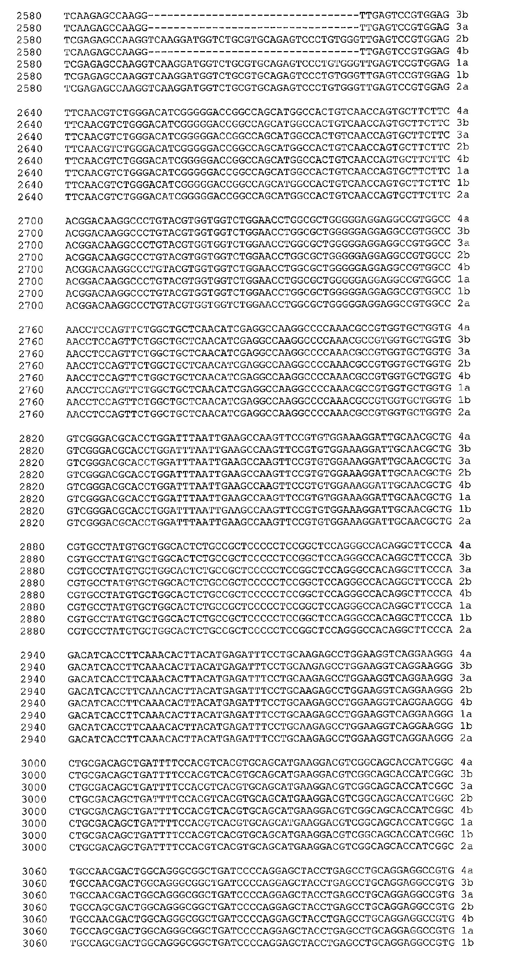

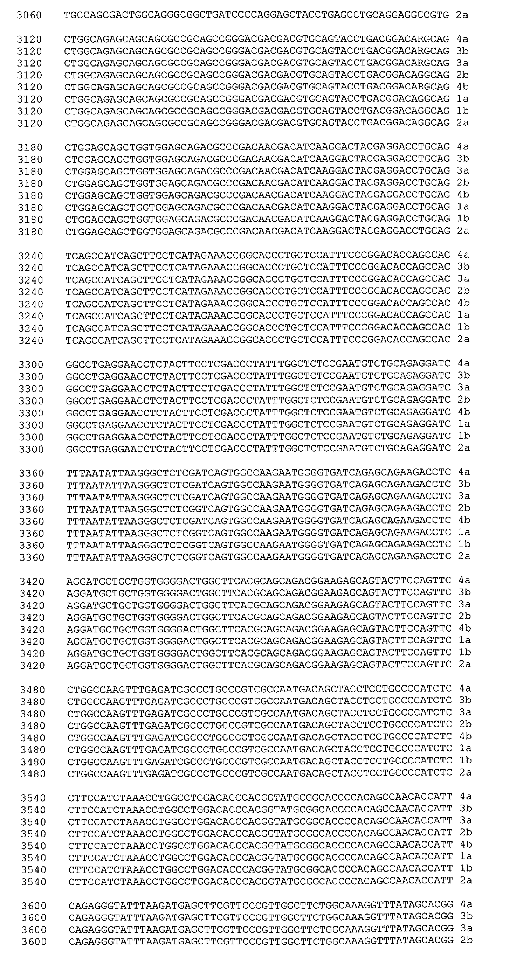

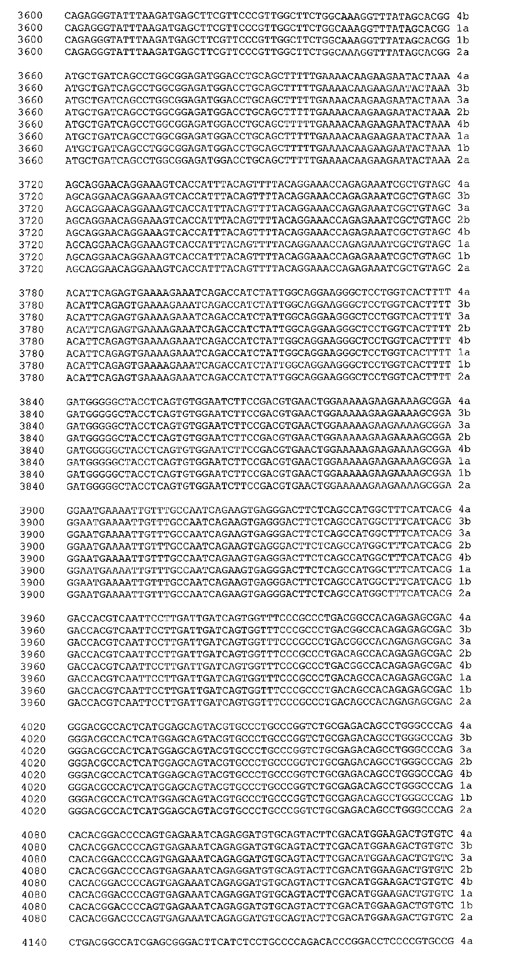

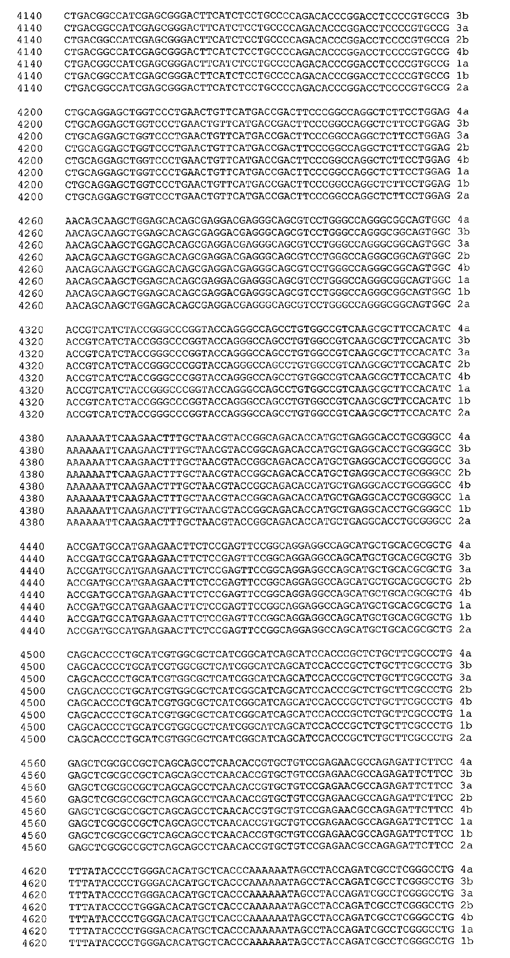

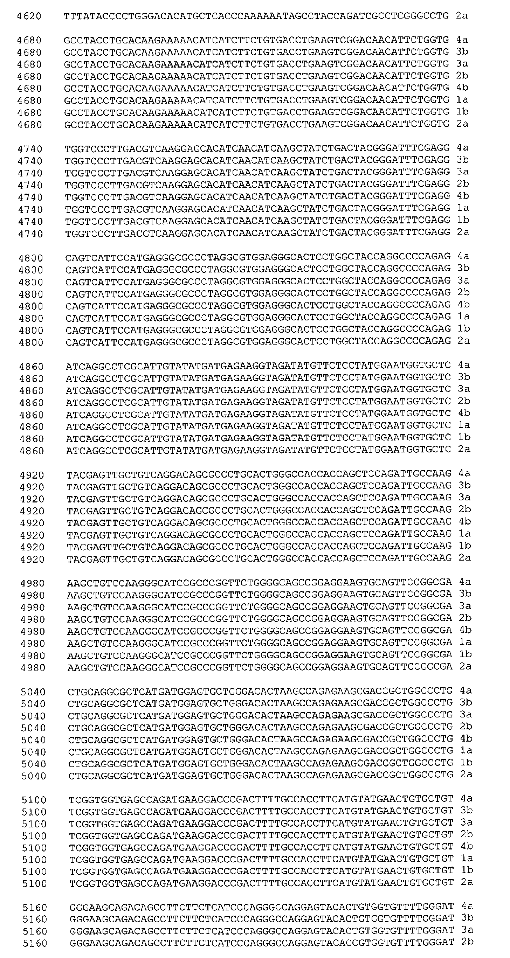

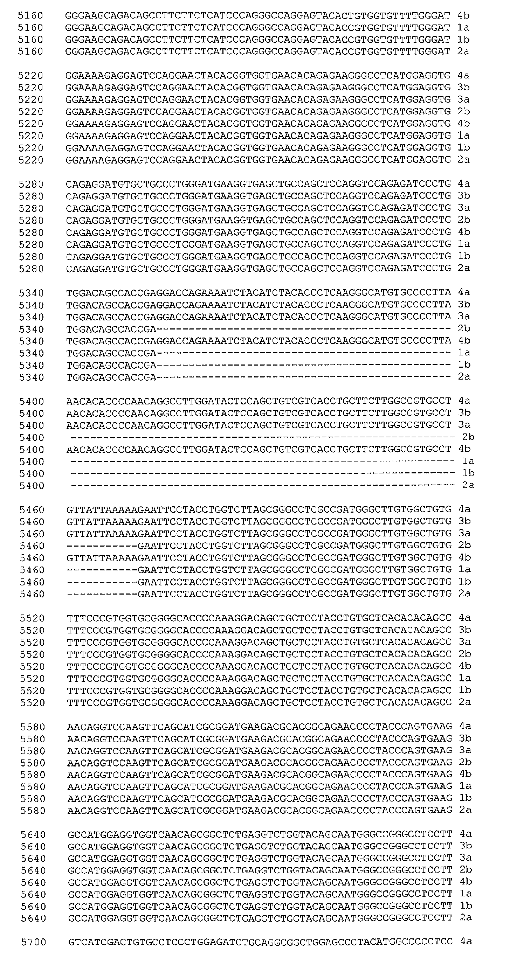

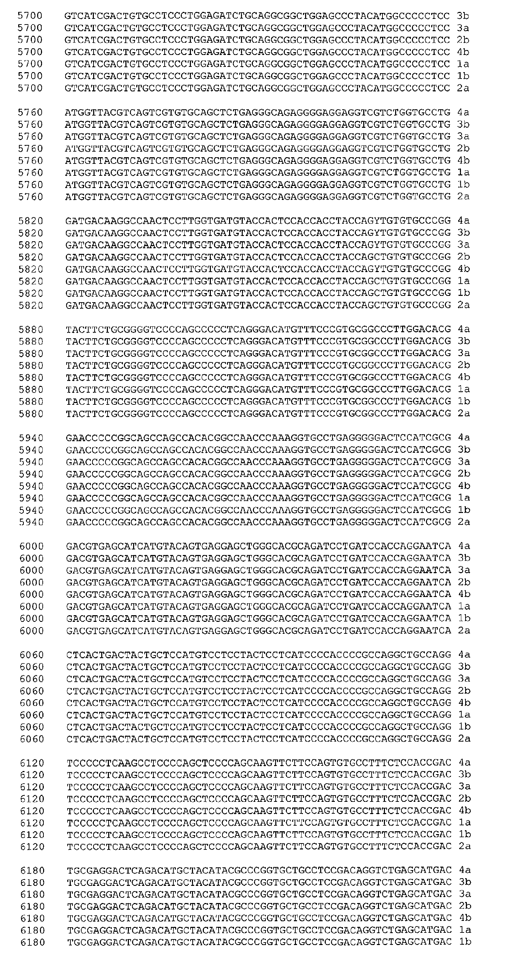

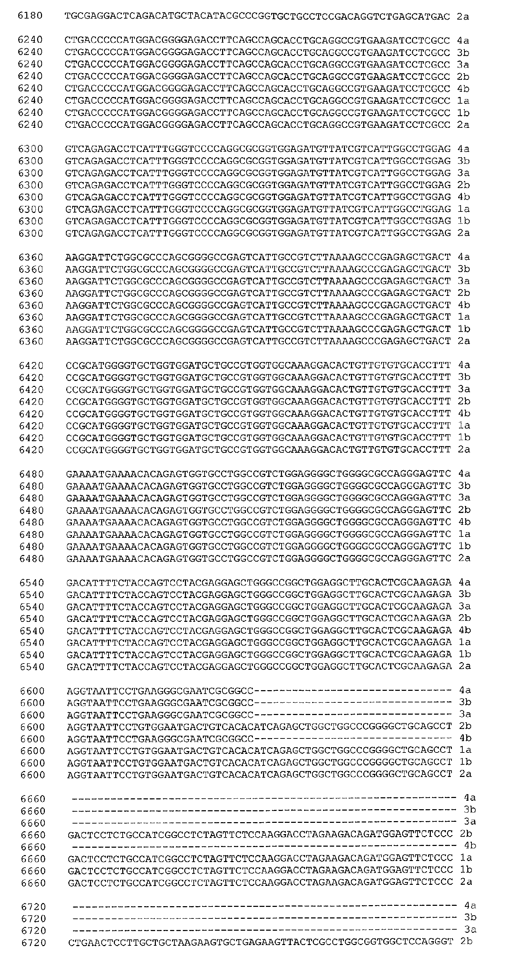

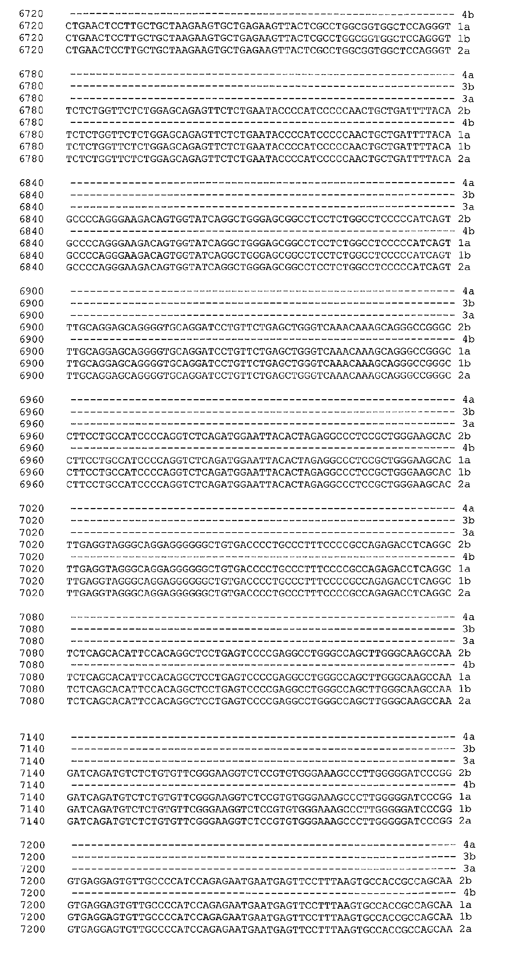

FIG. 2 zeigt das Ergebnis einer elektronischen Northern blot Analyse.

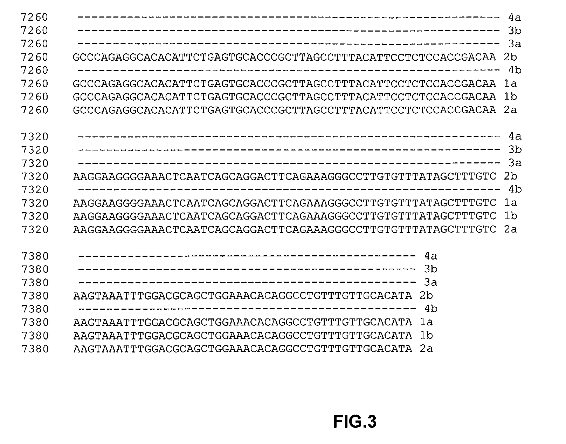

FIG. 3 zeigt einen Sequenzvergleich der DNA von allen 8 Splicevarianten. Dabei entspricht die Bezeichnung
- 1a: Seq ID No 1,
- 2a: Seq ID No 3,
- 1b: Seq ID No 5
- 2b: Seq ID No 7
- 3a: Seq ID No 9
- 3b: Seq ID No 11
- 4a: Seq ID No 13
- 4b: Seq ID No 15

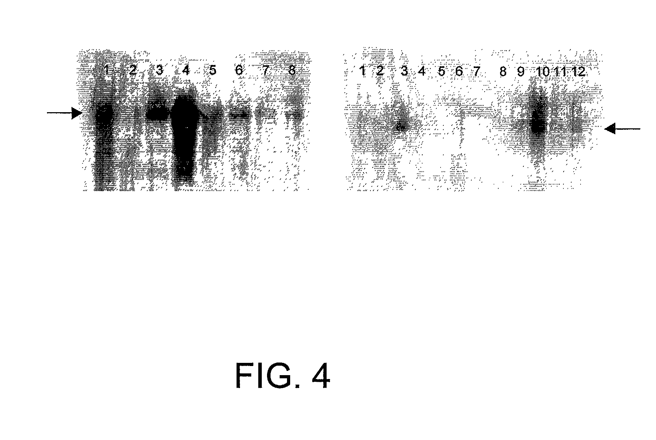

FIG.4 zeigt die Verteilung der erfindungsgemäßen RNA in verschiedenen humanen Geweben. Für die Amplifikation wurden die folgenden Primer verwendet: 5' gtatgaactgtgctgtgggaag 3' (4928) und 5' gatgtaccactccaccacctacc 3'(5532). Gezeigt ist ein Northern blot.
Im linken Bild bedeuten 1: Milz, 2: Thymus, 3: Prostata, 4: Testis, 5: Ovar, 6: Dünndarm, 7: Colon, 8: periphere Blut Leukozyten. Im rechten Bild bedeuten 1: Gehirn, 2: Herz, 3: Skelettmuskel, 4: Colon, 5: Thymus, 6: Milz, 7: Niere, 8: Leber, 9: Dünndarm, 10: Plazenta, 11: Lunge, 12: periphere Blut Leukozyten. Der Pfeil zeigt die Lage der Bande an, die der erfindungsgemäßen RNA entspricht.

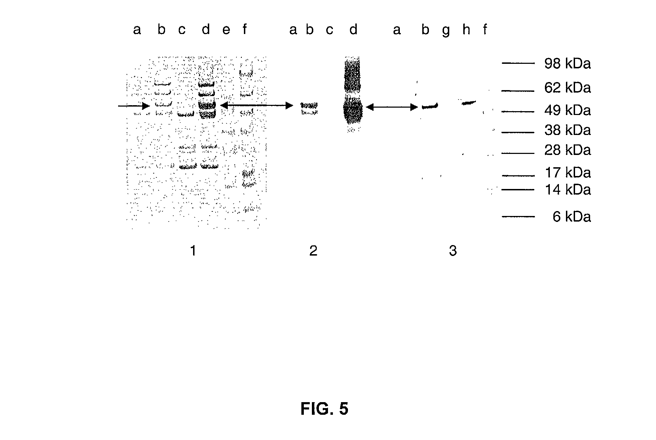

FIG.5 zeigt die Expression und Autophosphorylierung der Kinase-Domäne. In Bild 1 ist ein Polyacrylamidgel nach Proteinfärbung mit Coomassie G-250, in Bild 2 das entsprechende Autoradiogramm (³³P) und in Bild 3 ein Western blot dargestellt. Es bedeuten: a = Glutathion Eluat (Kontrolle), b = Glutathion Eluat (GST-SIN2), c = Glutathionsepharose mit gebundenen Proteinen (Kontrolle), d = Glutathionsepharose mit gebundenen Proteinen (GST-SIN2), e + f = Molekulargewichtsmarker (SeaBlue), g = SDS-Eluat der Glutathionsepharose (Kontrolle), h = SDS-Eluat der Glutathionsepharose (GST-SIN2). Die Versuchsbedingungen sind in Beispiel 3 und 4 beschrieben. Der Western blot wurde mit Anti-GST-Antikörpern (Ziegenserum) als erstem Antikörper und Anti-Ziege IgG-alkalische Phosphatase Konjugat als zweitem Antikörper hergestellt. Der Pfeil markiert die Position des GST-Kinase-Fusionsproteins.

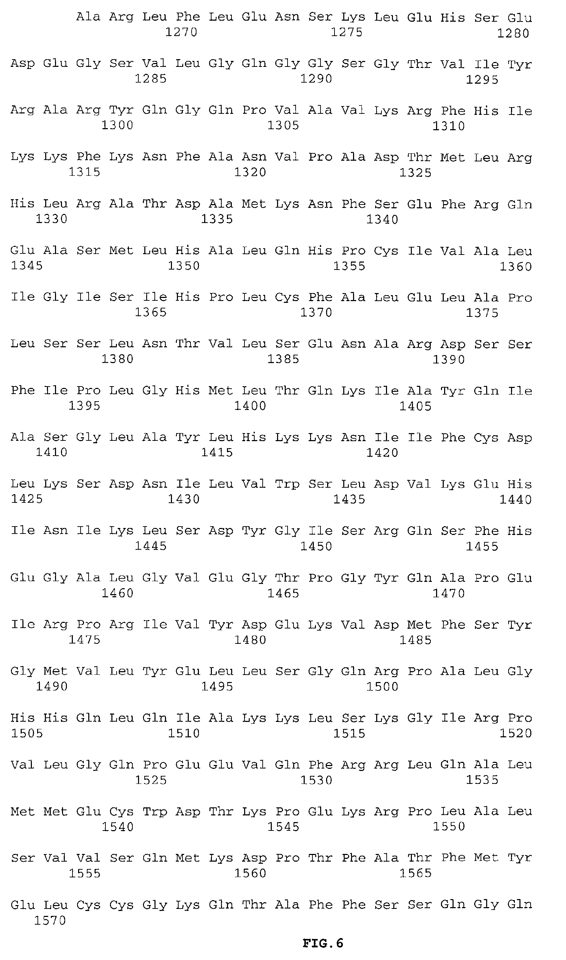

FIG. 6 zeigt die Aminosäuresequenz des Fusionsproteine aus Glutathion-S-Transferase und den Aminosäuren Ala1267-Leu1570.

### Beispiele

Die in den Beispielen verwendeten molekularbiologischen Methoden wie z.B. Polymerase-Kettenreaktion (PCR), Herstellung von cDNA, Klonierung von DNA, Sequenzierung von DNA, wurden wie in bekannten Lehrbüchern wie zum Beispiel in Molecular Cloning, A Laboratory Manual (Sambrook, J. et al., 1989, Cold Spring Harbor Laboratory Press) beschrieben, durchgeführt.

### Beispiel 1: Klonierung des membrangebundenen Steroidrezeptors

Für die Klonierung wurde eine cDNA Bank aus humaner Milchdrüse (Clontech) verwendet. Die cDNA der erfindungsgemäßen Splicevarianten wurde mittels PCR gewonnen. Dazu wurden folgende Primer verwendet:
- N-terminaler Bereich: 5'- ATGGAGACGCTTAACGGTGC -3'
- #3F: 5'- GTTCCCTGTCATCGTGCGCTTGCCCC -3' (790-815)
- #4F: 5'- CCAGCAACAAGTTGTCCCACCTCCC -3' (1233-1257)
- #5F: 5'- GGATCTCTCCAGAAACCAACTTGGC -3' (1729-1753)
- #6F: 5'- CCTGGAGATCTTACAGACGGGGAGGG -3' (2269-2294)
- #7F: 5'- CGTCGGCAGCACCATCGGCTGCCAG -3' (2824-2848)
- #8F: 5'- CCTCCTGCCCCATCTCCTTCCATC -3' (3307-3330)
- #9F: 5'- GAAATCAGAGGATGTGCAGTACTTC -3' (3880-3904)
- #10F: 5'- CACCGTGCTGTCCGAGAACGCCAG -3' (4369-4392)
- #11F: 5'- CCTTCATGTATGAACTGTGCTGTG -3' (4920-4943)
- #12F: 5'- CCATGGTTACGTCAGTCGTGTGC -3' (5424-5446)
- #13F: 5'- CCGACAGGTCTGAGCATGACCTG -3' (5886-5908)
- #2R: 5'- TTACCTTCTCTTGCGAGTGCAAGCC -3' (6247-6271)
- C-terminaler Primer: 5' GAGAACTCTGCTCCAGAGAAC 3'
Für die Amplifikation der Splicevarianten, die in Seq Id No 1 und 3 dargestellt sind, wurde auch der Primer 5'- CGGATGACAACCCAGCCGTGGTGG -3' (579-602) verwendet. Die in Klammern angegebene Numerierung bezieht sich auf die in Seq ID No 1 dargestellte Sequenz.

### Beispiel 2: Expression des membrangebundenen Steroidrezeptors

Für die Expression wurde der kodierende Bereich mittels PCR amplifiziert und in den Baculovirusexpressionsvektor pBlueBac4.5/V5-His-TOPO (Invitrogen) bzw. den eukaryontischen Expressionsvektor pcDNA3.1/V5/His-TOPO (Invitrogen) eingefügt. Um Nachweis und Reinigung zu vereinfachen, erfolgte eine Fusion mit einem His-Tag. Nach Cotransfektion von Insektenzellen mit der Bac-N-Blue DNA wurden rekombinante Viren erzeugt, die durch eine PCR-Verfahren identifiziert wurden. Ein Phagenstock wurde dann angelegt und für weitere Transfektionen und Produktion des Rezeptors in größeren Mengen verwendet. Die Reinigung der His-getaggten Proteinen erfolgte über eine Nickel-Affinitätssäule.

### Beispiel 3: Klonierung und Expression der Kinase-Domäne

Die Kinasedomäne wurde innerhalb einer größeren Sequenz aus einer cDNA Bibliothek (Mamm.Gland und Lung, Clontech) per PCR amplifiziert und in den TOPO Vektor kloniert. Aus diesem Vektor wird nur die Kinasedomäne herausamplifiziert.

Zur Expression der rekombinanten Kinasedomäne als Glutathion-S-Transferase-Fusionsprotein wird der Vektor pGex-KT (enthält Thrombin- Schnittstelle zwischen den Proteinen), zur Fusion mit MBP wird der Vektor pMal-TBVp3 (modifiziert, ebenfalls mit Thrombin- Schnittstelle) verwendet. Im Rahmen der Klonierung werden die Plasmide vorerst in den E.coli Stamm XL-1 und anschließend zur Expression der Proteine in den E.coli Stamm BL21 transformiert. Die Transformation erfolgt immer per Hitzeschock. Die Zellen werden in LB Medium mit 200µg/ml kultiviert, wobei bei der Expression von MBP-Fusionsproteinen immer 0.1% Glucose im Medium vorhanden sein muß.
Bei der Expression der Proteine wachsen die Zellen bis zu einer OD₆₀₀ von 0.8-0.9, dann erfolgt die Induktion mit 0.5 mM IPTG für 3 h bei 25°C. Der Zellaufschluß erfolgt in der French Press. Der Aufschlußpuffer enthält 20 mM Tris pH 7.4, 150 mM NaCl, 1 mM DTT, 0.1 mM EDTA und Protease Inhibitor. Um die unlöslichen Bestandteile abzutrennen wird nach dem Zellaufschluß eine Ultrazentrifugation bei 35000xg durchgeführt. Die MBP-Fusionsproteine werden anschließend über Amylose Resin im Puffer (20 mM Tris pH 7.4, 150 mM NaCI, 1 mM DTT, 0.1 mM EDTA und Protease Inhibitor) gereinigt. Die GST-Fusionsproteine werden über Sepharose Beads aufgereinigt (Puffer wie oben).

In einem weiteren Ansatz zur Expression der rekombinanten Kinasedomäne als Glutathion-S-Transferase-Fusionsprotein wird der eukaryonte Expressionsvektor pDEST27 (Invitrogen 11812-013) verwendet.
Dieser Vektor trägt die erfindungsgemäße Kinasedomäne kodierende cDNA im Leserahmen der N-terminal vorgeschalteten Glutathion-S-Transferase cDNA. Dies führt zur Expression eines Fusionsproteins aus Glutathion-S-Transferase und der Aminosäuren Ala1267-Leu1570 (Aminosäurenummerierung gemäß Seq ID No 2).

Zur Expression der rekombinanten Kinasedomäne werden HEK293EBNA Zellen (Invitrogen) mit dem Kinasedomäne-kodierenden pDEST27 Vektor transient transfiziert. Hierzu werden HEK293EBNA Zellen in DMEM Medium (plus 10% FCS, plus Glutamax) bis zur Subkonfluenz kultiviert. Die Zellen in einer Flasche werden gemäß Standardprotokoll des Herstellers mittels Lipofectamine Reagens (Invitrogen) transfiziert. Pro 150cm² Flasche werden 150µl Reagens und 75µg pDEST27- Kinasedomäne DNA zur Transfektion eingesetzt (= GST-SIN2). Die Zellen einer zweiten Flasche werden nicht transfiziert (= Kontrolle).

Die Zellen werden nach 48h Inkubation geeerntet: Das konditionierte Medium wird verworfen. Die Zellen werden auf Eis lysiert (25mM HEPES/NaOH pH 7,3 + 1% NP-40 + 2mM EGTA + 2mM EDTA + 10mM β-Glycerophosphat + 2mM DTT + Phosphatase Inhibitor Coktail Nr. 1 (SIGMA) + Phosphatase Inhibitor Coktail Nr. 2 (SIGMA) + Completeₘᵢₙᵢ -EDTA (Roche) + 200mM NaCl), eingefroren, aufgetaut und zentrifugiert. Der Zellüberstand wird weiter verwendet.

Zur Bindung des GST-Fusionsproteins an Glutathionsepharose wird der Zellüberstand zu Gluthathionsepharose gegeben, inkubiert und gewaschen. 20µl der Glutathionsepharose mit den daran gebundenen Proteinen wird direkt wie in Beispiel 4 beschrieben in den Kinaseassay eingesetzt (= Glutathionesepharose mit gebundenen Proteinen). Die restliche Glutathionsepharose wird mit einem Puffer, der reduziertes Glutathion enthält, eluiert (=Glutathion Eluat).

### Beispiel 4: Nachweis der Aktivität der Kinase-Domäne (Autophosphorylierung)

Es werden je 10 µl der gereinigten Proteinlösung (s. Beispiel 3, Glutathionesepharose mit gebundenen Proteinen und Glutathion Eluat) mit 10 µl Kinase Assaypuffer (25mM HEPES/NaOH ph 7,3 + 10mM MgCl₂+ 10mM MnCl₂+ 2mM DTT + Phosphatase Inhibitor Coktail Nr. 1 und 2 (SIGMA) + 200mM NaCI) versetzt. Die Phosphorylierungsreaktion wird gestartet durch Zugabe von je 1µl 100µM ATP + 4µCi γ[³³P]-ATP. Die Reaktion erfolgt für 60 min bei 30°C. Die Proben werden in einem SDS-PAGE aufgetrennt und das Gel mit einer Coomassie G-250 Lösung gefärbt. Nach Trocknung des Gels erfolgt eine Autoradiographie.

### Beispiel 5: Testsystem zum Auffinden von Effektoren (Bindungsassay)

### Homogenisierung

Zellen und Gewebe, in denen das erfindungsgemäße Protein exprimiert wird, wurden in Puffer 1 bei 4°C homogenisiert. Puffer 1: 0.02 mmol/l Tris-buffer, pH 7.5, 0.5mmol/l EDTA, 2 mmol/l DTT, 20% Glycerol, 0.4 M KCL, 20 mmol/l Molybdate, 0.3 µmol/l Aprotinin, 1 µmol/l Pepstatin, 10 µmol/l Leupeptin.

### Rezeptorbindungsstudien

Rezeptorbindungsstudien wurden mit Rohextrakten in Puffer 1, Cytosol -Preparationen (Überstand nach Zentrifugation des Rohextrakts bei 105.000 x g, 90 Min) und Mikrosomenfraktionen von Zellen und Geweben, die das erfindungsgemäße Protein exprimieren, durchgeführt. Rohextrakte und Cytosolfraktionen (0.04 ml) wurden bei 4°C für 2 Stunden mit 10 µl radioaktivem Steroid oder Sterol (Konzentration im Ansatz 10 nmol/L), entweder in Abwesenheit oder in Gegenwart von einem 100-fachen Überschuß nichtradioaktiver Substanz (zur Bestimmung der unspezifischen Bindung) inkubiert. Nach der Inkubation von Rohextrakt oder der Cytosolfraktion mit den radioaktiven Steroiden oder Sterolen wurden ungebundene Steroide oder Sterole an Aktivkohle adsorbiert, indem mit 0.25 ml einer Suspension von Dextran-Coated-Charcoal in Puffer 1 5 min bei 4°C inkubiert wurde. Nach Zentrifugation (5 min, 15000 g) wurde ein Aliquot des Überstandes abgenommen und die Radioaktivität wurde mittels Scintillationszähler bestimmt. Die spezifische Rezeptorbindung wurde als Differenz zwischen der gebundenen Radioaktivität in Abwesenheit und in Anwesenheit eines 100-fachen Überschusses nichtmarkierter Verbindung bestimmt. Als radioaktiv markierte Steroide und Sterole wurden ³[H]-Estradiol, ³[H]-Progesteron, ³[H]-Corticosteron, ³[H]-Cortisol, ³[H]-Aldosteron, ³[H]-Dihydrotestosteron, ³[H]-Testosteron, ³[H]-Pregnenolon, ³[H]-Cholesterol, ³[H]-Androsteron, ³[H]-Dihydroandrosteron, ³[H]-Caicitriol und ³[H]-FF-MAS verwendet.

### Beispiel 6: Kinase Assay

Das die Kinasedomäne enthaltende Peptid wird in SF9 bzw. COS Zellen exprimiert und über Affinitätschromatografie (tag) gereinigt. Das gereinigte Peptid wird in 20 mM Mops pH7,5, 25 mM β-glycerophosphat; 5 mM EGTA ; 0,04% NP40 und 0,2% BSA im Kinase Assay 30 min mit ³³P-ATP in MgCl₂ und dem Substrat Bio-Lys-Lys-Leu-Asn-Arg-Thr-Leu-Ser-Val-Ala-OH inkubiert. Dieses Substrat wird in Gegenwart von ATP an Serin bzw. Threonin phosphoryliert und kann mit Hilfe von Streptavidin-Beads nachgewiesen werden. Die Reaktion wird mit 50 µM ATP, 50 µM EDTA, 0,1% Triton X-100 (v/v) beendet und gleichzeitig mit den Streptavidin-Beads versetzt. Die Reaktionsansätze müssen mindestens 10 min stehen bleiben und anschließend 10 min abzentrifugiert werden. Die Bestimmung der Phosphorylierung erfolgt durch Messung der Radioaktivität. Als Referenzsubstanz zur Inhibierung der Kinaseaktivität der Peptide wird Staurosporin eingesetzt.

### Beispiel 7: Elektronischer Northern

Ein elektronischer Northern gibt basierend auf der Anzahl von ESTs für ein bestimmtes Gewebe einen Hinweis auf die Expressionstärke eines bestimmten Gens in diesem Gewebe. Zunächst wird die Anzahl aller ESTs für ein Gewebe in einer Datenbank identifiziert (genannt pool-size) und dann die Anzahl der ESTs von diesem Gewebe, die mit der Nukleinsäure gemäß Seq ID No.1 korrespondieren, bestimmt (genannt hits). Als Methode wird ein sogenannter "blast search" verwendet (NCBI BLAST v. 2.0.10; Altschul et al., Nucleic Acid Res. 1997, 25, 3389-3402). Gesucht wird in einer EST-Datenbank (LifeSeqGold from Incyte).

## Patentansprüche

1. Nukleinsäure umfassend
a. die in Seq ID NO 1, NO 3, NO 5, NO 7, NO 9, NO 11, NO 13 oder NO 15 dargestellte Nukleotidsequenz,
b. eine der Sequenzen aus a. im Rahmen der Degeneration des genetischen Codes entsprechende Nukleotidsequenz oder
c. eine mit den Sequenzen aus a. unter stringenten Bedingungen hybridisierende Nukleotidsequenz

2. Nukleinsäure nach Anspruch 1 umfassend einen Protein-kodierenden Abschnitt der in Seq ID NO 1, NO 3, NO 5, NO 7, NO 9, NO 11, NO 13 oder NO 15 dargestellten Nukleinsäuresequenz

3. Nukleinsäure kodierend für ein Polypeptid mit der in Seq ID NO 2, NO 4, NO 6, NO 8, NO 10, NO 12, NO 14 oder NO 16 dargestellten Aminosäuresequenz.

4. Polypeptid kodiert von einer Nukleinsäure nach einem der Ansprüche 1-3

5. Polypeptid umfassend die in Seq ID NO 2, NO 4, NO 6, NO 8, NO 10, NO 12, NO 14 oder NO 16 dargestellte Aminosäuresequenz oder Teile davon

6. Polypeptid umfassend die Kinase-Domäne der in Seq ID NO 2, NO 4, NO 6, NO 8, NO 10, NO 12, NO 14 oder NO 16 dargestellten Aminosäuresequenz

7. Verwendung eines Polypeptids nach Anspruch 4, 5 oder 6 oder von Teilen dieses Polypeptids zur Herstellung von Antikörpern.

8. Antikörper gegen ein Polypeptid nach einem der Ansprüche 4 bis 6

9. Verwendung einer Sonde mit Nukleinsäuresequenzen, die komplementär zu den Nukleinsäuresequenzen nach Anspruch 1-3 sind, zur Herstellung eines Reagenz zum Nachweis der Gegenwart von mRNA nach einem der Ansprüche 1 bis 3 in Zellen

10. Vektor enthaltend mindestens eine Kopie einer Nukleinsäure nach einem der Ansprüche 1-3

11. Zelle transfiziert mit einer Nukleinsäure nach einem der Ansprüche 1-3 oder mit einem Vektor nach Anspruch 10

12. Verwendung einer Zelle nach Anspruch 11 zur Expression der Nukleinsäure nach einem der Ansprüche 1-3.

13. Verwendung
a. einer Nukleinsäure nach einem der Ansprüche 1 bis 3,
b. eines Polypeptids nach einem der Ansprüche 4 bis 6 oder
c. einer Zelle nach Anspruch 11
zur Identifizierung von Effektoren eines Polypeptids nach Anspruch 4 oder 5.

14. Verwendung einer Nukleinsäure nach Anspruch 1 bis 3 oder eines Polypeptids nach Anspruch 4 bis 6 als Zielsubstanz zur Herstellung eines Mittels für Steroidhormonabhängige Erkrankungen.

15. Testsystem zur Identifizierung von Effektoren eines Polypeptids nach Anspruch 4 oder 5, wobei ein Polypeptid nach Anspruch 4 oder 5 mit einem Steroid, einem Steroidanalogon oder einem Sterol inkubiert wird und die Menge an gebundenem Steroid, Steroidanalogon oder Sterol detektiert wird.

16. Testsystem nach Anspruch 14 oder 15 wobei die Effektoren FF-MAS-Agonisten oder Antagonisten sind.

17. Testsystem zur Identifizierung von Inhibitoren der Kinasefunktion des Polypeptids nach Anspruch 4 oder 5, wobei ein Polypeptid nach Anspruch 4, 5 oder 6 mit den zu testenden Substanzen in Kontakt gebracht wird und die Kinaseaktivität bestimmt wird.

18. Verfahren zur Bereitstellung eines pharmazeutischen Mittels, wobei
a. Substanzen mit einem Testsystem nach Anspruch 15,16 oder 17 in Kontakt gebracht werden,
b. die Wirkung der Substanzen auf das Testsystem im Vergleich zu Kontrollen gemessen wird,
c. eine Substanz, die in Schritt b. eine Modulation der Aktivität des Polypeptids gemäß Anspruch 4 oder 5 zeigt, identifiziert wird
d. und die in Schritt c. identifizierte Substanz mit in der Pharmazie üblichen Formulierungsstoffen gemischt wird.
